# EUROPEAN PATENT APPLICATION

(11) **EP 4 420 676 A1**
(43) Date of publication of application: **28.08.2024**
(21) Application number: 23158624.9
(22) Date of filing: 24.02.2023
(51) Int. Cl.: A61K 39/00, C07K 16/28, A61P 37/06, A61P 3/10

(54) **CHIMERIC ANTIGEN RECEPTOR**

(71) Applicant: Medizinische Hochschule Hannover, 30625 Hannover (DE); Technische Universität Braunschweig, 38106 Braunschweig (DE)
(72) Inventor: The designation of the inventor has not yet been filed
(74) Representative: Dehns Germany Partnerschaft mbB

(57) **Abstract**

A chimeric antigen receptor (CAR), a cell (particularly an immune cell such as a regulatory T cell) expressing said CAR, a nucleic acid or vector encoding said CAR and various uses of said CAR, cell, nucleic acid or vector is disclosed herein. Particularly, a chimeric antigen receptor (CAR) comprising an antigen recognition domain that specifically binds to DGCR2 is provided.

## Description

### TECHNICAL FIELD

The present disclosure relates generally to the field of chimeric antigen receptors (CARs) and related therapies, such as the treatment of autoimmune or inflammatory diseases, particularly type 1 diabetes. More particularly, the disclosure provides CARs comprising an antigen recognition domain that binds to DGCR2 and that are expressed in immune cells (e.g., Tregs). Such immune cells have therapeutic uses in diseases and conditions associated with cells that express DGCR2 on their surface. The disclosure further provides nucleic acid molecules encoding such CARs and vectors containing them that may be used to modify host cells, e.g., immune cells, to express the CARs.

### BACKGROUND

Immunotherapy is emerging as a beneficial tool for the treatment of many conditions, ranging from cancer, autoimmune and inflammatory diseases, to the prevention of solid organ transplant rejection. In particular, there has been increased clinical activity in the area of adoptive cellular immunotherapy (ACT), particularly in regulatory T cell (Treg) cell therapies, across the autoimmune and inflammatory space.

CD4+FOXP3+ regulatory T cells (Tregs) are a lymphocyte subset that is essential for the maintenance of dominant immunological tolerance by inhibiting the function of various effector immune cell subsets such as T effector cells. In addition, Tregs are also known to promote tissue repair and regeneration. Tregs are able to confer immune tolerance through multiple contact-dependent and independent mechanisms. These include production of anti-inflammatory soluble mediators such as IL-10, TGF-β and IL-35, consumption of IL-2, expression of negative regulatory cell surface receptors, such as CTLA-4, and targeting T cells directly or indirectly through APCs. Importantly, once activated, Tregs can suppress immune responses in a non-antigen specific fashion (bystander suppression), i.e., once activated, Tregs have the ability to modulate the local immune micro-environment and suppress inflammation. Furthermore, they can confer a suppressive phenotype on other cells of the immune system, a process called "infectious tolerance".

Type 1 diabetes is a chronic autoimmune disease in which pancreatic beta cells, which are responsible for the production of insulin, are destroyed by the immune system. This is triggered by genetic and environmental factors. Destruction of beta cells reduces or eliminates production of insulin by the body and results in inflammation in the pancreatic islets. Insulin is a hormone required to regulate glucose levels in the bloodstream and, before treatment, subjects with type 1 diabetes will have excessively high blood sugar levels (hyperglycaemia). Type 1 diabetes is a serious and lifelong condition. Patients with type 1 diabetes currently need to closely monitor their blood glucose levels and take an appropriate dose of insulin (e.g. by injection or pump). This therapy is not a cure and must be continuously administered. Over time, irregularities in blood sugar levels, including large fluctuations in glycaemic level, can result in long-term complications such as damage to the heart, eyes, feet and kidneys, as well as a reduction in life expectancy. Type 1 diabetes thus imposes a substantial burden on health systems worldwide. Further, epidemiological data show a steady increase in the prevalence of autoimmune diseases such as type 1 diabetes in Western society over the last decades. The prevalence of type 1 diabetes in Western Europe and North America is approximately 0.5% or approximately 2 million people with a steady upward trend.

There is increasing evidence in mouse models of type 1 diabetes, as well as other autoimmune disorders, that dysregulation of Treg-mediated suppression of effector T cells (Teffs) contributes to the development of disease (see Visperas and Vignali, J Immunol. 2016; 197(10): 3762-3770). For example, defects in Treg phenotype and suppressive capacity have been observed in samples obtained from type 1 diabetes patients. Further, in mouse models of type 1 diabetes, depletion of Tregs has been shown to accelerate autoimmune diabetes development.

The prospect of ameliorating immunopathology and re-establishing tolerance in inflammatory diseases has prompted a growing interest in the clinical development of Treg-based immunotherapies. However, for a Treg immunotherapy to be successful it is beneficial to develop strategies that promote the trafficking of Tregs to the site of tissue damage and induce their activation *in situ.*

Autologous polyclonal Tregs have been administered to patients with type 1 diabetes with promising results, demonstrating the safety and potential of adoptive Treg therapy in this disease setting (see Marek-Trzonkowski et al, Clin Immunol. 2014; 153: 23-30 and Bluestone et al, Sci Transl Med. 2015; 7:315ra189). However, polyclonal Tregs may be associated with unwanted effects, such as systemic immunosuppression due to their lack of specificity. Further, it may be difficult to get sufficient numbers of polyclonal Tregs to traffic to the site of disease. A recent large clinical trial by Caladrius Biosciences (Sanford project) using polyclonal Tregs failed to have efficacy against type 1 diabetes.

Tregs from a transgenic mouse expressing a TCR specific for an islet antigen have been used to prevent or reverse diabetes in NOD mice (see Tang et al, J Exp Med. 2004; 199: 1455-65). However, this required activation of the Tregs through a TCR and, due to MHC restriction, this transgenic model is not transferable to patients since different patients would require different TCRs.

Artificial chimeric antigen receptors (CARs) have been used to confer antigen specificity on a cell. CARs are generally composed of an extracellular antigen-binding domain (e.g. a scFv specific for the target antigen), a transmembrane domain, and an intracellular signalling domain which sends signals into the cell and activates it upon binding of the antigen to the extracellular antigen-binding domain. CAR-Teff cell therapy has been approved for the treatment of certain blood cancers. However, CARs are artificial molecules engineered into cells and are less sensitive than TCRs, partly due to the number of molecules involved in the TCR machinery, i.e., CD4/CD8 co-receptors, immunoreceptor tyrosinase-rich activation motifs (ITAMs), and subunits within the receptor complex. It is therefore difficult to select an appropriate target for CAR-T cell therapy which will allow trafficking and subsequent activation of the CAR-T cells to have a therapeutic effect. For example, although insulin-specific CAR-Tregs proliferated in response to insulin and were suppressive *in vitro,* they did not prevent spontaneous diabetes in NOD mice (Tenspolde et al, J Autoimmunity. 2019; 103: 102289).

### SUMMARY

The present inventors have determined that a therapy for the treatment of an autoimmune or inflammatory disease may be developed by providing immune cell subsets with a chimeric antigen receptor (CAR) comprising an antigen recognition domain that is specific for DGCR2. The expression of such a CAR on the surface of Tregs provides a generic therapy that can be used for the treatment of autoimmune disease or inflammatory disease, where DGCR2 is expressed locally at the site of disease, in view of the well-known bystander effect of Tregs and their ability, once activated, to reduce the immune response and modulate the activation status of other immune cell subsets. Particularly, the inventors have developed an anti-DGCR2 CAR for the treatment of type 1 diabetes.

The inventors have thus identified DGCR2 as a surprisingly effective target for CAR-Treg therapy, particularly for type 1 diabetes and other pancreatic autoimmune or inflammatory disorders. In particular, the inventors have found that cells expressing CARs specific for DGCR2 are activated in the presence of antigen and T cells expressing a DGCR2-specific CAR traffic to the pancreas of NOD.SCID mice.

Whilst DGCR2 has been reported to be expressed on pancreatic tissue, its function on islet cells is still unclear. Further, expression of DGCR2 in other tissues (brain, heart, lung and fetal kidney) has been reported in the art, making selection of DGCR2 as a target for specific trafficking (thus allowing activation of Tregs to sufficient levels to treat type I diabetes) unintuitive. Particularly, RNA expression of DGCR2 in many other cell types and tissues has been reported in the art (Lindskog et al, 2012, J. Proteomics, 2611-2620). The inventors' finding that DGCR2 protein is an effective pancreatic target, allowing DGCR2-specific CAR-T cells to traffic to the pancreas, is therefore particularly surprising.

Accordingly, in one aspect, the present invention provides a chimeric antigen receptor (CAR) comprising an antigen recognition domain that specifically binds to DGCR2 (e.g. specifically binds to human DGCR2 and/or murine DGCR2).

In this respect, there is provided herein a CAR comprising:
a. an exodomain comprising the antigen recognition domain;
b. a transmembrane domain; and
c. an endodomain comprising an intracellular signalling domain.

The CAR may further comprise a hinge domain and/or one or more co-stimulatory domains.

The term "hinge domain" typically refers to the portion of the exodomain that connects the antigen recognition domain with the transmembrane domain. The hinge domain may be selected from the hinge regions of CD28, CD8α, CD4, CD7, CH2CH3, an immunoglobulin, or a part or variant thereof. Preferably, the CAR may comprise a CD8α or CH2CH3 hinge domain.

The co-stimulatory domain may be selected from the intracellular domains of CD28, ICOS, CD134 (OX40), CD137 (4-1 BB), CD27, or TNFRSF25, or a part or variant thereof. Preferably, the CAR may comprise a CD28 co-stimulatory domain.

The CAR may comprise one or more transmembrane domains, which may be selected from the transmembrane domains of CD28, ICOS, CD8α, CD4, CD134 (OX40), CD137 (4-1 BB), CD3 zeta, CD45, CD9, CD16, CD22, CD33, CD64, CD80, CD86, CD154, or a part or variant thereof. Preferably, the CAR comprises a CD4, CD28 or CD8α transmembrane domain.

The CAR (specifically the endodomain of the CAR) may comprise one or more intracellular signalling domains selected from the group consisting of the CD3 zeta signalling domain or any of its homologs, a CD3 polypeptide, a syk family tyrosine kinase, a src family tyrosine kinase, CD2, CD5, and CD28, or a part or variant thereof. Preferably, the CAR may comprise the CD3 zeta signalling domain.

In one embodiment, the CAR may comprise a CD8α or CH2CH3 hinge domain (i.e. a hinge domain derived from CD8α or CH2CH3); a CD8α or CD28 transmembrane domain (i.e. a transmembrane domain derived from CD8α or CD28); a CD28 co-stimulatory domain (i.e. a co-stimulatory domain derived from CD28); and the CD3 zeta signalling domain (i.e. a signalling domain derived from CD3 zeta), wherein when the hinge domain is a CD8α hinge domain, the transmembrane domain is a CD8α transmembrane domain, and when the hinge domain is a CH2CH3 hinge domain, the transmembrane domain is a CD28 transmembrane domain. Alternatively viewed, in one embodiment, the CAR may comprise a CD8α hinge domain, a CD8α transmembrane domain, a CD28 co-stimulatory domain, and the CD3 zeta signalling domain. In a separate embodiment, the CAR may comprise a CH2CH3 hinge domain, a CD28 transmembrane domain, a CD28 co-stimulatory domain, and the CD3 zeta signalling domain. Further, the CAR may comprise a CD28 transmembrane domain (i.e. a transmembrane domain derived from CD28), particularly in combination with a CD28 co-stimulatory domain.

The CAR of the present invention may comprise a signal peptide and/or a reporter peptide. In one embodiment, the polynucleotide sequence encoding a CAR of the present invention may comprise a further polynucleotide sequence encoding a reporter peptide linked by a self-cleaving or cleavage domain.

The antigen recognition domain of the CAR of the present invention may be an antibody, an antibody fragment, or derived from an antibody. Preferably, the antigen recognition domain is a single chain antibody (scFv).

The CAR of the present invention may, for example, comprise an antigen recognition domain which comprises:
(i) VH CDR 1, 2 and 3 sequences as set forth in SEQ ID NOs: 198, 2 and 3 respectively and VL CDR 1, 2 and 3 sequences as set forth in (a) SEQ ID NOs: 4, 202 and 205 respectively, or (b) SEQ ID NOs: 4, 202 and 206;
(ii) VH CDR 1, 2 and 3 sequences as set forth in SEQ ID NOs: 198, 8 and 9 respectively and VL CDR 1, 2 and 3 sequences as set forth in SEQ ID NOs: 201, 204 and 12 respectively;
(iii) VH CDR 1, 2 and 3 sequences as set forth in SEQ ID NOs: 13, 14 and 15 respectively and VL CDR 1, 2 and 3 sequences as set forth in SEQ ID NOs: 16, 202 and 206 respectively;
(iv) VH CDR 1, 2 and 3 sequences as set forth in SEQ ID NOs: 198, 26 and 27 respectively and VL CDR 1, 2 and 3 sequences as set forth in SEQ ID NOs: 28, 202 and 30 respectively;
(v) VH CDR1, 2 and 3 sequences set forth in SEQ ID NOs: 200, 32 and 33 respectively and VL CDR1, 2 and 3 sequences set forth in SEQ ID NOs: 201, 203 and 36 respectively;
(vi) VH CDR1, 2 and 3 sequences set forth in SEQ ID NOs: 199, 38 and 39 respectively and VL CDR1, 2 and 3 sequences set forth in SEQ ID NOs: 40, 41 and 42 respectively;
(vii) VH CDR1, 2 and 3 sequences set forth in (a) SEQ ID NOs: 198, 44 and 45 respectively or (b) SEQ ID NOs: 199, 44 and 45 or (c) SEQ ID NOs: 200, 44 and 45, and VL CDR1, 2 and 3 sequences set forth in (a) SEQ ID NOs: 46, 203 and 48 respectively or (b) SEQ ID NOs: 46, 204 and 48 respectively; or
(viii) VH CDR1, 2 and 3 sequences set forth in SEQ ID NOs: 198, 50 and 51 respectively and VL CDR1, 2 and 3 sequences set forth in SEQ ID NOs: 52, 202 and 205 respectively;
wherein one or more of said CDR sequences of (i) to (viii) may optionally comprise 1 to 3 amino acid modifications relative to an aforementioned CDR sequence, particularly wherein one or more of said CDR sequences may optionally be modified by substitution, addition or deletion of 1 to 3 amino acids.

The CAR of the present invention may, for example, comprise an antigen recognition domain which comprises:
(i) VH CDR1, 2 and 3 sequences set forth in SEQ ID NOs: 1, 2 and 3 respectively and VL CDR1, 2 and 3 sequences set forth in SEQ ID NOs: 4, 5 and 6 respectively;
(ii) VH CDR1, 2 and 3 sequences set forth in SEQ ID NOs: 7, 8 and 9 respectively and VL CDR1, 2 and 3 sequences set forth in SEQ ID NOs: 10, 11 and 12 respectively;
(iii) VH CDR1, 2 and 3 sequences set forth in SEQ ID NOs: 13, 14 and 15 respectively and VL CDR1, 2 and 3 sequences set forth in SEQ ID NOs: 16, 17 and 18 respectively;
(iv) VH CDR1, 2 and 3 sequences set forth in SEQ ID NOs: 19. 20 and 21 respectively and VL CDR1, 2 and 3 sequences set forth in SEQ ID NOs: 22, 23 and 24 respectively;
(v) VH CDR1, 2 and 3 sequences set forth in SEQ ID NOs: 25, 26 and 27 respectively and VL CDR1, 2 and 3 sequences set forth in SEQ ID NOs: 28, 29 and 30 respectively;
(vi) VH CDR1, 2 and 3 sequences set forth in SEQ ID NOs: 31, 32 and 33 respectively and VL CDR1, 2 and 3 sequences set forth in SEQ ID NOs: 34, 35 and 36 respectively;
(vii) VH CDR1, 2 and 3 sequences set forth in SEQ ID NOs: 37, 38 and 39 respectively and VL CDR1, 2 and 3 sequences set forth in SEQ ID NOs: 40, 41 and 42 respectively;
(viii) VH CDR1, 2 and 3 sequences set forth in SEQ ID NOs: 43, 44 and 45 respectively and VL CDR1, 2 and 3 sequences set forth in SEQ ID NOs: 46, 47 and 48 respectively; or
(ix) VH CDR1, 2 and 3 sequences set forth in SEQ ID NOs: 49, 50 and 51 respectively and VL CDR1, 2 and 3 sequences set forth in SEQ ID NOs: 52, 53 and 54 respectively;
wherein one or more of said CDR sequences of (i) to (ix) may optionally comprise 1 to 3 amino acid modifications relative to an aforementioned CDR sequence, particularly wherein one or more of said CDR sequences may optionally be modified by substitution, addition or deletion of 1 to 3 amino acids.

Also in this respect, the antigen recognition domain of the CAR may comprise:
(i) a VH domain comprising the sequence set forth in SEQ ID NO: 55, or a sequence having at least 70% sequence identity thereto, and a VL domain comprising the sequence as set forth in SEQ ID NO: 56, or a sequence having at least 70% identity thereto;
(ii) a VH domain comprising the sequence set forth in SEQ ID NO: 57, or a sequence having at least 70% sequence identity thereto, and a VL domain comprising the sequence as set forth in SEQ ID NO: 58, or a sequence having at least 70% identity thereto;
(iii) a VH domain comprising the sequence set forth in SEQ ID NO: 59, or a sequence having at least 70% sequence identity thereto, and a VL domain comprising the sequence as set forth in SEQ ID NO: 60, or a sequence having at least 70% identity thereto;
(iv) a VH domain comprising the sequence set forth in SEQ ID NO: 61, or a sequence having at least 70% sequence identity thereto, and a VL domain comprising the sequence as set forth in SEQ ID NO: 62, or a sequence having at least 70% identity thereto;
(v) a VH domain comprising the sequence set forth in SEQ ID NO: 63, or a sequence having at least 70% sequence identity thereto, and a VL domain comprising the sequence as set forth in SEQ ID NO: 64, or a sequence having at least 70% identity thereto;
(vi) a VH domain comprising the sequence set forth in SEQ ID NO: 65, or a sequence having at least 70% sequence identity thereto, and a VL domain comprising the sequence as set forth in SEQ ID NO: 66, or a sequence having at least 70% identity thereto;
(vii) a VH domain comprising the sequence set forth in SEQ ID NO: 67, or a sequence having at least 70% sequence identity thereto, and a VL domain comprising the sequence as set forth in SEQ ID NO: 68, or a sequence having at least 70% identity thereto;
(viii) a VH domain comprising the sequence set forth in SEQ ID NO: 69, or a sequence having at least 70% sequence identity thereto, and a VL domain comprising the sequence as set forth in SEQ ID NO: 70, or a sequence having at least 70% identity thereto; or
(ix) a VH domain comprising the sequence set forth in SEQ ID NO: 71, or a sequence having at least 70% sequence identity thereto, and a VL domain comprising the sequence as set forth in SEQ ID NO: 72, or a sequence having at least 70% identity thereto.

The antigen recognition domain may, for example, comprise:
(i) a VH domain comprising the sequence encoded by SEQ ID NO: 73, or a sequence having at least 70% identity to the sequence encoded by SEQ ID NO: 73, and a VL domain comprising the sequence encoded by SEQ ID NO: 74, or a sequence having at least 70% identity to the sequence encoded by SEQ ID NO: 74;
(ii) a VH domain comprising the sequence encoded by SEQ ID NO: 75, or a sequence having at least 70% identity to the sequence encoded by SEQ ID NO: 75, and a VL domain comprising the sequence encoded by SEQ ID NO: 76, or a sequence having at least 70% identity to the sequence encoded by SEQ ID NO: 76;
(iii) a VH domain comprising the sequence encoded by SEQ ID NO: 77, or a sequence having at least 70% identity to the sequence encoded by SEQ ID NO: 77, and a VL domain comprising the sequence encoded by SEQ ID NO: 78, or a sequence having at least 70% identity to the sequence encoded by SEQ ID NO: 78;
(iv) a VH domain comprising the sequence encoded by SEQ ID NO: 79, or a sequence having at least 70% identity to the sequence encoded by SEQ ID NO: 79, and a VL domain comprising the sequence encoded by SEQ ID NO: 80, or a sequence having at least 70% identity to the sequence encoded by SEQ ID NO: 80;
(v) a VH domain comprising the sequence encoded by SEQ ID NO: 81, or a sequence having at least 70% identity to the sequence encoded by SEQ ID NO: 81, and a VL domain comprising the sequence encoded by SEQ ID NO: 82, or a sequence having at least 70% identity to the sequence encoded by SEQ ID NO: 82;
(vi) a VH domain comprising the sequence encoded by SEQ ID NO: 83, or a sequence having at least 70% identity to the sequence encoded by SEQ ID NO: 83, and a VL domain comprising the sequence encoded by SEQ ID NO: 84, or a sequence having at least 70% identity to the sequence encoded by SEQ ID NO: 84;
(vii) a VH domain comprising the sequence encoded by SEQ ID NO: 85, or a sequence having at least 70% identity to the sequence encoded by SEQ ID NO: 85, and a VL domain comprising the sequence encoded by SEQ ID NO: 86, or a sequence having at least 70% identity to the sequence encoded by SEQ ID NO: 86;
(viii) a VH domain comprising the sequence encoded by SEQ ID NO: 87, or a sequence having at least 70% identity to the sequence encoded by SEQ ID NO: 87, and a VL domain comprising the sequence encoded by SEQ ID NO: 88, or a sequence having at least 70% identity to the sequence encoded by SEQ ID NO: 88; or
(ix) a VH domain comprising the sequence encoded by SEQ ID NO: 89, or a sequence having at least 70% identity to the sequence encoded by SEQ ID NO: 89, and a VL domain comprising the sequence encoded by SEQ ID NO: 90, or a sequence having at least 70% identity to the sequence encoded by SEQ ID NO: 90.

Furthermore, the antigen recognition domain of the CAR may comprise or consist of:
(i) the sequence set forth in SEQ ID NO: 91 or a sequence having at least 80% sequence identity thereto;
(ii) the sequence set forth in SEQ ID NO: 92 or a sequence having at least 80% sequence identity thereto;
(iii) the sequence set forth in SEQ ID NO: 93 or a sequence having at least 80% sequence identity thereto;
(iv) the sequence set forth in SEQ ID NO: 94 or a sequence having at least 80% sequence identity thereto;
(v) the sequence set forth in SEQ ID NO: 95 or a sequence having at least 80% sequence identity thereto;
(vi) the sequence set forth in SEQ ID NO: 96 or a sequence having at least 80% sequence identity thereto;
(vii) the sequence set forth in SEQ ID NO: 97 or a sequence having at least 80% sequence identity thereto;
(viii) the sequence set forth in SEQ ID NO: 98 or a sequence having at least 80% sequence identity thereto; or
(ix) the sequence set forth in SEQ ID NO: 99 or a sequence having at least 80% sequence identity thereto.

The antigen recognition domain may, for example, comprise or consist of:
(i) the sequence encoded by the sequence set forth in SEQ ID NO: 100 or a sequence having at least 80% identity to the sequence encoded by the sequence set forth in SEQ ID NO: 100;
(ii) the sequence encoded by the sequence set forth in SEQ ID NO: 101 or a sequence having at least 80% identity to the sequence encoded by the sequence set forth in SEQ ID NO: 101;
(iii) the sequence encoded by the sequence set forth in SEQ ID NO: 102 or a sequence having at least 80% identity to the sequence encoded by the sequence set forth in SEQ ID NO: 102;
(iv) the sequence encoded by the sequence set forth in SEQ ID NO: 103 or a sequence having at least 80% identity to the sequence encoded by the sequence set forth in SEQ ID NO: 103;
(v) the sequence encoded by the sequence set forth in SEQ ID NO: 104 or a sequence having at least 80% identity to the sequence encoded by the sequence set forth in SEQ ID NO: 104;
(vi) the sequence encoded by the sequence set forth in SEQ ID NO: 105 or a sequence having at least 80% identity to the sequence encoded by the sequence set forth in SEQ ID NO: 105;
(vii) the sequence encoded by the sequence set forth in SEQ ID NO: 106 or a sequence having at least 80% identity to the sequence encoded by the sequence set forth in SEQ ID NO: 106;
(viii) the sequence encoded by the sequence set forth in SEQ ID NO: 107 or a sequence having at least 80% identity to the sequence encoded by the sequence set forth in SEQ ID NO: 107; or
(ix) the sequence encoded by the sequence set forth in SEQ ID NO: 108 or a sequence having at least 80% identity to the sequence encoded by the sequence set forth in SEQ ID NO: 108.

In a second aspect, the invention provides a nucleic acid molecule comprising a nucleotide sequence encoding the CAR according to the invention.

In a third aspect, the invention provides a vector comprising the nucleic acid molecule according to the invention. The vector may further comprise a nucleic acid molecule comprising a nucleotide sequence encoding a FOXP3 polypeptide, or a derivative or variant thereof.

In a further aspect, the invention provides a cell comprising the CAR, nucleic acid molecule or vector according to the invention. The cell may further comprise an exogenous FOXP3 polypeptide or an exogenous nucleic acid encoding FOXP3. The cell may be an immune cell or a progenitor or precursor thereof. Preferably, the cell may be a T cell, or a precursor thereof, or a stem cell. In particular, the cell may be a Treg, or a precursor thereof, or an iPSC cell. The cell may be a production host cell. In a specific embodiment, the invention provides a Treg comprising a CAR, wherein the antigen recognition domain of the CAR specifically binds to DGCR2. In a further specific embodiment, the invention provides a T-effector cell (Teff) comprising a CAR, wherein the antigen recognition domain of the CAR specifically binds to DGCR2.

The cell may be provided in a cell population, which forms a further aspect of the invention. In particular, the cell population may comprise a plurality of cells according to the invention, particularly a plurality of T cells (e.g. a plurality of Tregs or a plurality of Teffs) according to the invention.

The invention also provides a pharmaceutical composition comprising the cell, cell population or vector according to the invention.

In another aspect, the invention provides a cell, cell population or pharmaceutical composition according to the invention for use in therapy (e.g., for use in treating and/or preventing an autoimmune or inflammatory disease, or for use in inducing immunosuppression, or for use in promoting tissue repair and/or tissue regeneration). The therapy may be adoptive cell transfer therapy.

Alternatively viewed, the invention provides a method for treating and/or preventing an autoimmune or inflammatory disease, or for inducing immunosuppression, or for promoting tissue repair and/or tissue regeneration, wherein the method comprises administering a cell, particularly a Treg cell, a cell population, or a pharmaceutical composition, particularly comprising a Treg, according to the invention.

In this respect, the method may comprise the following steps:
(i) isolation or provision of a Treg-enriched cell sample from a subject;
(ii) introduction into the Treg cells of a nucleic acid molecule or vector of the invention; and
(iii) administering the Treg cells from (ii) to the subject.

The invention also provides use of a cell, cell population or pharmaceutical composition according to the invention in the manufacture of a medicament for treating and/or preventing an autoimmune or inflammatory disease, or for inducing immunosuppression, or for promoting tissue repair and/or tissue regeneration in a subject, particularly wherein the cell is a Treg cell.

The autoimmune or inflammatory disease may particularly be type 1 diabetes (T1D), for example recent-onset type 1 diabetes.

In another aspect, the invention provides a method of making a cell according to the invention, which comprises the step of introducing into the cell (e.g., transducing or transfecting a cell with) the nucleic acid molecule or vector according to the invention. The cell may be a Treg cell, and the method may comprise isolating or providing a cell-containing sample comprising Tregs, and/or enriching Tregs or generating Tregs from the cell-containing sample prior to or after the step of introducing the nucleic acid molecule or vector into the cell. The invention also provides a cell obtainable by this method, which forms a further aspect of the invention.

In a further aspect, the invention provides the use of a CAR-Treg to reduce death (e.g., reduce the rate of death) or prevent death of pancreatic beta cells in a subject. In a further aspect, the invention provides the use of a CAR-Treg to maintain or increase fasting blood insulin levels in a subject. In a further aspect, the invention provides the use of a CAR-Treg to maintain or increase fasting C-peptide levels in a subject. In a further aspect, the invention provides the use of a CAR-Treg to reduce or prevent hyperglycaemia in a subject. In a further aspect, the invention provides the use of a CAR-Treg to maintain or decrease fasting blood glucose levels in a subject. In a further aspect, the invention provides the use of a CAR-Treg to maintain or reduce HbA1c levels in a subject. The CAR is the CAR of the present invention, i.e., it comprises an antigen recognition domain that specifically binds to DGCR2 (e.g. to human DGCR2 and/or murine DGCR2), and it may have any of the features of the CAR as disclosed herein. The subject may, for example, have or be at risk of developing type 1 diabetes, particularly the subject may have recent-onset type 1 diabetes. The subject may, for example, not be being administered exogenous insulin. Alternatively, the subject may be being administered a reduced dose of insulin compared to the required dose of insulin prior to administration of the CAR-Tregs.

### BRIEF DESCRIPTION OF THE FIGURES

Figure 1 shows binding of various scFvs to HEK293T cells expressing either human DGCR2 (first row) or murine DGCR2 (second row).
Figure 2 shows immunohistochemistry staining of a DGCR2-specific scFv to murine pancreas sections.
Figure 3 shows activation of murine hybridoma cells expressing a DGCR2-specific CAR by human DGCR2 protein, HEK cells expressing human DGCR2 antigen and HEK cells expressing murine DGCR2 antigen.
Figure 4 shows an overview of the experimental protocol used to observe enrichment of T cells expressing a DGCR2-specific CAR in pancreatic tissue of NOD mice.
Figure 5 shows enrichment of T cells expressing DGCR2-specific CARs within pancreatic islets of NOD mice compared to spleen and lymph nodes.
Figure 6 shows the configuration of the CARs used in the Examples - (A) shows the schematic structure of CARs with a CD8 hinge, (B) shows the schematic structure of the constructs used in gamma retroviral vectors for transduction of cells.

### DETAILED DESCRIPTION

The present invention provides CAR-Tregs specific for DGCR2 that are activated in the presence of DGCR2 antigen which is particularly expressed on pancreatic beta cells. Thus, these CAR-Tregs have therapeutic potential in treating autoimmune and inflammatory disorders where DGCR2 is expressed locally at the site of disease. In particular, these CAR-Tregs have therapeutic potential for type 1 diabetes. Particularly, due to the bystander effect of Treg cells, the antigen (DGCR2) may be simply present and/or expressed at the site of inflammation or disease.

Accordingly, the present invention provides a chimeric antigen receptor (CAR) comprising an antigen recognition domain that binds to DGCR2, a cell or cell population expressing the CAR (e.g. a Treg or a population of Tregs expressing the CAR) and use of the cell or cell population in treating certain diseases, e.g., type 1 diabetes.

A "chimeric antigen receptor", "CAR" or "CAR construct" refers to engineered receptors which can confer an antigen specificity onto cells (e.g., immune cells, such as Tregs). In particular, a CAR enables a cell to bind specifically to a particular antigen, e.g., a target molecule such as a target protein, whereupon a signal is generated by the endodomain (comprising an intracellular signalling domain) of the CAR, e.g., a signal resulting in activation of the cell. CARs are also known as artificial T-cell receptors, chimeric T-cell receptors or chimeric immunoreceptors.

The structure of CARs is well-known in the art and several generations of CARs have been produced. For instance, as a minimum a CAR may contain an extracellular antigen-specific targeting region, antigen binding domain, target binding domain or ligand binding domain, which is or forms part of the exodomain (also known as the extracellular domain or ectodomain) of the CAR, a transmembrane domain, and an intracellular signalling domain (which is, or is comprised within, an endodomain). However, the CAR may contain further domains to improve its functionality, e.g., one or more costimulatory domains to improve T cell proliferation, cytokine secretion, resistance to apoptosis, and *in vivo* persistence.

Thus, a chimeric receptor or CAR construct generally comprises a binding domain (which may be viewed as an antigen (i.e., target) or ligand binding domain and the terms binding domain, antigen recognition domain, antigen binding domain and ligand binding domain are used interchangeably herein), optionally a hinge domain, which functions as a spacer to extend the binding domain away from the plasma membrane of the cell (e.g., immune cell) on which it is expressed, a transmembrane domain, an intracellular signalling domain (e.g., the signalling domain from the zeta chain of the CD3 molecule (CD3Q of the TCR complex, or an equivalent) and optionally one or more co-stimulatory domains, which may assist in signalling or functionality of the cell expressing the CAR. A CAR may also comprise a signal or leader sequence or domain which functions to target the protein to the membrane and may form part of the exodomain of the CAR. The different domains may be linked directly or by linkers, and/or may occur within different polypeptides, e.g., within two polypeptides which associate with one another.

When the CAR binds its target antigen (i.e., DGCR2), this results in the transmission of an activating signal to the cell in which it is expressed. Thus, the CAR directs the specificity of the engineered cells towards DGCR2, particularly towards cells expressing DGCR2.

The term "directed towards" or "directed against" is synonymous with "specific for" or "anti". Put another way, the CAR recognises the DGCR2 target molecule. Accordingly, it is meant that the CAR is capable of binding specifically to DGCR2. In particular, the antigen-binding domain of the CAR is capable of binding specifically to DGCR2 (more particularly when the CAR is expressed on the surface of a cell, notably an immune effector cell). Specific binding may be distinguished from non-specific binding to a non-target molecule or antigen. Thus, a cell expressing the CAR is directed, or re-directed, to bind specifically to a target cell, expressing DGCR2, particularly a target cell expressing DGCR2 on its cell surface. Particularly, "specific" binding means that binding only or mainly occurs to DGCR2 and not to other proteins or polypeptides (i.e., binding to other proteins or polypeptides is insignificant or weaker). Some cross-reaction with other proteins may occur but this level of binding can be considered as background. As mentioned above, the CAR is capable of binding to DGCR2 and of transducing a signal into a cell in which it is expressed. The cell may then be activated and may exert a suppressive effect within the local environment. Activation of a cell expressing a CAR after antigen binding can be determined by an increased level of CD69 as compared to the same cells expressing a CAR in the absence of antigen. For example, an increase of at least 10, 20, 30, 40, 50, 60, 70, 80 or 90% in CD69. Expression levels of CD69 can be determined using standard techniques, for example FACS, using commercially available antibodies (e.g. FITC anti-human CD69 antibody, Biolegend). Thus, CAR function within a cell can be determined by activation status of the cell in which the CAR is expressed, e.g., by determining CD69 expression.

DGCR2 (DiGeorge Syndrom Critical Region 2) may also be referred to as DGS-C, IDD, LAN, SEZ-12, 930034O06Rik, Dgsc, Sez12 and mKIAA0163. DGCR2 is a C-type lectin-like transmembrane protein. The function of DGCR2 is not known, however deletions have been associated with various neurological diseases and it is thought that it could be involved in cell-cell or cell-matrix interaction required for cell differentiation and migration. DGCR2 is expressed on pancreatic beta cells. The amino acid sequence of human DGCR2 is shown in SEQ ID NO: 170 and the amino acid sequence of mouse DGCR2 is shown in SEQ ID NO: 171.

The antigen-binding domain of a CAR may be derived or obtained from any protein or polypeptide which binds (i.e., has affinity for) DGCR2 (e.g. which binds to any region or part of DGCR2, or alternatively viewed which binds to any epitope within DGCR2, either in an isolated protein form or when expressed on cells). Particularly, the antigen-binding domain of a CAR may be derived or obtained from any protein or polypeptide which binds (i.e., has affinity for) the extracellular domain of DGCR2. This may be for example, a ligand of DGCR2, or a physiological binding protein for DGCR2, or a part thereof, or a synthetic or derivative protein. The target molecule (i.e., DGCR2) may commonly be expressed on the surface of a cell, for example a target cell (e.g., a pancreatic beta cell), or a cell in the vicinity of a target cell (for a bystander effect), but need not be.

The antigen-binding domain is most commonly derived from antibody variable chains (for example it commonly takes the form of a scFv), but may also be generated from other molecules, such as ligands or other binding molecules.

The CAR is typically expressed as a polypeptide also comprising a signal sequence (also known as a leader sequence), and in particular a signal sequence which targets the CAR to the plasma membrane of the cell. This will generally be positioned next to or close to the antigen binding domain, generally upstream of the antigen binding domain. The extracellular domain, or ectodomain, of the CAR may thus comprise, consist essentially of or consist of a signal sequence and an antigen binding domain.

As noted above, the antigen binding domain may be any protein or peptide that possesses the ability to specifically recognize and bind to DGCR2. The antigen binding domain includes any naturally occurring, synthetic, semi-synthetic, or recombinantly produced binding partner for DGCR2. Illustrative antigen-specific targeting domains include antibodies or antibody fragments or derivatives, or ligands for soluble or membrane bound DGCR2.

In an embodiment, the antigen binding domain is, or is derived from, an antibody. The term "antibody" as used herein refers broadly to any immunological binding agent or molecule that comprises an antigen binding domain, including polyclonal and monoclonal antibodies. Depending on the type of constant domain in the heavy chains, whole antibodies are assigned to one of five major classes: IgA, IgD, IgE, IgG and IgM and the antibodies as described herein may be in any one of these classes. Several of these are further divided into subclasses or isotypes such as IgG1, IgG2, IgG3, IgG4 and the like. Generally, IgG or IgM antibodies are the most common antibodies utilised in physiological settings. As will be understood by those in the art, the term "antibody" extends to all antibodies including whole antibodies, dimeric, trimeric and multimeric antibodies; bispecific antibodies; chimeric antibodies; recombinant and engineered antibodies and fragments thereof.

An antibody-derived binding domain can be a fragment of an antibody or a genetically engineered product of one or more fragments of the antibody, which fragment is involved in binding with the antigen. Examples include a variable region (Fv), a complementarity determining region (CDR), Fab or F(ab')₂, or the light and heavy chain variable regions can be joined together in a single chain (e.g. as a scFv) and in either orientation (e.g. V_{L}-V_{H} or V_{H}-V_{L}). The V_{L} and/or V_{H} sequences may be modified. In particular, the framework regions may be modified (e.g., substituted, for example to humanise the antigen-binding domain). Other examples include a heavy chain variable region (VH), a light chain variable region (VL) and a single domain antibody (sAb) which may be referred to as a nanobody. An example of a single domain antibody is a camelid heavy-chain antibody (HCAb) which has an antigen recognition site formed by a single domain, termed VHH.

In a preferred embodiment, the antigen-binding domain is a single chain antibody (scFv). The scFv may be murine, human or humanized scFv.

In an alternative preferred embodiment, the antigen-binding domain is derived from a camelid heavy-chain antibody (HCAb), for example the antigen-binding domain may be a VHH domain of a HCAb. VHHs have a similar structure to that of VH domains from conventional IgGs and include three variable CDRs, although CDRs 1 and 3 often have more amino acids than those of VH domains. In certain embodiments, the VHH domain may comprise one or more CDRs comprising or consisting of the sequence(s) set forth in SEQ ID NOs: 1 to 54 specified herein, or sequence having at least 70%, 75%, 80%, 85%, 90%, 95%, 96%, 97%, 98%, or 99% sequence identity thereto. For example, the VHH domain may comprise one, two or three of the VH CDRs comprising or consisting of the sequence(s) set forth in SEQ ID NOs: 1 to 54 specified herein, or sequences having at least 70%, 75%, 80%, 85%, 90%, 95%, 96%, 97%, 98%, or 99% sequence identity thereto. Where the VHH domain comprises two or three VH CDRs of SEQ ID NOs: 1 to 54 or 198 to 200, the CDRs may be from the same binder described herein (e.g. all from the G3, G9, G11 binder etc).

"Complementarity determining region" or "CDR" with regard to an antibody or antigen- binding fragment thereof refers to a highly variable loop in the variable region of the heavy chain or the light chain of an antibody. CDRs can interact with the antigen conformation and largely determine binding to the antigen (although some framework regions are known to be involved in binding). The heavy chain variable region and the light chain variable region each contain 3 CDRs. "Heavy chain variable region" or "VH" refers to the fragment of the heavy chain of an antibody that contains three CDRs interposed between flanking stretches known as framework regions, which are more highly conserved than the CDRs and form a scaffold to support the CDRs. "Light chain variable region" or "VL" refers to the fragment of the light chain of an antibody that contains three CDRs interposed between framework regions.

"Fv" refers to the smallest fragment of an antibody to bear the complete antigen binding site. An Fv fragment consists of the variable region of a single light chain bound to the variable region of a single heavy chain. "Single-chain Fv antibody" or "scFv" refers to an engineered antibody consisting of a light chain variable region and a heavy chain variable region connected to one another, in either orientation, directly or via a peptide linker sequence.

Antibodies that specifically bind a predetermined antigen, i.e., DGCR2, can be prepared using methods well known in the art. Such methods include phage display, methods to generate human or humanized antibodies, or methods using a transgenic animal or plant engineered to produce human antibodies. Phage display libraries of partially or fully synthetic antibodies are available and can be screened for an antibody or fragment thereof that can bind to the target molecule, i.e., to DGCR2. Phage display libraries of human antibodies are also available. Once identified, the amino acid sequence or polynucleotide sequence coding for the antibody can be isolated and/or determined.

The antigen recognition domain may bind, suitably specifically bind, one or more regions or epitopes within DGCR2. An epitope, also known as antigenic determinant, is the part of an antigen that is recognised by an antigen recognition domain (e.g., an antibody). In other words, the epitope is the specific piece of the antigen to which an antibody binds. Suitably, the antigen recognition domain binds, suitably specifically binds, to one region or epitope within DGCR2.

The antigen recognition domain may comprise at least one CDR (e.g. CDR3), which can be predicted from an antibody which binds to an antigen, i.e., DGCR2 (or a variant of such a predicted CDR (e.g. a variant with one, two or three amino acid substitutions)). It will be appreciated that molecules containing three or fewer CDR regions (e.g. a single CDR or even a part thereof) may be capable of retaining the antigen-binding activity of the antibody from which the CDR is derived. Molecules containing two CDR regions are described in the art as being capable of binding to a target antigen, e.g. in the form of a minibody (Vaughan and Sollazzo, 2001, Combinational Chemistry & High Throughput Screening, 4, 417-430). Molecules containing a single CDR have been described which can display strong binding activity to target (Nicaise et al, 2004, Protein Science, 13: 1882-91).

In this respect, the antigen binding domain may comprise one or more variable heavy chain CDRs, e.g., one, two or three variable heavy chain CDRs. Alternatively, or additionally, the antigen binding domain may comprise one or more variable light chain CDRs, e.g. one, two or three variable light chain CDRs. The antigen binding domain may comprise three heavy chain CDRs and/or three light chain CDRs (and more particularly a heavy chain variable region comprising three CDRs and/or a light chain variable region comprising three CDRs) wherein at least one CDR, preferably all CDRs, may be from an antibody which binds to DGCR2.

The antigen binding domain may comprise any combination of variable heavy and light chain CDRs, e.g. one variable heavy chain CDR together with one variable light chain CDR, two variable heavy chain CDRs together with one variable light chain CDR, two variable heavy chain CDRs together with two or three variable light chain CDRs, three variable heavy chain CDRs together with one or two variable light chain CDRs, one variable heavy chain CDR together with two or three variable light chain CDRs, or three variable heavy chain CDRs together with three variable light chain CDRs. Preferably, the antigen binding domain comprises three variable heavy chain CDRs (CDR1, CDR2 and CDR3) and/or three variable light chain CDRs (CDR1, CDR2 and CDR3).

The one or more CDRs present within the antigen binding domain may not all be from the same antibody, as long as the domain has the desired binding activity. Thus, one CDR may be predicted from the heavy or light chains of an antibody which binds to DGCR2 whilst another CDR present may be predicted from a different antibody which binds to DGCR2. A combination of CDRs may be used from different antibodies, particularly from antibodies that bind to the same desired region or epitope.

In a particularly preferred embodiment, the antigen binding domain comprises three CDRs predicted from the variable heavy chain sequence of an antibody which binds to DGCR2 and/or three CDRs predicted from the variable light chain sequence of an antibody which binds to DGCR2 (preferably the same antibody).

In an embodiment, the antigen-binding domain comprises VH CDR 1, 2 and 3 sequences as set forth in SEQ ID NOs: 198, 2 and 3 respectively and VL CDR 1, 2 and 3 sequences as set forth in (a) SEQ ID NOs: 4, 202 and 205 respectively, or (b) SEQ ID NOs: 4, 202 and 206, or the CDRs may contain 1 to 3, or more particularly 1 or 2 amino acid sequence modifications in any of the aforementioned sequences.

In an embodiment, the antigen-binding domain comprises VH CDR1, 2 and 3 sequences as set forth in SEQ ID NOs. 1, 2 and 3 respectively and VL CDR1, 2 and 3 sequences as set forth in SEQ ID NOs. 4, 5 and 6 respectively, or the CDRs may contain 1 to 3, or more particularly 1 or 2 amino acid sequence modifications in any of the aforementioned sequences.

More particularly, in such an embodiment the antigen binding domain of the CAR comprises a VH domain comprising the sequence as set forth in SEQ ID NO: 55, or a sequence having at least 70% sequence identity thereto, and a VL domain comprising the sequence as set forth in SEQ ID NO: 56, or a sequence having at least 70% sequence identity thereto.

For example, in such an embodiment, the antigen binding domain of the CAR comprises a VH domain comprising the sequence encoded by SEQ ID NO: 73, or a sequence having at least 70% identity to the sequence encoded by SEQ ID NO: 73, and a VL domain comprising the sequence encoded by SEQ ID NO: 74, or a sequence having at least 70% identity to the sequence encoded by SEQ ID NO: 74.

In an embodiment, the antigen-binding domain comprises VH CDR 1, 2 and 3 sequences as set forth in SEQ ID NOs: 198, 8 and 9 respectively and VL CDR 1, 2 and 3 sequences as set forth in SEQ ID NOs: 201, 204 and 12 respectively, or the CDRs may contain 1 to 3, or more particularly 1 or 2 amino acid sequence modifications in any of the aforementioned sequences.

In an embodiment, the antigen-binding domain comprises VH CDR1, 2 and 3 sequences as set forth in SEQ ID NOs. 7, 8 and 9 respectively and VL CDR1, 2 and 3 sequences as set forth in SEQ ID NOs. 10, 11 and 12 respectively, or the CDRs may contain 1 to 3, or more particularly 1 or 2 amino acid sequence modifications in any of the aforementioned sequences.

More particularly, in such an embodiment the antigen binding domain of the CAR comprises a VH domain comprising the sequence as set forth in SEQ ID NO: 57, or a sequence having at least 70% sequence identity thereto, and a VL domain comprising the sequence as set forth in SEQ ID NO: 58, or a sequence having at least 70% sequence identity thereto.

For example, in such an embodiment, the antigen binding domain of the CAR comprises a VH domain comprising the sequence encoded by SEQ ID NO: 75, or a sequence having at least 70% identity to the sequence encoded by SEQ ID NO: 75, and a VL domain comprising the sequence encoded by SEQ ID NO: 76, or a sequence having at least 70% identity to the sequence encoded by SEQ ID NO: 76.

In an embodiment, the antigen-binding domain comprises VH CDR 1, 2 and 3 sequences as set forth in SEQ ID NOs: 13, 14 and 15 respectively and VL CDR 1, 2 and 3 sequences as set forth in SEQ ID NOs: 16, 202 and 206 respectively, or the CDRs may contain 1 to 3, or more particularly 1 or 2 amino acid sequence modifications in any of the aforementioned sequences.

In an embodiment, the antigen-binding domain comprises VH CDR1, 2 and 3 sequences as set forth in SEQ ID NOs. 13, 14 and 15 respectively and VL CDR1, 2 and 3 sequences as set forth in SEQ ID NOs. 16, 17 and 18 respectively, or the CDRs may contain 1 to 3, or more particularly 1 or 2 amino acid sequence modifications in any of the aforementioned sequences.

More particularly, in such an embodiment the antigen binding domain of the CAR comprises a VH domain comprising the sequence as set forth in SEQ ID NO: 59, or a sequence having at least 70% sequence identity thereto, and a VL domain comprising the sequence as set forth in SEQ ID NO: 60, or a sequence having at least 70% sequence identity thereto.

For example, in such an embodiment, the antigen binding domain of the CAR comprises a VH domain comprising the sequence encoded by SEQ ID NO: 77, or a sequence having at least 70% identity to the sequence encoded by SEQ ID NO: 77, and a VL domain comprising the sequence encoded by SEQ ID NO: 78, or a sequence having at least 70% identity to the sequence encoded by SEQ ID NO: 78.

In an embodiment, the antigen-binding domain comprises VH CDR1, 2 and 3 sequences as set forth in SEQ ID NOs. 19, 20 and 21 respectively and VL CDR1, 2 and 3 sequences as set forth in SEQ ID NOs. 22, 23 and 24 respectively, or the CDRs may contain 1 to 3, or more particularly 1 or 2 amino acid sequence modifications in any of the aforementioned sequences.

More particularly, in such an embodiment the antigen binding domain of the CAR comprises a VH domain comprising the sequence as set forth in SEQ ID NO: 61, or a sequence having at least 70% sequence identity thereto, and a VL domain comprising the sequence as set forth in SEQ ID NO: 62, or a sequence having at least 70% sequence identity thereto.

For example, in such an embodiment, the antigen binding domain of the CAR comprises a VH domain comprising the sequence encoded by SEQ ID NO: 79, or a sequence having at least 70% identity to the sequence encoded by SEQ ID NO: 79, and a VL domain comprising the sequence encoded by SEQ ID NO: 80, or a sequence having at least 70% identity to the sequence encoded by SEQ ID NO: 80.

In an embodiment, the antigen-binding domain comprises VH CDR 1, 2 and 3 sequences as set forth in SEQ ID NOs: 198, 26 and 27 respectively and VL CDR 1, 2 and 3 sequences as set forth in SEQ ID NOs: 28, 202 and 30 respectively, or the CDRs may contain 1 to 3, or more particularly 1 or 2 amino acid sequence modifications in any of the aforementioned sequences.

In an embodiment, the antigen-binding domain comprises VH CDR1, 2 and 3 sequences as set forth in SEQ ID NOs. 25, 26 and 27 respectively and VL CDR1, 2 and 3 sequences as set forth in SEQ ID NOs. 28, 29 and 30 respectively, or the CDRs may contain 1 to 3, or more particularly 1 or 2 amino acid sequence modifications in any of the aforementioned sequences.

More particularly, in such an embodiment the antigen binding domain of the CAR comprises a VH domain comprising the sequence as set forth in SEQ ID NO: 63, or a sequence having at least 70% sequence identity thereto, and a VL domain comprising the sequence as set forth in SEQ ID NO: 64, or a sequence having at least 70% sequence identity thereto.

For example, in such an embodiment, the antigen binding domain of the CAR comprises a VH domain comprising the sequence encoded by SEQ ID NO: 81, or a sequence having at least 70% identity to the sequence encoded by SEQ ID NO: 81, and a VL domain comprising the sequence encoded by SEQ ID NO: 82, or a sequence having at least 70% identity to the sequence encoded by SEQ ID NO: 82.

In an embodiment, the antigen-binding domain comprises VH CDR1, 2 and 3 sequences set forth in SEQ ID NOs: 200, 32 and 33 respectively and VL CDR1, 2 and 3 sequences set forth in SEQ ID NOs: 201, 203 and 36 respectively, or the CDRs may contain 1 to 3, or more particularly 1 or 2 amino acid sequence modifications in any of the aforementioned sequences.

In an embodiment, the antigen-binding domain comprises VH CDR1, 2 and 3 sequences as set forth in SEQ ID NOs. 31, 32 and 33 respectively and VL CDR1, 2 and 3 sequences as set forth in SEQ ID NOs. 34, 35 and 36 respectively, or the CDRs may contain 1 to 3, or more particularly 1 or 2 amino acid sequence modifications in any of the aforementioned sequences.

More particularly, in such an embodiment the antigen binding domain of the CAR comprises a VH domain comprising the sequence as set forth in SEQ ID NO: 65, or a sequence having at least 70% sequence identity thereto, and a VL domain comprising the sequence as set forth in SEQ ID NO: 66, or a sequence having at least 70% sequence identity thereto.

For example, in such an embodiment, the antigen binding domain of the CAR comprises a VH domain comprising the sequence encoded by SEQ ID NO: 83, or a sequence having at least 70% identity to the sequence encoded by SEQ ID NO: 83, and a VL domain comprising the sequence encoded by SEQ ID NO: 84, or a sequence having at least 70% identity to the sequence encoded by SEQ ID NO: 84.

In an embodiment, the antigen-binding domain comprises VH CDR1, 2 and 3 sequences set forth in SEQ ID NOs: 199, 38 and 39 respectively and VL CDR1, 2 and 3 sequences set forth in SEQ ID NOs: 40, 41 and 42 respectively, or the CDRs may contain 1 to 3, or more particularly 1 or 2 amino acid sequence modifications in any of the aforementioned sequences.

In an embodiment, the antigen-binding domain comprises VH CDR1, 2 and 3 sequences as set forth in SEQ ID NOs. 37, 38 and 39 respectively and VL CDR1, 2 and 3 sequences as set forth in SEQ ID NOs. 40, 41 and 42 respectively, or the CDRs may contain 1 to 3, or more particularly 1 or 2 amino acid sequence modifications in any of the aforementioned sequences.

More particularly, in such an embodiment the antigen binding domain of the CAR comprises a VH domain comprising the sequence as set forth in SEQ ID NO: 67, or a sequence having at least 70% sequence identity thereto, and a VL domain comprising the sequence as set forth in SEQ ID NO: 68, or a sequence having at least 70% sequence identity thereto.

For example, in such an embodiment, the antigen binding domain of the CAR comprises a VH domain comprising the sequence encoded by SEQ ID NO: 85, or a sequence having at least 70% identity to the sequence encoded by SEQ ID NO: 85, and a VL domain comprising the sequence encoded by SEQ ID NO: 86, or a sequence having at least 70% identity to the sequence encoded by SEQ ID NO: 86.

In an embodiment, the antigen-binding domain comprises VH CDR1, 2 and 3 sequences set forth in (a) SEQ ID NOs: 198, 44 and 45 respectively or (b) SEQ ID NOs: 199, 44 and 45 or (c) SEQ ID NOs: 200, 44 and 45, and VL CDR1, 2 and 3 sequences set forth in (a) SEQ ID NOs: 46, 203 and 48 respectively or (b) SEQ ID NOs: 46, 204 and 48 respectively, or the CDRs may contain 1 to 3, or more particularly 1 or 2 amino acid sequence modifications in any of the aforementioned sequences.

In an embodiment, the antigen-binding domain comprises VH CDR1, 2 and 3 sequences as set forth in SEQ ID NOs. 43, 44 and 45 respectively and VL CDR1, 2 and 3 sequences as set forth in SEQ ID NOs. 46, 47 and 48 respectively, or the CDRs may contain 1 to 3, or more particularly 1 or 2 amino acid sequence modifications in any of the aforementioned sequences.

More particularly, in such an embodiment the antigen binding domain of the CAR comprises a VH domain comprising the sequence as set forth in SEQ ID NO: 69, or a sequence having at least 70% sequence identity thereto, and a VL domain comprising the sequence as set forth in SEQ ID NO: 70, or a sequence having at least 70% sequence identity thereto.

For example, in such an embodiment, the antigen binding domain of the CAR comprises a VH domain comprising the sequence encoded by SEQ ID NO: 87, or a sequence having at least 70% identity to the sequence encoded by SEQ ID NO: 87, and a VL domain comprising the sequence encoded by SEQ ID NO: 88, or a sequence having at least 70% identity to the sequence encoded by SEQ ID NO: 88.

In an embodiment, the antigen-binding domain comprises VH CDR1, 2 and 3 sequences set forth in SEQ ID NOs: 198, 50 and 51 respectively and VL CDR1, 2 and 3 sequences set forth in SEQ ID NOs: 52, 202 and 205 respectively, or the CDRs may contain 1 to 3, or more particularly 1 or 2 amino acid sequence modifications in any of the aforementioned sequences.

In an embodiment, the antigen-binding domain comprises VH CDR1, 2 and 3 sequences as set forth in SEQ ID NOs. 49, 50 and 51 respectively and VL CDR1, 2 and 3 sequences as set forth in SEQ ID NOs. 52, 53 and 54 respectively, or the CDRs may contain 1 to 3, or more particularly 1 or 2 amino acid sequence modifications in any of the aforementioned sequences.

More particularly, in such an embodiment the antigen binding domain of the CAR comprises a VH domain comprising the sequence as set forth in SEQ ID NO: 71, or a sequence having at least 70% sequence identity thereto, and a VL domain comprising the sequence as set forth in SEQ ID NO: 72, or a sequence having at least 70% sequence identity thereto.

For example, in such an embodiment, the antigen binding domain of the CAR comprises a VH domain comprising the sequence encoded by SEQ ID NO: 89, or a sequence having at least 70% identity to the sequence encoded by SEQ ID NO: 89, and a VL domain comprising the sequence encoded by SEQ ID NO: 90, or a sequence having at least 70% identity to the sequence encoded by SEQ ID NO: 90.

Where a CDR does contain an amino acid sequence modification, this may be a deletion, addition, or substitution of an amino acid residue of the CDR sequence as set out in the above-mentioned SEQ ID NOs. More particularly, the modification may be an amino acid substitution, for example a conservative amino acid substitution, e.g., as set out above. A longer CDR may tolerate more amino acid residue modifications. In the case of CDRs which are 5 or more, or 7 or more, amino acid residues long, the modifications may be of 0, 1, 2 or 3 residues, e.g. 2 residues. In general, there may be 0, 1, 2, or 3 modifications to any particular CDR sequence. Further, in an embodiment, CDRs 1 and 2 may be modified, and CDR3 may be unmodified. In another embodiment all 3 CDRs may be modified. In another embodiment, the CDRs are not modified.

The antigen binding domain may be in the form of a scFv comprising the VH and VL domain sequences as set out above, in either order, for example VH-VL. The VH and VL sequences may be linked by a linker sequence.

Suitable linkers can be readily selected and can be of any of a suitable length, such as from 1 amino acid (e.g. Gly) to 30 amino acids, e.g. from any one of 2, 3, 4, 5, 6, 7,8, 9, or 10 amino acids to any one of 12, 15, 18, 20, 21, 25, 30 amino acids, for example, 5-30, 5-25, 6-25, 10-15, 12-25, 15 to 25 etc.

Exemplary linkers include glycine polymers (G), glycine-serine polymers, glycine-alanine polymers, alanine-serine polymers, and other flexible linkers known in the art, as discussed above. The linker may comprise 1 or more "GS" domains as discussed above.

The linker sequence may be a flexible linker sequence. Flexible linkers are a category of linker sequences well known and described in the art. Linker sequences are generally known as sequences which may be used to link, or join together, proteins or protein domains, to create for example fusion proteins or chimeric proteins, or multifunctional proteins or polypeptides. They can have different characteristics, and for example may be flexible, rigid or cleavable. Protein linkers are reviewed for example in Chen et al., 2013, Advanced Drug Delivery Reviews 65, 1357-1369, which compares the category of flexible linkers with those of rigid and cleavable linkers. Flexible linkers are also described in Klein et al., 2014, Protein Engineering Design and Selection, 27(10), 325-330; van Rosmalen et al., 2017, Biochemistry, 56,6565-6574; and Chichili et al., 2013, Protein Science, 22, 153-167.

A flexible linker is a linker which allows a degree of movement between the domains, or components, which are linked. They are generally composed of small non-polar (e.g. Gly) or polar (e.g. Ser or Thr) amino acid residues. The small size of the amino acids provides flexibility and allows for mobility of the connected parts (domains or components). The incorporation of polar amino acids can maintain the stability of the linker in aqueous environments by forming hydrogen bonds with water molecules.

The most commonly used flexible linkers have sequences primarily composed of Ser and Gly residues (so-called "GS linkers"). However, many other flexible linkers have also been described (see Chen et al,. 2013, supra, for example), which may contain additional amino acids such as Thr and/or Ala, and/or Lys and/or Glu which may improve solubility. Any flexible linker known and reported in the art may be used.

Although the length of the linker is not critical, it may in some embodiments be desirable to have a shorter linker sequence. For example, the linker sequence may have a length of no more than 25, preferably no more than 24, 23, 22 or 21 amino acids.

In other embodiments, a longer linker sequence may be desired, for example composed of, or comprising, multiple repeats of a GS domain.

In some embodiments the linker may be from any one of 2, 3, 4, 5 or 6 to any one of 24, 23, 22 or 21 amino acids in length. In other embodiments it may be from any one of 2, 3, 4, 5 or 6 to any one of 21, 20, 19, 18, 17, 16, or 15 amino acids in length. In other embodiments it may be intermediate between these ranges, from example from 6 to 21, 6 to 20, 7 to 20, 8-20, 9-20, 10-20, 8-18, 9-18, 10-18, 9-17, 10-17, 9-16, 10-16 etc. It may accordingly be in a range made up from any of the integers listed above.

The use of GS linkers, or more particularly GS ("Gly-Ser") domains in linkers, may allow the length of the linker readily to be varied by varying the number of GS domain repeats, and so such linkers represent one preferred class of linkers. However, flexible linkers are not limited to those based on "GS" repeats, and other linkers comprising Ser and Gly residues dispersed throughout the linker sequence have been reported, including in Chen et al., supra.

Accordingly, in one embodiment the linker sequence may comprise at least 40% Gly or Gly and Ser residues.

In another embodiment, the linker sequence may comprise Ser and/or Gly residues, and no more than 15 other amino acid residues, preferably no more than 14, 13, 12, 11, 10, 9, 8, 6, 7, 5, or 4 other amino acid residues. It will be understood than an "other" amino acid residue may be any amino acid which is not Ser or Gly.

Pro residues in linkers tend to confer rigidity and so in one embodiment the linker sequence does not comprise any Pro residues. However, this is not absolute, as depending on the sequence context, a flexible linker sequence may contain one or more Pro residues.

In one preferred embodiment, the linker sequence comprises at least one Gly-Ser domain composed solely of Ser and Gly residues. In such an embodiment, the linker may contain no more than 15 other amino acid residues, preferably no more than 14, 13, 12, 11, 10, 9, 8, 6, 7, 5, or 4 other amino acid residues.

The Gly-Ser domain may have the formula:

(S)q-[(G)m-(S)m]n-(G)p

wherein q is 0 or 1; m is an integer from 1-8; n is an integer of at least 1 (e.g. from 1 to 8, or more particularly 1 to 6); and p is 0 or an integer from 1 to 3.

More particularly, the Gly-Ser domain may have the formula:
(i) S-[(G)m-S]n;
(ii) [(G)m-S]n; or
(iii) [(G)m-S]n-(G)p
wherein m is an integer from 2-8 (for example 3-4); n is an integer of at least 1 (for example from 1 to 8, or more particularly 1 to 6); and p is 0 or an integer from 1 to 3.

In a representative example, the Gly-Ser domain may have the formula:

S-[G-G-G-G-S]n

wherein n is an integer of at least one (preferably 1 to 8, or 1-6, 1-5, 1-4, or 1-3). In the formula above, the sequence GGGGS is SEQ ID NO: 112.

A representative sequence GGGS is shown in SEQ ID NO: 109.

A linker sequence may be composed solely of, or may consist of, one or more Gly-Ser domains as described or defined above. However, as noted above, in another embodiment, the linker sequence may comprise one or more Gly-Ser domains, and additional amino acids. The additional amino acids may be at one or both ends of a Gly-Ser domain, or at one or both ends of a stretch of repeating Gly-Ser domains. Thus, the additional amino acid, which may be other amino acids, may lie at one or both ends of the linker sequence, e.g. they may flank the Gly-Ser domain(s). In other embodiments, the additional amino acids may lie between Gly-Ser domains. For example, two Gly-Ser domains may flank a stretch of other amino acids in the linker sequence. Further, as also noted above, in other linkers, GS domains need not be repeated, and G and/or S residues, or a short domain such as GS, may simply be distributed along the length or the sequence.

Representative exemplary linker sequences are listed below:
ETSGGGGSRL (SEQ ID NO. 110)
SGGGGSGGGGSGGGGS ((SEQ ID NO. 111)
S(GGGGS)₁₋₅ (where GGGGS is SEQ ID NO. 112)
(GGGGS)₁₋₅ (where GGGGS is SEQ ID NO. 112)
S(GGGS)₁₋₅ (where GGGS is SEQ ID NO. 109)
(GGGS)₁₋₅ (where GGGS is SEQ ID NO. 109)
S(GGGGGS)₁₋₅ (where GGGGGS is SEQ ID NO. 113)
(GGGGGS)₁₋₅ (where GGGGGS is SEQ ID NO. 113)
S(GGGGGGS)₁₋₅ (where GGGGGGS is SEQ ID NO. 114)
(GGGGGGS)₁₋₅ (where GGGGGGS is SEQ ID NO. 114)
GGGGSGGGGSGGGGS (SEQ ID NO. 115)
GGGGG (SEQ ID NO. 116)
GGGGSGGGGS (SEQ ID NO. 117)
GGGGSGGGGSGGGGSGGGGS (SEQ ID NO. 118)
GGGGGG (SEQ ID NO. 119)
G₆ (SEQ ID NO. 120)
G₈ (SEQ ID NO. 121)
KESGSVSSEQLAQFRSLD (SEQ ID NO. 122)
EGKSSGSGSESKST (SEQ ID NO. 123)
GSAGSAAGSGEF (SEQ ID NO.124)
SGGGGSAGSAAGSGEF (SEQ ID NO. 125)
SGGGLLLLLLLLGGGS (SEQ ID NO. 126)
SGGGAAAAAAAAGGGS (SEQ ID NO. 127)
SGGGAAAAAAAAAAAAAAAAGGGS (SEQ ID NO. 128)
SGALGGLALAGLLLAGLGLGAAGS (SEQ ID NO. 129)
SLSLSPGGGGGPAR (SEQ ID NO. 130)
SLSLSPGGGGGPARSLSLSPGGGGG (SEQ ID NO. 131)
GSSGSS (SEQ ID NO. 132)
GSSSSSS (SEQ ID NO. 133)
GGSSSS (SEQ ID NO. 134)
GSSSSS (SEQ ID NO. 135)
SGGGGS (SEQ ID NO. 136).

In certain embodiments, the linker has the sequence (GGGGS)s (SEQ ID NO: 115).

Although the linker sequences of the polypeptides of the invention, as defined above, are flexible sequences, the present disclosure includes also other polypeptides, including those which comprise linkers which are not flexible, and/or which do not meet the definitions and requirements set out above.

A further example of a linker that may be used to join the VH and VL domains is KLEEGEFSEARV (SEQ ID NO: 172) or a sequence having at least about 60% identity thereto, for example a sequence having at least about 65% or at least about 70% or at least about 75% or at least about 80% or at least about 85% or at least about 90% or at least about 95% identity thereto. Alternatively, the linker may have the sequence of SEQ ID NO: 172 or a sequence that differs by no more than 6, for example no more than 5 or no more than 4 or no more than 3 or no more than 2 or no more than 1 amino acid. In particular, this linker may be truncated or elongated at the N terminus and/or the C terminus by one or more amino acids (e.g. see Schmiedl A. et al (Protein Eng. 2000 Oct; 13(10): 725-34 where this linker is shortened by one amino acid at each terminus compared to SEQ ID NO: 172). In order to improve the stability of this linker and to delete the two putative trypsin cleavage sites (lysine and arginine), the linker may be modified by exchanging lysine (K) against isoleucine (I), arginine (R) against glycine (G) and valine (V) against cysteine (C). The resulting linker has the amino acid sequence ILEEGEFSEAGC (SEQ ID NO: 173). Any peptide linker carrying the consensus amino acid sequence X₁LEEGEFSEAX₂X₃ (SEQ ID NO: 174) wherein X₁ is K or I, X₂ is R or G and X₃ is V or C, may also be used.

Accordingly in one embodiment the antigen binding domain may comprise, or consist of, a VH sequence as set forth in SEQ ID NO: 55 linked via a linker of sequence (X)n, where X is any amino acid and n is an integer of between 15 and 25, to the VL sequence of SEQ ID NO: 56.

Accordingly in one embodiment the antigen binding domain may comprise, or consist of, a VH sequence as set forth in SEQ ID NO: 57 linked via a linker of sequence (X)n, where X is any amino acid and n is an integer of between 15 and 25, to the VL sequence of SEQ ID NO: 58.

Accordingly in one embodiment the antigen binding domain may comprise, or consist of, a VH sequence as set forth in SEQ ID NO: 59 linked via a linker of sequence (X)n, where X is any amino acid and n is an integer of between 15 and 25, to the VL sequence of SEQ ID NO: 60.

Accordingly in one embodiment the antigen binding domain may comprise, or consist of, a VH sequence as set forth in SEQ ID NO: 61 linked via a linker of sequence (X)n, where X is any amino acid and n is an integer of between 15 and 25, to the VL sequence of SEQ ID NO: 62.

Accordingly in one embodiment the antigen binding domain may comprise, or consist of, a VH sequence as set forth in SEQ ID NO: 63 linked via a linker of sequence (X)n, where X is any amino acid and n is an integer of between 15 and 25, to the VL sequence of SEQ ID NO: 64.

Accordingly in one embodiment the antigen binding domain may comprise, or consist of, a VH sequence as set forth in SEQ ID NO: 65 linked via a linker of sequence (X)n, where X is any amino acid and n is an integer of between 15 and 25, to the VL sequence of SEQ ID NO: 66.

Accordingly in one embodiment the antigen binding domain may comprise, or consist of, a VH sequence as set forth in SEQ ID NO: 67 linked via a linker of sequence (X)n, where X is any amino acid and n is an integer of between 15 and 25, to the VL sequence of SEQ ID NO: 68.

Accordingly in one embodiment the antigen binding domain may comprise, or consist of, a VH sequence as set forth in SEQ ID NO: 69 linked via a linker of sequence (X)n, where X is any amino acid and n is an integer of between 15 and 25, to the VL sequence of SEQ ID NO: 70.

Accordingly in one embodiment the antigen binding domain may comprise, or consist of, a VH sequence as set forth in SEQ ID NO: 71 linked via a linker of sequence (X)n, where X is any amino acid and n is an integer of between 15 and 25, to the VL sequence of SEQ ID NO: 72.

In this respect, in one embodiment, the antigen binding domain of the CAR comprises or consists of the sequence as set forth in SEQ ID NO: 91 or a sequence having at least 80% identity thereto.

In another embodiment, the antigen binding domain of the CAR comprises or consists of the sequence as set forth in SEQ ID NO: 92 or a sequence having at least 80% identity thereto.

In a further embodiment, the antigen binding domain of the CAR comprises or consists of the sequence as set forth in SEQ ID NO: 93 or a sequence having at least 80% identity thereto.

In a further embodiment, the antigen binding domain of the CAR comprises or consists of the sequence as set forth in SEQ ID NO: 94 or a sequence having at least 80% identity thereto.

In a further embodiment, the antigen binding domain of the CAR comprises or consists of the sequence as set forth in SEQ ID NO: 95 or a sequence having at least 80% identity thereto.

In a further embodiment, the antigen binding domain of the CAR comprises or consists of the sequence as set forth in SEQ ID NO: 96 or a sequence having at least 80% identity thereto.

In a further embodiment, the antigen binding domain of the CAR comprises or consists of the sequence as set forth in SEQ ID NO: 97 or a sequence having at least 80% identity thereto.

In a further embodiment, the antigen binding domain of the CAR comprises or consists of the sequence as set forth in SEQ ID NO: 98 or a sequence having at least 80% identity thereto.

In a further embodiment, the antigen binding domain of the CAR comprises or consists of the sequence as set forth in SEQ ID NO: 99 or a sequence having at least 80% identity thereto.

In one embodiment, the antigen binding domain of the CAR comprises or consists of the amino acid sequence encoded by a sequence as set forth in SEQ ID NO: 100 or an amino acid sequence having at least 80% identity to the amino acid sequence encoded by the sequence set forth in SEQ ID NO: 100. For example, the antigen binding domain of the CAR may comprise or consist of the amino acid sequence encoded by the sequence remaining after the sequence encoding the signal sequence and/or any tag sequence (e.g. myc tag and/or his-tag) is removed from SEQ ID NO: 100 or variant thereof.

In one embodiment, the antigen binding domain of the CAR comprises or consists of the amino acid sequence encoded by a sequence as set forth in SEQ ID NO: 101 or an amino acid sequence having at least 80% identity to the amino acid sequence encoded by the sequence set forth in SEQ ID NO: 101. For example, the antigen binding domain of the CAR may comprise or consist of the amino acid sequence encoded by the sequence remaining after the sequence encoding the signal sequence and/or any tag sequence (e.g. myc tag and/or his-tag) is removed from SEQ ID NO: 101 or variant thereof.

In one embodiment, the antigen binding domain of the CAR comprises or consists of the amino acid sequence encoded by a sequence as set forth in SEQ ID NO: 102 or an amino acid sequence having at least 80% identity to the amino acid sequence encoded by the sequence set forth in SEQ ID NO: 102. For example, the antigen binding domain of the CAR may comprise or consist of the amino acid sequence encoded by the sequence remaining after the sequence encoding the signal sequence and/or any tag sequence (e.g. myc tag and/or his-tag) is removed from SEQ ID NO: 102 or variant thereof.

In one embodiment, the antigen binding domain of the CAR comprises or consists of the amino acid sequence encoded by a sequence as set forth in SEQ ID NO: 103 or an amino acid sequence having at least 80% identity to the amino acid sequence encoded by the sequence set forth in SEQ ID NO: 103. For example, the antigen binding domain of the CAR may comprise or consist of the amino acid sequence encoded by the sequence remaining after the sequence encoding the signal sequence and/or any tag sequence (e.g. myc tag and/or his-tag) is removed from SEQ ID NO: 103 or variant thereof.

In one embodiment, the antigen binding domain of the CAR comprises or consists of the amino acid sequence encoded by a sequence as set forth in SEQ ID NO: 104 or an amino acid sequence having at least 80% identity to the amino acid sequence encoded by the sequence set forth in SEQ ID NO: 104. For example, the antigen binding domain of the CAR may comprise or consist of the amino acid sequence encoded by the sequence remaining after the sequence encoding the signal sequence and/or any tag sequence (e.g. myc tag and/or his-tag) is removed from SEQ ID NO: 104 or variant thereof.

In one embodiment, the antigen binding domain of the CAR comprises or consists of the amino acid sequence encoded by a sequence as set forth in SEQ ID NO: 105 or an amino acid sequence having at least 80% identity to the amino acid sequence encoded by the sequence set forth in SEQ ID NO: 105. For example, the antigen binding domain of the CAR may comprise or consist of the amino acid sequence encoded by the sequence remaining after the sequence encoding the signal sequence and/or any tag sequence (e.g. myc tag and/or his-tag) is removed from SEQ ID NO: 105 or variant thereof.

In one embodiment, the antigen binding domain of the CAR comprises or consists of the amino acid sequence encoded by a sequence as set forth in SEQ ID NO: 106 or an amino acid sequence having at least 80% identity to the amino acid sequence encoded by the sequence set forth in SEQ ID NO: 106. For example, the antigen binding domain of the CAR may comprise or consist of the amino acid sequence encoded by the sequence remaining after the sequence encoding the signal sequence and/or any tag sequence (e.g. myc tag and/or his-tag) is removed from SEQ ID NO: 106 or variant thereof.

In one embodiment, the antigen binding domain of the CAR comprises or consists of the amino acid sequence encoded by a sequence as set forth in SEQ ID NO: 107 or an amino acid sequence having at least 80% identity to the amino acid sequence encoded by the sequence set forth in SEQ ID NO: 107. For example, the antigen binding domain of the CAR may comprise or consist of the amino acid sequence encoded by the sequence remaining after the sequence encoding the signal sequence and/or any tag sequence (e.g. myc tag and/or his-tag) is removed from SEQ ID NO: 107 or variant thereof.

In one embodiment, the antigen binding domain of the CAR comprises or consists of the amino acid sequence encoded by a sequence as set forth in SEQ ID NO: 108 or an amino acid sequence having at least 80% identity to the amino acid sequence encoded by the sequence set forth in SEQ ID NO: 108. For example, the antigen binding domain of the CAR may comprise or consist of the amino acid sequence encoded by the sequence remaining after the sequence encoding the signal sequence and/or any tag sequence (e.g. myc tag and/or his-tag) is removed from SEQ ID NO: 108 or variant thereof.

In a further embodiment, the CAR construct may comprise, or consist of, a sequence as set forth in any one of SEQ ID Nos: 178 to 195, or a variant thereof, e.g., a sequence having at least 80% identity thereto.

The variant sequences disclosed and described herein, including the variant CAR, VH, VL and antigen binding domain sequences, may have at least 75, 80, 85, 90, 92, 95, 96, 97, 98, or 99% sequence identity to the specified SEQ ID NOs.

The CAR also preferably comprises a hinge domain to hold the extracellular domain, particularly the antigen binding domain, away from the cell surface, and further comprises a transmembrane domain. The hinge and transmembrane domains may comprise the hinge and transmembrane sequences from any protein which has a hinge domain and/or a transmembrane domain, including any of the type I, type II or type III transmembrane proteins. The hinge domain may be selected from the hinge regions of CD28, CD8alpha, CD4, CD7, CH2CH3, an immunoglobulin, or a part or variant thereof. Typically, the hinge may be derived from CD8, particularly, CD8alpha, or from CH2CH3. In one embodiment, the hinge may comprise one or more cysteine residues e.g., to allow disulphide bonding. For example, the CD8 hinge may comprise one or more cysteine residues e.g., one cysteine residue, two cysteine residues or three cysteine residues.

The transmembrane domain of the CAR may also comprise an artificial hydrophobic sequence. The transmembrane domains of the CAR may be selected so as not to dimerize. Additional transmembrane domains will be apparent to those of skill in the art. Examples of transmembrane (TM) regions used in CAR constructs are: 1) The CD28 TM region (Pule et al, Mol Ther, 2005, Nov;12(5):933-41; Brentjens et al, CCR, 2007, Sep 15;13(18 Pt 1):5426-35; Casucci et al, Blood, 2013, Nov 14;122(20):3461-72.); 2) The OX40 TM region (Pule et al, Mol Ther, 2005, Nov;12(5):933-41); 3) The 4-1BB TM region (Brentjens et al, CCR, 2007, Sep 15;13(18 Pt 1):5426-35); 4). The CD3 zeta TM region (Pule et al, Mol Ther, 2005, Nov;12(5):933-41; Savoldo B, Blood, 2009, Jun 18;113(25):6392-402.); 5) The CD8α TM region (Maher et al, Nat Biotechnol, 2002, Jan;20(1):70-5.; Imai C, Leukemia, 2004, Apr;18(4):676-84; Brentjens et al, CCR, 2007, Sep 15;13(18 Pt 1):5426-35; Milone et al, Mol Ther, 2009, Aug;17(8):1453-64.). Other transmembrane domains which may be used include those from ICOS, CD4, CD45, CD9, CD16, CD22, CD33, CD64, CD80, CD86, or CD154. Preferably, the transmembrane domain may be derived from CD4, CD28, or CD8α.

In one embodiment, the CAR may not comprise a dimerisation domain which binds to a regulating molecule. A regulating molecule is any molecule that can bind to at least one dimerisation domain in a CAR and can prevent the interaction, or cause the interaction, of a pair of dimerisation domains. Examples of regulating molecules include soluble proteins (e.g., cytokines, TGF-beta, VEGF) or small molecules. In a further embodiment, when dimerisation with other CAR molecules occurs, it may not be controlled. In another embodiment, monovalent binding of the CAR to an antigen may allow activation of the cell in which the CAR is expressed.

A hinge domain may conveniently be obtained from the same protein as the transmembrane domain. In one embodiment, where the transmembrane domain is derived from the CD8α transmembrane domain, the hinge domain is derived from the CD8α hinge domain.

Alternatively, the hinge domain may be obtained from a different protein to the transmembrane domain. For example, the hinge domain may be derived from the CH2CH3 hinge domain and the transmembrane domain may be derived from the CD28 transmembrane domain.

For example, the hinge domain may be derived from the CD8α hinge domain and may comprise the amino acid sequence shown in SEQ ID NO: 176, or a variant thereof which is at least 80% identical to SEQ ID NO; 176. Suitably, the variant may be at least 85, 90, 95, 97, 98 or 99% identical to SEQ ID NO: 176. An example of a modified CD8α hinge domain is shown in SEQ ID NO: 140.

For example, the hinge domain may comprise the amino acid sequence shown in SEQ ID NO: 196 or encoded by the nucleotide sequence shown in SEQ ID NO: 197, or a variant thereof which is at least 80% identical to SEQ ID NO: 196 or SEQ ID NO: 197. Suitably, the variant may be at least 85, 90, 95, 97, 98 or 99% identical to SEQ ID NO: 196 or SEQ ID NO: 197.

For example, the transmembrane domain may be derived from the CD8α transmembrane domain and may comprise the amino acid sequence shown as SEQ ID NO: 137 which represents amino acids 183 to 203 of human CD8α, or a variant which is at least 80% identical to SEQ ID NO: 137. Suitably, the variant may be at least 85, 90, 95, 97, 98 or 99% identical to SEQ ID NO: 137.

The CD8α transmembrane domain may be combined with a CD8α hinge domain. In an embodiment, the CAR comprises a combined CD8α hinge and transmembrane domain sequence as shown in SEQ ID NO. 139 or SEQ ID NO: 141, or a variant thereof which has at least 80% sequence identity thereto. The variant may be at least 85, 90, 95, 97, 98 or 99% identical to SEQ ID NO: 139 or SEQ ID NO: 141 respectively. SEQ ID NO. 139 comprises a modified hinge domain comprising 2 amino acid modifications of cysteine residues relative to the wild-type CD8α hinge sequence. The modified CD8α hinge domain sequence is shown in SEQ ID NO. 140. The wildtype CD8α hinge and transmembrane domain sequence is shown in SEQ ID NO. 141. The 6 amino acids at the end of SEQ ID NO. 139 and 141, when present, are not located in the membrane and form part of the endodomain of the CAR. A variant of such hinge sequences having at least 80% sequence identity to SEQ ID NO. 140 or 141 may be used.

For example, the hinge domain may be derived from the CH2CH3 hinge domain and may comprise the sequence shown in SEQ ID NO. 138 or SEQ ID NO. 175 or a variant thereof which is at least 80% identical to SEQ ID NO. 138 or 175 respectively. The variant may be at least 85, 90, 95, 97, 98 or 99% identical to SEQ ID NO. 138 or 175 respectively.

Alternatively, an example of a CD28 hinge and transmembrane sequence which may be used is SEQ ID NO: 142 or a variant thereof which is at least 80% identical to SEQ ID NO: 142. The variant may be at least 85, 90, 95, 97, 98 or 99% identical to SEQ ID NO: 142.

By way of further example, the CAR may comprise a native or modified CD8α hinge domain and a CD28 transmembrane domain, or a CD28 hinge domain and CD8α transmembrane domain, for example based on the sequences given above.

In one embodiment, a CH2CH3 hinge sequence comprising one or more cysteine residues may be used. For example, a CH2CH3 hinge sequence comprising one, two, three, four or more cysteine residues. Other hinge domains which may be used include those from CD4, CD7, or an immunoglobulin, or a part or variant thereof. These hinge domains may comprise one or more cysteine residues, for example one, two, three, four or more cysteine residues.

In one embodiment, the transmembrane domain may be derived from the CD4 transmembrane domain and may comprise the sequence shown in SEQ ID NO: 177 or a variant thereof which is at least 80% identical to SEQ ID NO: 177. The variant may be at least 85, 90, 95, 97, 98 or 99% identical to SEQ ID NO: 177.

The CAR may further comprise a signal (or alternatively termed, leader) sequence which targets it to the endoplasmic reticulum pathway for expression on the cell surface. An illustrative signal/leader sequence is MALPVTALLLPLALLLHAAAP as shown in SEQ ID NO. 143. This comprises a single amino acid substitution compared to the wild type CD8α sequence MALPVTALLLPLALLLHAARP as shown in SEQ ID NO. 144. Either sequence, or a variant sequence having at least 70% sequence identity thereto may be used. For example, a variant sequence may have at least 75, 80, 85, 90, 95, 97, 98 or 99% sequence identity thereto.

The endodomain of a CAR as described herein comprises motifs necessary to transduce the effector function signal and direct a cell expressing the CAR to perform its specialized function upon antigen binding. Particularly, the endodomain may comprise one or more (e.g. two or three) Immunoreceptor tyrosine-based activation motifs (ITAMs), typically comprising the amino acid sequence of YXXL/I, where X can be any amino acid. Examples of intracellular signaling domains include, but are not limited to, ζ chain endodomain of the T-cell receptor or any of its homologs (e.g., η chain, FcεR1γ and β chains, MB1 (Igα) chain, B29 (Igβ) chain, etc.), CD3 polypeptide domains (Δ, δ and ε), syk family tyrosine kinases (Syk, ZAP 70, etc.), src family tyrosine kinases (Lck, Fyn, Lyn, etc.) and other molecules involved in T-cell transduction, such as CD2, CD5 and CD28. The intracellular signaling domain may comprise human CD3 zeta chain endodomain, FcyRIII, FcsRl, cytoplasmic tails of Fc receptors, immunoreceptor tyrosine-based activation motif (ITAM) bearing cytoplasmic receptors or combinations thereof.

Commonly, the intracellular signaling domain comprises the intracellular signaling domain of a human CD3 zeta chain. The sequence of the intracellular signaling domain of human CD3 zeta chain is set out in SEQ ID NO. 145. The CAR may comprise a CD3ζ signalling domain comprising or consisting of a sequence as set out in SEQ ID NO. 145 or a sequence having at least 80, 85, 90, 95, 97, 98 or 99% identity to SEQ ID NO: 145. In an embodiment the signaling domain comprises or consists of SEQ ID NO. 145.

Other signaling domains which may be used include the signaling domains of CD28 or CD27 or variants thereof. Additional intracellular signaling domains will be apparent to those of skill in the art and may be used in connection with alternate embodiments of the invention. In one embodiment, the present CAR may not comprise a costimulatory domain derived from 4-1BB within the endodomain.

The present CAR may comprise a compound endodomain comprising a fusion of the intracellular part of a T-cell co-stimulatory molecule to that of e.g. CD3ζ. Such a compound endodomain may be referred to as a second-generation CAR which can transmit an activating and co-stimulatory signal simultaneously after antigen recognition. The co-stimulatory domain most commonly used is that of CD28. This supplies the most potent co-stimulatory signal - namely immunological signal 2, which triggers T-cell proliferation. The CAR endodomain may also comprise one or more TNF receptor family signalling domain, such as the signalling domain of ICOS, (CD134) OX40, 4-1BB, CD27 or TNFRSF25, or a part or variant thereof, although preferably the CAR may not comprise an endodomain comprising the signalling domains of both CD28 and 4-1BB.

An intracellular signaling domain of CD28 which may be used as a co-stimulatory domain is shown in SEQ ID NO. 147. Illustrative sequences for OX40, 4-1BB, ICOS and TNFRSF25 signalling domains are shown in SEQ ID NO: 148 to 151. The CAR may comprise one or more co-stimulatory domains comprising or consisting of the sequence of any one of SEQ ID NO: 147, 148, 149, 150, 151, or a variant thereof having at least 80, 85, 90, 95, 97, 98 or 99% sequence identity thereto.

In certain embodiments, the transmembrane domain and the intracellular signalling domain derived from a T-cell co-stimulatory molecule may be derived from the same protein. For example, in certain embodiments, the transmembrane domain and intracellular signalling domain derived from a T-cell costimulatory molecule may be derived from CD28. For example, the CAR may comprise a combined CD28 transmembrane and CD28 intracellular signalling domain as shown in SEQ ID NO: 146, or a variant thereof having at least 80% sequence identity thereto. The variant may be at least 85, 90, 95, 97, 98 or 99% identical to SEQ ID NO: 146.

In one embodiment, the CAR comprises a human CD8 hinge domain or a variant thereof and a human CD8 transmembrane domain. Alternatively, or additionally, the CAR comprises an endodomain comprising, consisting essentially of or consisting of a human CD28 co-stimulatory domain and a human CD3 zeta signalling domain.

In one preferred embodiment the CAR comprises a hinge, transmembrane, and intracellular (or endo) domains as follows:
(i) a CH2CH3 hinge sequence comprising or consisting of the sequence as set forth in SEQ ID NO. 138 or SEQ ID NO. 175, or a sequence having at least 80% sequence identity thereto;
(ii) a CD28 transmembrane and co-stimulatory domain comprising or consisting of the sequence as set forth in SEQ ID NO. 146, or a sequence having at least 80% sequence identity thereto;
(iii) a CD3ζ signalling domain comprising or consisting of the sequence as set forth in SEQ ID NO. 145, or a sequence having at least 80% sequence identity thereto.

In an alternative preferred embodiment the CAR comprises a hinge, transmembrane, and intracellular (or endo) domains as follows:
(i) a CD8α hinge and transmembrane domain sequence comprising or consisting of the sequence as set forth in SEQ ID NO. 141, or a sequence having at least 80% sequence identity thereto;
(ii) a CD28 co-stimulatory domain comprising or consisting of the sequence as set forth SEQ ID NO. 147, or a sequence having at least 80% sequence identity thereto;
(iii) a CD3ζ signalling domain comprising or consisting of the sequence as set forth in SEQ ID NO. 145, or a sequence having at least 80% sequence identity thereto.

In a further preferred embodiment the CAR comprises a hinge, transmembrane, and intracellular (or endo) domains as follows:
(i) a CD8α hinge and transmembrane domain sequence comprising or consisting of the sequence as set forth in SEQ ID NO. 139, or a sequence having at least 80% sequence identity thereto;
(ii) a CD28 co-stimulatory domain comprising or consisting of the sequence as set forth in SEQ ID NO. 147, or a sequence having at least 80% sequence identity thereto;
(iii) a CD3ζ signalling domain comprising or consisting of the sequence as set forth in SEQ ID NO. 145, or a sequence having at least 80% sequence identity thereto.

The CAR, as encoded and expressed, may further comprise a leader sequence comprising or consisting of a sequence as set out in SEQ ID NO. 143 or SEQ ID NO: 144, or a sequence having at least 80% sequence identity thereto.

The antigen binding domain of the CAR may comprise or consist of a sequence as set out in SEQ ID NO. 91, 92, 93, 94, 95, 96, 97, 98, 99 or a sequence having at least 80% sequence identity thereto which is capable of binding to DGCR2.

Thus, in its entirety one preferred representative CAR may comprise:
i.a leader sequence comprising or consisting of a sequence as set out in SEQ ID NO. 143 or 144, or a sequence having at least 80% sequence identity thereto;
ii.an antigen binding domain comprising or consisting of a sequence as set out in SEQ ID NO. 91, 92, 93, 94, 95, 96, 97, 98, 99 or a sequence having at least 80% sequence identity thereto;
iii.a CH2CH3 hinge sequence comprising or consisting of the sequence as set forth in SEQ ID NO. 138 or 175, or a sequence having at least 80% sequence identity thereto;
iv.a CD28 transmembrane and co-stimulatory domain comprising or consisting of the sequence as set forth in SEQ ID NO. 146, or a sequence having at least 80% sequence identity thereto;
v.a CD3ζ signalling domain comprising or consisting of the sequence as set forth in SEQ ID NO. 145, or a sequence having at least 80% sequence identity thereto.

An alternative preferred representative CAR may comprise:
i.a leader sequence comprising or consisting of a sequence as set out in SEQ ID NO. 143 or 144, or a sequence having at least 80% sequence identity thereto;
ii.an antigen binding domain comprising or consisting of a sequence as set out in SEQ ID NO. 91, 92, 93, 94, 95, 96, 97, 98, 99 or a sequence having at least 80% sequence identity thereto;
iii.a CD8 hinge and transmembrane domain sequence comprising or consisting of the sequence as set forth in SEQ ID NO. 141 or 139, or a sequence having at least 80% sequence identity thereto;
iv.a CD28 co-stimulatory domain comprising or consisting of the sequence as set forth in SEQ ID NO. 147, or a sequence having at least 80% sequence identity thereto;
v.a CD3ζ signalling domain comprising or consisting of the sequence as set forth in SEQ ID NO. 145, or a sequence having at least 80% sequence identity thereto.

The CAR of the present invention may comprise, or consist of, any one or more of the sequences as set out in SEQ ID NOs. 178 to 195, or a variant thereof having at least about 80% identity thereto. For example, any variant may have at least about 85, 90, 95, 97, 98 or 99% identity to SEQ ID NOs 178 to 195. The CARs of SEQ ID NOs 178 to 195, or variants thereof, may further comprise a signal sequence, for example a signal sequence having the sequence set out in SEQ ID NO: 143 or 144.

The CAR is capable of binding to DGCR2 and of transducing a signal into a cell in which it is expressed.

The cell may express only one type of CAR, i.e., where the cell expresses more than one CAR molecule, the amino acid sequences of each of these expressed molecules are identical to one another.

The endodomain of a CAR herein may contain further domains. For example, the CAR may comprise domains that confer the ability on the CAR to provide a productive IL signal to the cell in which it is expressed in an antigen specific manner, without requiring exogenous IL to be administered. For example, it may comprise a domain comprising a STAT5 association motif, a JAK1 and/or JAK 2 binding motif and optionally a JAK 3 binding motif. In such an embodiment the endodomain may comprise one or more sequences from an endodomain of a cytokine receptor, for example an interleukin receptor (IL) receptor. Such CARs are described in WO2020/044055 (also incorporated herein by reference). Exemplary amino acid sequences derived from IL-2 receptor beta that include STAT5 association motif and JAK binding motifs are provided in SEQ ID NOs: 152-154. The inclusion of such domains confers the ability on the CAR to provide a productive IL signal to the cell in which it is expressed in an antigen-specific manner, without requiring exogenous IL to be administered. For example, IL-2 is important for the survival, proliferation and *in vivo* persistence of Treg cells, but IL-2 levels may frequently be low or impaired in patients needing treatment. A CAR may thus comprise a sequence corresponding to all or part of a β chain endodomain of an IL receptor or a variant thereof, such as the IL2 receptor, optionally in combination with the γ chain endodomain of an IL receptor or a variant thereof, such as the IL2 receptor.

Alternatively or additionally, further nucleic acid sequences or polypeptides may be introduced into the cell or population of cells in order to improve *in vivo* persistence or survival of the cells, for example to provide a productive IL signal to the cell without requiring exogenous IL to be administered. Such IL signal may be constitutive or inducible. Exemplary technologies may, for example, involve the use of engineered or chimeric receptors that can transmit an IL signal without requiring exogenous IL to be administered. For example, inducible engineered receptors such as those described in WO 2018/111834, WO 2019/169290 and WO 2020/264039; or constitutive engineered receptors such as those described in WO 2018/038954, WO 2019/102207, WO 2019/053420, WO 2020/180694 and WO 2017/218850; chimeric cytokine receptors such as those described in WO 2020/183131, WO 2017/029512, WO 2012/138858, WO 2014/172584, WO 2017/068360, WO 2021/023987, WO 2020/180664 and WO 2020/044239; or engineered receptors having a tethered activation molecule such as those described in WO 2017/201432 and WO 2019/183389.

As mentioned above, the cell or cell population of the invention may further comprise additional polypeptides, particularly exogenous polypeptides, such as a FOXP3 and/or safety switch polypeptide. The polypeptides of the present invention, e.g., the CAR, FOXP3 and safety switch, may be encoded by a single nucleic acid molecule. The nucleic acid molecule may comprise nucleotide sequences encoding self-cleavage sequences in between the encoded polypeptides, allowing the polypeptides to be expressed and/or produced as separate, or discrete components. By this it is meant that, although the polypeptides are encoded by a single nucleic acid molecule, through "cleavage" during or after translation at the encoded cleavage sites, they may be expressed or produced as separate polypeptides, and thus at the end of the protein production process in the cell, they may be present in the cell as separate entities, or separate polypeptide chains. Alternatively, additional exogenous polypeptides may be encoded by separate nucleic acid molecules or vectors.

By "discrete" or "separate" polypeptides it is meant that the polypeptides are not linked to one another and are physically distinct. Indeed, following expression, they are located in different, or separate cellular locations. The CAR, FOXP3 and safety switch polypeptide are thus ultimately expressed as single and separate components. The CAR is expressed as a cell surface molecule. The safety switch polypeptide may be expressed inside a cell, or on the cell surface. In a particular embodiment, the safety switch polypeptide and the CAR are expressed on the surface of a cell which is intended for ACT. The FOXP3 is expressed inside the cell, where it can exert its effect as a transcription factor to regulate cell development and/or activity, as described further below.

The safety switch polypeptide provides a cell in or on which it is expressed with a suicide moiety. This is useful as a safety mechanism which allows a cell which has been administered to a subject to be deleted should the need arise, or indeed more generally, according to desire or need, for example once a cell has performed or completed its therapeutic effect.

A suicide moiety possesses an inducible capacity to lead to cellular death, or more generally to elimination or deletion of a cell. An example of a suicide moiety is a suicide protein, encoded by a suicide gene, which may be expressed in or on a cell alongside a desired transgene, in this case the CAR, which when expressed allows the cell to be deleted to turn off expression of the transgene (CAR). A suicide moiety herein is a suicide polypeptide that is a polypeptide that under permissive conditions, namely conditions that are induced or turned on, is able to cause the cell to be deleted.

The suicide moiety may be a polypeptide, or amino acid sequence, which may be activated to perform a cell-deleting activity by an activating agent which is administered to the subject, or which is active to perform a cell-deleting activity in the presence of a substrate which may be administered to a subject. In a particular embodiment, the suicide moiety may represent a target for a separate cell-deleting agent which is administered to the subject. By binding to the suicide moiety, the cell-deleting agent may be targeted to the cell to be deleted. In particular, the suicide moiety may be recognised by an antibody, and binding of the antibody to the safety switch polypeptide, when expressed on the surface of a cell, causes the cell to be eliminated, or deleted.

The suicide moiety may be HSV-TK or iCasp9. However, it is preferred for the suicide moiety to be, or to comprise, an epitope which is recognised by a cell-deleting antibody or other binding molecule capable of eliciting deletion of the cell. In such an embodiment, the safety switch polypeptide is expressed on the surface of a cell.

The term "delete" as used herein in the context of cell deletion is synonymous with "remove" or "ablate" or "eliminate". The term is used to encompass cell killing, or inhibition of cell proliferation, such that the number of cells in the subject may be reduced. 100% complete removal may be desirable but may not necessarily be achieved. Reducing the number of cells, or inhibiting their proliferation, in the subject may be sufficient to have a beneficial effect.

In particular, the suicide moiety may be a CD20 epitope which is recognised by the antibody Rituximab. Thus, in the safety switch polypeptide the suicide moiety may comprise a minimal epitope based on the epitope from CD20 that is recognised by the antibody Rituximab. Biosimilars for Rituximab are available and may be used. A person of skill in the art is readily able to use routine methods to prepare an antibody having the binding specificity of Rituximab using the available amino acid sequences therefor. CAR-cells specific for DGCR2, which also express a safety switch polypeptide comprising this sequence can be selectively killed using the antibody Rituximab, or an antibody having the binding specificity of Rituximab. The safety switch polypeptide is expressed on the cell surface and when the expressed polypeptide is exposed to or contacted with Rituximab, or an antibody with the same binding specificity, death of the cell ensues.

Thus, Rituximab, or an antibody having the binding specificity thereof, may be provided for use in ACT in combination with a cell of the invention. The cell or nucleic acid or vector or construct for production of the cell and the Rituximab or equivalent antibody may be provided in a kit, or as a combination product.

For example, the suicide constructs of WO2013/153391 or WO2021/239812 (both incorporated herein by reference) may be used in a cell or cell population (e.g., Treg or Treg population) as described herein.

The nucleic acid molecule of the present invention may be designed to increase FOXP3 expression in cells (e.g., Tregs) by introducing into the cells a nucleotide sequence encoding FOXP3, which term is synonymous with the term "a FOXP3 polypeptide". The nucleic acid molecule, and constructs and vectors containing it, thus provide a means for increasing FOXP3 in a cell, e.g., in a Treg or a CD4+ cell. As discussed above, a single nucleic acid molecule may encode the CAR of the invention and a FOXP3 polypeptide or the CAR and FOXP3 may be encoded by separate or discrete nucleic acid molecules. Thus, the invention provides a cell comprising a nucleic acid molecule comprising a nucleotide sequence encoding a CAR and a nucleic acid molecule comprising a nucleotide sequence encoding FOXP3, particularly a pluripotent cell (e.g. an iPSC), an HPC cell (e.g. expressing CD34), a CD4+ T cell or a Treg cell.

"FOXP3" is the abbreviated name of the forkhead box P3 protein. FOXP3 is a member of the FOX protein family of transcription factors and functions as a master regulator of the regulatory pathway in the development and function of regulatory T cells. "FOXP3" as used herein encompasses variants, isoforms, and functional fragments of FOXP3.

"Increasing FOXP3 expression" means to increase the levels of FOXP3 mRNA and/or protein in a cell (or population of cells) in comparison to a corresponding cell which has not been modified (or population of cells) by introduction of the nucleic acid molecule, construct or vector. For example, the level of FOXP3 mRNA and/or protein in a cell modified according to the present invention (or a population of such cells) may be increased to at least 1.5-fold, at least 2-fold, at least 5-fold, at least 10-fold, at least 50-fold, at least 100-fold, at least 150-fold greater than the level in a corresponding cell which has not been modified according to the present invention (or population of such cells). Preferably the cell is a Treg or the population of cells is a population of Tregs.

Suitably, the level of FOXP3 mRNA and/or protein in a modified cell (or a population of such cells) may be increased to at least 1.5-fold greater, 2-fold greater, or 5-fold greater than the level in a corresponding cell which has not been so modified (or population of such cells). Preferably the cell is a Treg or the population of cells is a population of Tregs.

Techniques for measuring the levels of specific mRNA and protein are well known in the art. mRNA levels in a population of cells, such as Tregs, may be measured by techniques such as the Affymetrix ebioscience prime flow RNA assay, Northern blotting, serial analysis of gene expression (SAGE) or quantitative polymerase chain reaction (qPCR). Protein levels in a population of cells may be measured by techniques such as flow cytometry, high-performance liquid chromatography (HPLC), liquid chromatography-mass spectrometry (LC/MS), Western blotting or enzyme-linked immunosorbent assay (ELISA).

A "FOXP3 polypeptide" is a polypeptide having FOXP3 activity i.e., a polypeptide able to bind FOXP3 target DNA and function as a transcription factor regulating development and function of Tregs. Particularly, a FOXP3 polypeptide may have the same or similar activity to wildtype FOXP3 (SEQ ID NO. 155), e.g., may have at least 40, 50, 60, 70, 80, 90, 95, 100, 110, 120, 130, 140 or 150% of the activity of the wildtype FOXP3 polypeptide. Thus, a FOXP3 polypeptide encoded by the nucleotide sequence in the nucleic acid, construct or vector described herein may have increased or decreased activity compared to wildtype FOXP3. Techniques for measuring transcription factor activity are well known in the art. For example, transcription factor DNA-binding activity may be measured by ChIP. The transcription regulatory activity of a transcription factor may be measured by quantifying the level of expression of genes which it regulates. Gene expression may be quantified by measuring the levels of mRNA and/or protein produced from the gene using techniques such as Northern blotting, SAGE, qPCR, HPLC, LC/MS, Western blotting or ELISA. Genes regulated by FOXP3 include cytokines such as IL-2, IL-4 and IFN-γ (Siegler et al. Annu. Rev. Immunol. 2006, 24: 209-26, incorporated herein by reference). As discussed in detail below, FOXP3 or a FOXP3 polypeptide includes functional fragments, variants, and isoforms thereof, e.g., of SEQ ID NO. 155.

A "functional fragment of FOXP3" may refer to a portion or region of a FOXP3 polypeptide or a polynucleotide (i.e., nucleotide sequence) encoding a FOXP3 polypeptide that has the same or similar activity to the full-length FOXP3 polypeptide or polynucleotide. The functional fragment may have at least 40%, at least 50%, at least 60%, at least 70%, at least 80%, at least 90%, at least 95%, or 100% of the activity of the full-length FOXP3 polypeptide or polynucleotide. A person skilled in the art would be able to generate functional fragments based on the known structural and functional features of FOXP3. These are described, for instance, in Song, X., et al., 2012. Cell reports, 1(6), pp.665-675; Lopes, J.E., et al., 2006. The Journal of Immunology, 177(5), pp.3133-3142; and Lozano, T., et al, 2013. Frontiers in oncology, 3, p.294. Further, a N and C terminally truncated FOXP3 fragment is described within WO2019/241549 (incorporated herein by reference), for example, having the sequence SEQ ID NO. 156 as discussed below.

A "FOXP3 variant" may include an amino acid sequence or a nucleotide sequence which may be at least 50%, at least 55%, at least 65%, at least 70%, at least 75%, at least 80%, at least 85% or at least 90% identical, preferably at least 95% or at least 97% or at least 99% identical to a FOXP3 polypeptide or a polynucleotide encoding a FOXP3 polypeptide, e.g., to SEQ ID NO. 155. FOXP3 variants may have the same or similar activity to a wildtype FOXP3 polypeptide or polynucleotide, e.g., may have at least 40, 50, 60, 70, 80, 90, 95, 100, 110, 120, 130, 140 or 150% of the activity of a wildtype FOXP3 polypeptide or polynucleotide. A person skilled in the art would be able to generate FOXP3 variants based on the known structural and functional features of FOXP3 and/or using conservative substitutions. FOXP3 variants may have similar or the same turnover time (or degradation rate) within a Treg cell as compared to wildtype FOXP3, e.g., at least 40, 50, 60, 70, 80, 90, 95, 99 or 100% of the turnover time (or degradation rate) of wildtype FOXP3 in a Treg. Some FOXP3 variants may have a reduced turnover time (or degradation rate) as compared to wildtype FOXP3, for example, FOXP3 variants having amino acid substitutions at amino acid 418 and/or 422 of SEQ ID NO. 155, for example S418E and/or S422A, as described in WO2019/241549 (incorporated herein by reference) and are set out in SEQ ID NO.s 157 to 159, which represent the aa418, aa422 and aa418 and aa422 mutants respectively.

Suitably, the FOXP3 polypeptide encoded by a nucleic acid molecule, construct or vector as described herein may comprise or consist of the polypeptide sequence of a human FOXP3, such as UniProtKB accession Q9BZS1 (SEQ ID NO: 155), or a functional fragment or variant thereof.

In some embodiments of the invention, the FOXP3 polypeptide comprises or consists of an amino acid sequence which is at least 70% identical to SEQ ID NO: 155 or a functional fragment thereof. Suitably, the FOXP3 polypeptide comprises or consists of an amino acid sequence which is at least 80%, at least 85%, at least 90%, at least 95%, at least 98% or at least 99% identical to SEQ ID NO: 155 or a functional fragment thereof. In some embodiments, the FOXP3 polypeptide comprises or consists of SEQ ID NO: 155 or a functional fragment thereof.

In some embodiments, as discussed above, the FOXP3 polypeptide may comprise mutations at residues 418 and/or 422 of SEQ ID NO. 155, as set out in SEQ ID NO. 157, SEQ ID NO. 158, or SEQ ID NO. 159.

In some embodiments of the invention, the FOXP3 polypeptide may be truncated at the N and/or C terminal ends, resulting in the production of a functional fragment. Particularly, an N and C terminally truncated functional fragment of FOXP3 may comprise or consist of an amino acid sequence of SEQ ID NO. 156 or a functional variant thereof having at least 80, 85, 90, 95 or 99% identity thereto.

Suitably, the FOXP3 polypeptide may be a variant of SEQ ID NO: 155, for example a natural variant. Suitably, the FOXP3 polypeptide is an isoform of SEQ ID NO: 155. For example, the FOXP3 polypeptide may comprise a deletion of amino acid positions 72-106 relative to SEQ ID NO: 155. Alternatively, the FOXP3 polypeptide may comprise a deletion of amino acid positions 246-272 relative to SEQ ID NO: 155.

Suitably, the FOXP3 polypeptide comprises SEQ ID NO: 160 or a functional fragment thereof. SEQ ID NO: 160 represents an illustrative FOXP3 polypeptide.

Suitably the FOXP3 polypeptide comprises or consists of an amino acid sequence which is at least 70% identical to SEQ ID NO: 160 or a functional fragment thereof. Suitably, the FOXP3 polypeptide comprises an amino acid sequence which is at least 80%, at least 85%, at least 90%, at least 95%, at least 98% or at least 99% identical to SEQ ID NO: 160 or a functional fragment thereof. In some embodiments, the FOXP3 polypeptide comprises or consists of SEQ ID NO: 160 or a functional fragment thereof.

Suitably, the FOXP3 polypeptide may be a variant of SEQ ID NO: 160, for example a natural variant. Suitably, the FOXP3 polypeptide is an isoform of SEQ ID NO: 160 or a functional fragment thereof. For example, the FOXP3 polypeptide may comprise a deletion of amino acid positions 72-106 relative to SEQ ID NO: 160. Alternatively, the FOXP3 polypeptide may comprise a deletion of amino acid positions 246-272 relative to SEQ ID NO: 160.

Suitably, the polynucleotide encoding a FOXP3 polypeptide comprises or consists of a nucleotide sequence set forth in SEQ ID NO: 161, which represents an illustrative FOXP3 nucleotide sequence.

In some embodiments of the invention, the polynucleotide encoding the FOXP3 polypeptide or variant comprises nucleotide sequence which is at least 70% identical to SEQ ID NO: 161 or a fragment thereof which encodes a functional FOXP3 polypeptide. Suitably, the polynucleotide encoding the FOXP3 polypeptide or variant comprises a polynucleotide sequence which is at least 80%, at least 85%, at least 90%, at least 95%, at least 98% or at least 99% identical to SEQ ID NO: 161 or a fragment thereof which encodes a functional FOXP3 polypeptide. In some embodiments of the invention, the polynucleotide encoding the FOXP3 polypeptide or variant comprises or consists of SEQ ID NO: 161 or a fragment thereof which encodes a functional FOXP3 polypeptide.

Suitably, the polynucleotide encoding a FOXP3 polypeptide comprises or consists of a polynucleotide sequence set forth in SEQ ID NO: 162, which represents another illustrative FOXP3 nucleotide.

In some embodiments of the invention, the polynucleotide encoding the FOXP3 polypeptide or variant comprises a nucleotide sequence which is at least 70% identical to SEQ ID NO: 162 or a fragment thereof which encodes a functional FOXP3 polypeptide. Suitably, the polynucleotide encoding the FOXP3 polypeptide or variant comprises a polynucleotide sequence which is at least 80%, at least 85%, at least 90%, at least 95%, at least 98% or at least 99% identical to SEQ ID NO: 162 or a fragment thereof which encodes a functional FOXP3 polypeptide. In some embodiments of the invention, the polynucleotide encoding the FOXP3 polypeptide or variant comprises or consists of SEQ ID NO: 162 or a fragment thereof which encodes a functional FOXP3 polypeptide.

A skilled person will appreciate that FOXP3 expression within a Treg may be increased indirectly by introducing a polynucleotide into the cell which encodes a protein which increases transcription and/or translation of FOXP3 or which increases the half life (e.g. by at least 10, 20, 30, 40, 50, 60, 70, 80 or 90%) or function of FOXP3 (e.g. determined by suppressive ability of a transduced Treg, measured as previously discussed). For example, it may be possible to introduce a polynucleotide into a Treg which increases transcription of endogenous FOXP3 by interacting with the endogenous FOXP3 promoter or non-coding sequences (CNS, e.g. CNS1, 2 or 3) which are found upstream of the coding region.

Suitably, the polynucleotide encoding the FOXP3 polypeptide or functional fragment or variant thereof may be codon optimised. Suitably, the polynucleotide encoding the FOXP3 polypeptide or functional fragment or variant thereof may be codon optimised for expression in a human cell.

As mentioned above, the nucleic acid molecule may comprise nucleotide sequences encoding self-cleavage sequences. Particularly, the self-cleaving sequences are self-cleaving peptides. Such sequences auto-cleave during protein production. Self-cleaving peptides which may be used are 2A peptides or 2A-like peptides which are known and described in the art, for example in Donnelly et al., Journal of General Virology, 2001, 82, 1027-1041, herein incorporated by reference. 2A and 2A-like peptides are believed to cause ribosome skipping and result in a form of cleavage in which a ribosome skips the formation of peptide bond between the end of a 2A peptide and the downstream amino acid sequence. The "cleavage" occurs between the Glycine and Proline residues at the C-terminus of the 2A peptide meaning the upstream cistron will have a few additional residues added to the end, while the downstream cistron will start with the Proline. The term "cleavage" as used herein thus includes the skipping of peptide bond formation.

Suitable self-cleaving domains include P2A, T2A, E2A, and F2A sequences as shown in SEQ ID NO: 163 - 166 respectively. The sequences may be modified to include the amino acids GSG at the N-terminus of the 2A peptides. Thus, also included as possible options are sequences corresponding to SEQ ID NOs. 163 - 166, but with GSG at the N termini thereof. Such modified alternative 2A sequences are known and reported in the art. Alternative 2A-like sequences which may be used are shown in Donnelly et al (supra), for example a TaV sequence.

The self-cleaving sequences included in the nucleic acid molecule may be the same or different. In an embodiment they are both 2A sequences, in particular P2A and/or T2A sequences.

The self-cleaving sequence may include an additional cleavage site, which may be cleaved by common enzymes present in the cell. This may assist in achieving complete removal of the 2A sequences after translation. Such an additional cleavage site may for example comprise a Furin cleavage site RXXR (SEQ ID NO: 167), for example RRKR (SEQ ID NO: 168).

In a representative embodiment, the nucleic acid molecule may comprise a nucleotide sequence encoding a CAR directed against DGCR2 having the sequence of any one of SEQ ID NOs. 178 to 195 or a variant thereof as described herein, a nucleotide sequence encoding a safety switch and a nucleotide sequence encoding FOXP3.

In such an embodiment, the CAR may comprise:
(a) a leader sequence comprising or consisting of a sequence as set out in SEQ ID NO. 143 or 144, or a sequence having at least 80% sequence identity thereto;
(b) an antigen binding domain comprising or consisting of a sequence as set out in SEQ ID NO. 91, 92, 93, 94, 95, 96, 97, 98, 99, or a sequence having at least 80% sequence identity thereto;
(c) a CD8α hinge and transmembrane domain sequence comprising or consisting of the sequence as set forth in SEQ ID NO. 139 or 141, or a sequence having at least 80% sequence identity thereto;
(d) a CD28 co-stimulatory domain comprising or consisting of the sequence as set forth in SEQ ID NO. 147, or a sequence having at least 80% sequence identity thereto;
(e) a CD3ζ signalling domain comprising or consisting of the sequence as set forth in SEQ ID NO. 145, or a sequence having at least 80% sequence identity thereto.

In an alternative embodiment, the CAR may comprise:
(a) a leader sequence comprising or consisting of a sequence as set out in SEQ ID NO. 143 or 144, or a sequence having at least 80% sequence identity thereto;
(b) an antigen binding domain comprising or consisting of a sequence as set out in SEQ ID NO. 91, 92, 93, 94, 95, 96, 97, 98, 99, or a sequence having at least 80% sequence identity thereto;
(c) a CH2CH3 hinge domain sequence comprising or consisting of the sequence as set forth in SEQ ID NO. 138 or 175, or a sequence having at least 80% sequence identity thereto;
(d) a CD28 transmembrane and co-stimulatory domain comprising or consisting of the sequence as set forth in SEQ ID NO. 146, or a sequence having at least 80% sequence identity thereto;
(e) a CD3ζ signalling domain comprising or consisting of the sequence as set forth in SEQ ID NO. 145, or a sequence having at least 80% sequence identity thereto.

As is clear from the above description in addition to the specific polypeptide and nucleotide sequences mentioned herein, also encompassed is the use of variants, or derivatives and fragments thereof.

The term "derivative" or "variant" as used interchangeably herein, in relation to proteins or polypeptides of the present invention includes any substitution of, variation of, modification of, replacement of, deletion of and/or addition of one (or more) amino acid residues from or to the sequence providing that the resultant protein or polypeptide retains the desired function (for example, where the derivative or variant is an antigen binding domain, the desired function may be the ability of the antigen binding domain to bind its target antigen (for example, a variant of an antigen binding domain which binds to DGCR2 retains the ability to bind DGCR2), where the derivative or variant is a signalling domain, the desired function may be the ability of that domain to signal (e.g. activate or inactivate a downstream molecule), where the derivative or variant is a transcription factor (e.g. FOXP3), the desired function may be the ability of the transcription factor to bind to target DNA and/or to induce transcription or where the derivative or variant is a safety switch polypeptide, the desired function may be the ability of that polypeptide to induce cell death e.g. upon binding of a molecule thereto. Alternatively viewed, the variants or derivatives referred to herein are functional variants or derivatives. For example, variant or derivative may have at least at least 10%, at least 20%, at least 30%, at least 40%, at least 50%, at least 60%, at least 70%, at least 80%, or at least 90% function compared to the corresponding, reference sequence. The variant or derivative may have a similar or the same level of function as compared to the corresponding reference sequence or may have an increased level of function (e.g. increased by at least 10%, at least 20%, at least 30%, at least 40% or at least 50%).

Typically, amino acid substitutions may be made, for example from 1, 2 or 3 to 10 or 20 substitutions provided that the modified sequence retains the required activity or ability. Amino acid substitutions may include the use of non-naturally occurring analogues. For example, the variant or derivative may have at least 10%, at least 20%, at least 30%, at least 40%, at least 50%, at least 60%, at least 70%, at least 80%, or at least 90% activity or ability compared to the corresponding, reference sequence. The variant or derivative may have a similar or the same level of activity or ability as compared to the corresponding, reference sequence or may have an increased level of activity or ability (e.g., increased by at least 10%, at least 20%, at least 30%, at least 40% or at least 50%).

Proteins or peptides may also have deletions, insertions or substitutions of amino acid residues which produce a silent change and result in a functionally equivalent protein. Deliberate amino acid substitutions may be made on the basis of similarity in polarity, charge, solubility, hydrophobicity, hydrophilicity and/or the amphipathic nature of the residues as long as the endogenous function is retained. For example, negatively charged amino acids include aspartic acid and glutamic acid; positively charged amino acids include lysine and arginine; and amino acids with uncharged polar head groups having similar hydrophilicity values include asparagine, glutamine, serine, threonine and tyrosine.

Conservative substitutions may be made, for example according to Table 1 below.

**Table 1**

| | | |
|---|---|---|
| ALIPHATIC | Non-polar | GAP |
| | | ILV |
| | Polar - uncharged | CSTM |
| | | NO |
| | Polar - charged | DE |
| | | KR |
| AROMATIC | | HFWY |

The derivative may be a homologue. The term "homologue" as used herein means an entity having a certain homology with the wild type amino acid sequence and the wild type nucleotide sequence. The term "homology" can be equated with "identity".

A homologous or variant sequence may include an amino acid sequence which may be at least 70%, 75%, 85% or 90% identical, preferably at least 95%, 96%, 97%, 98% or 99% identical to the subject sequence. Typically, the variants will comprise the same active sites etc. as the subject amino acid sequence. Although homology can also be considered in terms of similarity (i.e., amino acid residues having similar chemical properties/functions), in the context herein it is preferred to express homology in terms of sequence identity.

Homology comparisons can be conducted by eye or, more usually, with the aid of readily available sequence comparison programs. These commercially available computer programs can calculate percentage homology or identity between two or more sequences.

Percentage homology or sequence identity may be calculated over contiguous sequences, i.e., one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion in the nucleotide sequence may cause the following codons to be put out of alignment, thus potentially resulting in a large reduction in percent homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible, reflecting higher relatedness between the two compared sequences, will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example, when using the GCG Wisconsin Bestfit package the default gap penalty for amino acid sequences is -12 for a gap and -4 for each extension.

Calculation of maximum percentage homology/sequence identity therefore firstly requires the production of an optimal alignment, taking into consideration gap penalties. A suitable computer program for carrying out such an alignment is the GCG Wisconsin Bestfit package (University of Wisconsin, U.S.A.; Devereux et al. (1984) Nucleic Acids Res. 12: 387). Examples of other software that can perform sequence comparisons include, but are not limited to, the BLAST package (see Ausubel et al. (1999) ibid - Ch. 18), FASTA (Atschul et al. (1990) J. Mol. Biol. 403-410) and the GENEWORKS suite of comparison tools. Both BLAST and FASTA are available for offline and online searching (see Ausubel et al. (1999) ibid, pages 7-58 to 7-60). However, for some applications, it is preferred to use the GCG Bestfit program. Another tool, called BLAST 2 Sequences is also available for comparing protein and nucleotide sequences (see FEMS Microbiol. Lett. (1999) 174: 247-50; FEMS Microbiol. Lett. (1999) 177: 187-8).

Although the final percentage homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see the user manual for further details). For some applications, it is preferred to use the public default values for the GCG package, or in the case of other software, the default matrix, such as BLOSUM62. Suitably, the percentage identity is determined across the entirety of the reference and/or the query sequence.

Once the software has produced an optimal alignment, it is possible to calculate percentage homology, preferably percentage sequence identity. The software typically does this as part of the sequence comparison and generates a numerical result.

"Fragment" typically refers to a selected region of the polypeptide or polynucleotide that is of interest functionally, e.g. is functional or encodes a functional fragment. "Fragment" thus refers to an amino acid or nucleic acid sequence that is a portion (or part) of a full-length polypeptide or polynucleotide.

Such variants, derivatives and fragments may be prepared using standard recombinant DNA techniques such as site-directed mutagenesis. Where insertions are to be made, synthetic DNA encoding the insertion together with 5' and 3' flanking regions corresponding to the naturally-occurring sequence either side of the insertion site may be made. The flanking regions will contain convenient restriction sites corresponding to sites in the naturally-occurring sequence so that the sequence may be cut with the appropriate enzyme(s) and the synthetic DNA ligated into the cut. The DNA is then expressed in accordance with the invention to make the encoded protein. These methods are only illustrative of the numerous standard techniques known in the art for manipulation of DNA sequences and other known techniques may also be used.

Nucleic acid molecules and polynucleotides/nucleotides/nucleic acid sequences as defined herein may comprise DNA or RNA. They may be single-stranded or double-stranded. It will be understood by a skilled person that numerous different nucleic acid molecules/polynucleotides can encode the same polypeptide as a result of the degeneracy of the genetic code. In addition, it is to be understood that the skilled person may, using routine techniques, make nucleotide substitutions that do not affect the polypeptide sequence encoded by the nucleic acid molecules/polynucleotides/nucleotide sequences as defined herein to reflect the codon usage of any particular host organism in which the polypeptides of the invention are to be expressed.

The nucleic acid molecules/nucelootides/polynucleotides may be modified by any method available in the art. Such modifications may be carried out in order to enhance the in vivo activity or lifespan of the nucleic acid molecules/polynucleotides as defined herein.

Nucleic acid molecules/polynucleotides/nucleotide sequences such as DNA nucleic acid molecules/polynucleotides/sequences may be produced recombinantly, synthetically or by any means available to those of skill in the art. They may also be cloned by standard techniques.

Longer nucleic acid molecules/polynucleotides/nucleotide sequences will generally be produced using recombinant means, for example using polymerase chain reaction (PCR) cloning techniques. This will involve making a pair of primers (e.g. of about 15 to 30 nucleotides) flanking the target sequence which it is desired to clone, bringing the primers into contact with mRNA or cDNA obtained from an animal or human cell, performing a polymerase chain reaction under conditions which bring about amplification of the desired region, isolating the amplified fragment (e.g. by purifying the reaction mixture with an agarose gel) and recovering the amplified DNA. The primers may be designed to contain suitable restriction enzyme recognition sites so that the amplified DNA can be cloned into a suitable vector.

The present nucleic acid molecules/nucleotides/polynucleotides may further comprise a nucleic acid sequence encoding a selectable marker. Suitably selectable markers are well known in the art and include, but are not limited to, fluorescent proteins - such as GFP. Suitably, the selectable marker may be a fluorescent protein, for example GFP, YFP, RFP, tdTomato, dsRed, or variants thereof. In some embodiments the fluorescent protein is GFP or a GFP variant. The nucleic acid sequence encoding a selectable marker may be provided in combination with a nucleic acid molecule herein in the form of a nucleic acid construct. Such a nucleic acid construct may be provided in a vector.

Suitably, the selectable marker/reporter domain may be a luciferase-based reporter, a PET reporter (e.g. Sodium Iodide Symporter (NIS)), or a membrane protein (e.g. CD34, or Thy1.1).

The nucleic acid sequences encoding one or more selectable markers may be separated from the present nucleic acid molecule, and/or from each other, by one or more co-expression sites which enables expression of each polypeptide as a discrete entity. Suitable co-expression sites are known in the art and include, for example, internal ribosome entry sites (IRES) and self-cleaving sites such as those included in the present nucleic acid molecules, and as defined above. In an embodiment this may be a 2A cleavage sites, as discussed above.

The use of a selectable marker is advantageous as it allows cells (e.g., Tregs) in which a nucleic acid molecule, construct or vector of the present invention has been successfully introduced (such that the encoded DGCR2 CAR and other modules, e.g., FOXP3 and safety switch polypeptide, are expressed) to be selected and isolated from a starting cell population using common methods, e.g., flow cytometry.

The nucleic acid molecules/nucleotides/polynucleotides used in the present invention may be codon-optimised. Codon optimisation has previously been described in WO1999/41397 and WO2001/79518. Different cells differ in their usage of particular codons. This codon bias corresponds to a bias in the relative abundance of particular tRNAs in the cell type. By altering the codons in the sequence so that they are tailored to match with the relative abundance of corresponding tRNAs, it is possible to increase expression. By the same token, it is possible to decrease expression by deliberately choosing codons for which the corresponding tRNAs are known to be rare in the particular cell type. Thus, an additional degree of translational control is available.

The constructs of the present invention may comprise one or more regulatory sequences, for example a promoter. A "promoter" is a region of DNA that leads to initiation of transcription of a gene. Promoters are located near the transcription start sites of genes, upstream on the DNA (towards the 5' region of the sense strand). Any suitable promoter may be used, the selection of which may be readily made by the skilled person. The promoter may be from any source, and may be a viral promoter, or a eukaryotic promoter, including mammalian or human promoters (i.e. a physiological promoter). In an embodiment the promoter is a viral promoter. Particular promoters include LTR promoters, EFS (or functional truncations thereof), SFFV, PGK, and CMV. In an embodiment the promoter is SFFV or a viral LTR promoter. Particularly, a SFFV promoter may be used within a nucleic acid molecule, construct or vector of the invention to allow initiation of transcription of the nucleotide sequence(s). The promoter may thus control the expression of the CAR of the invention. Where there is more than one nucleotide sequence, each sequence may be operably linked to the same promoter, e.g. nucleotide sequences encoding the CAR, FOXP3 and/or the safety switch

The SFFV promoter may comprise a nucleotide sequence as set out in SEQ ID NO. 169.

"Operably linked to the same promoter" means that transcription of the nucleic acid/nucleotide/polynucleotide sequences may be initiated from the same promoter (e.g., transcription of the first, second and third polynucleotide sequences is initiated from the same promoter) and that the nucleotide sequences are positioned and oriented for transcription to be initiated from the promoter. Nucleic acids/nucleotides/polynucleotides operably linked to a promoter are under transcriptional regulation of the promoter.

In some embodiments of the invention, the nucleic acid/nucleotide/polynucleotide sequence is within an expression vector. The term "expression vector" as used herein means a construct enabling expression of the CAR polypeptide and any additional polypeptides such as a FOXP3 polypeptide or safety switch polypeptide.

A vector is a tool that allows or facilitates the transfer of an entity from one environment to another. As used herein, and by way of example, some vectors used in recombinant nucleic acid techniques allow entities, such as a segment of nucleic acid (e.g., a heterologous DNA segment, such as a heterologous cDNA segment), to be transferred into a target cell. Vectors may be non-viral or viral. Examples of vectors used in recombinant nucleic acid techniques include, but are not limited to, plasmids, mRNA molecules (e.g., in vitro transcribed mRNAs), chromosomes, artificial chromosomes and viruses. The vector may also be, for example, a naked nucleic acid (e.g., DNA). In its simplest form, the vector may itself be a nucleotide of interest.

The vectors used herein may be, for example, plasmid, mRNA or virus vectors and may include a promoter (as described above) for the expression of a nucleic acid molecule/polynucleotide and optionally a regulator of the promoter.

In an embodiment the vector is a viral vector, for example a retroviral, e.g., a lentiviral vector or a gamma retroviral vector.

The vectors may further comprise additional promoters, for example, in one embodiment, the promoter may be a LTR, for example, a retroviral LTR or a lentiviral LTR. Long terminal repeats (LTRs) are identical sequences of DNA that repeat hundreds or thousands of times found at either end of retrotransposons or proviral DNA formed by reverse transcription of retroviral RNA. They are used by viruses to insert their genetic material into the host genomes. Signals of gene expression are found in LTRs: enhancer, promoter (can have both transcriptional enhancers or regulatory elements), transcription initiation (such as capping), transcription terminator and polyadenylation signal.

Suitably, the vector may include a 5'LTR and a 3'LTR.

The vector may comprise one or more additional regulatory sequences which may act pre- or post-transcriptionally. "Regulatory sequences" are any sequences which facilitate expression of the polypeptides, e.g., act to increase expression of a transcript or to enhance mRNA stability. Suitable regulatory sequences include for example enhancer elements, post-transcriptional regulatory elements and polyadenylation sites. Suitably, the additional regulatory sequences may be present in the LTR(s). Suitably, the vector may comprise a Woodchuck Hepatitis Virus Posttranscriptional Regulatory Element (WPRE), e.g., operably linked to the promoter.

Vectors comprising the present nucleic acid molecules/polynucleotides may be introduced into cells using a variety of techniques known in the art, such as transformation and transduction. Several techniques are known in the art, for example infection with recombinant viral vectors, such as retroviral, lentiviral, adenoviral, adeno-associated viral, baculoviral and herpes simplex viral vectors; direct injection of nucleic acids and biolistic transformation.

Non-viral delivery systems include but are not limited to DNA transfection methods. Here, transfection includes a process using a non-viral vector to deliver a gene to a target cell. Non-viral delivery systems can include liposomal or amphipathic cell penetrating peptides, preferably complexed with a nucleic acid molecule or construct.

Typical transfection methods include electroporation, DNA biolistics, lipid-mediated transfection, compacted DNA-mediated transfection, liposomes, immunoliposomes, lipofectin, cationic agent-mediated transfection, cationic facial amphiphiles (CFAs) (Nat. Biotechnol. (1996) 14: 556) and combinations thereof.

Although the present nucleic acid molecules are designed to be used as single constructs, and this would be contained in a single vector, it is not precluded that they are introduced into a cell in conjunction with other vectors, for example encoding other polypeptides it may be desired also to introduce into the cell.

Engineered cells may be generated by introducing a nucleic acid molecule, construct, or vector as defined herein, by one of many means including transduction with a viral vector, and transfection with DNA or RNA.

The present cell may be made by: introducing to a cell (e.g. by transduction or transfection) the nucleic acid molecule/polynucleotide, construct or vector as defined herein.

Suitable cells are discussed further below, but the cell may be from a sample isolated from a subject. The subject may be a donor subject, or a subject for therapy (i.e., the cell may be an autologous cell, or a donor cell, for introduction to another recipient, e.g., an allogeneic cell).

The cell may be generated by a method comprising the following steps:
(i) isolation of a cell-containing sample from a subject or provision of a cell-containing sample; and
(ii) introduction into (e.g., by transduction or transfection) the cell-containing sample of a nucleic acid molecule, construct, or vector as defined herein, to provide a population of engineered cells.

A target cell-enriched sample may be isolated from, enriched, and/or generated from the cell-containing sample prior to and/or after step (ii) of the method. For example, isolation, enrichment and/or generation of Tregs (or other target cells) may be performed prior to and/or after step (ii) to isolate, enrich or generate a Treg-enriched sample. Isolation and/or enrichment from a cell-containing sample may be performed after step (ii) to enrich for cells and/or Tregs (or other target cells) comprising the CAR, the nucleic acid molecule/polynucleotide, the construct and/or the vector as described herein.

A Treg-enriched sample may be isolated or enriched by any method known to those of skill in the art, for example by FACS and/or magnetic bead sorting. A Treg-enriched sample may be generated from the cell-containing sample by any method known to those of skill in the art, for example, from Tcon cells by introducing DNA or RNA coding for FOXP3 and/or from ex-vivo differentiation of inducible progenitor cells or embryonic progenitor cells. Methods for isolating and/or enriching other target cells are known in the art.

Suitably, an engineered target cell may be generated by a method comprising the following steps:
(i) isolation of a target-cell enriched sample from a subject or provision of a target cell-enriched sample; and
(ii) introduction into (e.g., by transduction or transfection) the target cell-enriched sample of a nucleic acid, construct or vector as defined herein, to provide a population of engineered target cells.

The target cell may be a Treg cell, or precursor or a progenitor thereof.

An "engineered cell" means a cell which has been modified to comprise or express a polynucleotide which is not naturally encoded by the cell. Methods for engineering cells are known in the art and include, but are not limited to, genetic modification of cells e.g., by transduction such as retroviral or lentiviral transduction, transfection (such as transient transfection - DNA or RNA based) including lipofection, polyethylene glycol, calcium phosphate and electroporation, as discussed above. Any suitable method may be used to introduce a nucleic acid sequence into a cell. Non-viral technologies such as amphipathic cell penetrating peptides may be used to introduce nucleic acid. A cell may also be genetically modified e.g. using any known gene editing technique to insert a nucleotide, polynucleotide or nucleic acid sequence as described herein into the genome, e.g. using CRISPR, Talens or Zn fingers.

Accordingly, the nucleic acid molecule as described herein is not naturally expressed by a corresponding, unmodified cell. Indeed, the nucleic acid molecule encoding the CAR is an artificial construct, and in an embodiment the safety switch polypeptide is an artificial construct, such they could not occur or be expressed naturally. Suitably, an engineered cell is a cell which has been modified e.g., by transduction or by transfection. Suitably, an engineered cell is a cell which has been modified or whose genome has been modified e.g., by transduction or by transfection. Suitably, an engineered cell is a cell which has been modified or whose genome has been modified by retroviral transduction. Suitably, an engineered cell is a cell which has been modified or whose genome has been modified by lentiviral transduction.

As used herein, the term "introduced" refers to methods for inserting foreign nucleic acid, e.g., DNA or RNA, into a cell. As used herein the term introduced includes both transduction and transfection methods. Transfection is the process of introducing nucleic acids into a cell by non-viral methods. Transduction is the process of introducing foreign DNA or RNA into a cell via a viral vector. Engineered cells may be generated by introducing a nucleic acid as described herein by one of many means including transduction with a viral vector, transfection with DNA or RNA. Cells may be activated and/or expanded prior to, or after, the introduction of a nucleic acid as described herein, for example by treatment with an anti-CD3 monoclonal antibody or both anti-CD3 and anti-CD28 monoclonal antibodies. The cells may also be expanded in the presence of anti-CD3 and anti-CD28 monoclonal antibodies in combination with IL-2. Suitably, IL-2 may be substituted with IL-15. Other components which may be used in a cell (e.g., Treg) expansion protocol include, but are not limited to rapamycin, all-trans retinoic acid (ATRA) and TGFβ. As used herein "activated" means that a cell has been stimulated, causing the cell to proliferate. As used herein "expanded" means that a cell or population of cells has been induced to proliferate. The expansion of a population of cells may be measured for example by counting the number of cells present in a population. The phenotype of the cells may be determined by methods known in the art such as flow cytometry.

The cell may be an immune cell, or a precursor therefor. A precursor cell may be a progenitor cell. Representative immune cells thus include T-cells, in particular, cytotoxic T-cells (CTLs; CD8+ T-cells), helper Tcells (HTLs; CD4+ T-cells) and regulatory T cells (Tregs). Other populations of T-cells are also useful herein, for example naive T-cells and memory T-cells. Other immune cells include NK cells, NKT cells, tolerogenic NK or NKT cells, dendritic cells, MDSC, neutrophils, and macrophages. Precursors of immune cells include pluripotent stem cells, e.g., induced PSC (iPSC), or more committed progenitors including multipotent stem cells (e.g. HPCs), or cells which are committed to a lineage. Precursor cells can be induced to differentiate into immune cells *in vivo* or *in vitro.* In one aspect, a precursor cell may be a somatic cell which is capable of being transdifferentiated to an immune cell of interest.

Most notably, the immune cell may be an NK cell, a dendritic cell, a MDSC, or a T cell, such as a cytotoxic T lymphocyte (CTL), helper T cell or a Treg cell.

In a preferred embodiment the immune cell is a Treg cell. "Regulatory T cells (Treg) or T regulatory cells" are immune cells with immunosuppressive function that control cytopathic immune responses and are essential for the maintenance of immunological tolerance. As used herein, the term Treg refers to a T cell with immunosuppressive function.

A T cell as used herein is a lymphocyte including any type of T cell, such as an alpha beta T cell (e.g., CD8 or CD4+), a gamma delta T cell, a memory T cell, a Treg cell.

Suitably, immunosuppressive function may refer to the ability of the Treg to reduce or inhibit one or more of a number of physiological and cellular effects facilitated by the immune system in response to a stimulus such as a pathogen, an alloantigen, or an autoantigen. Examples of such effects include increased proliferation of conventional T cell (Tconv) and secretion of proinflammatory cytokines. Any such effects may be used as indicators of the strength of an immune response. A relatively weaker immune response by Tconv in the presence of Tregs would indicate an ability of the Treg to suppress immune responses. For example, a relative decrease in cytokine secretion would be indicative of a weaker immune response, and thus indicative of the ability of Tregs to suppress immune responses. Tregs can also suppress immune responses by modulating the expression of co-stimulatory molecules on antigen presenting cells (APCs), such as B cells, dendritic cells and macrophages. Expression levels of CD80 and CD86 can be used to assess suppression potency of activated Tregs *in vitro* after co-culture.

Assays are known in the art for measuring indicators of immune response strength, and thereby the suppressive ability of Tregs. In particular, antigen-specific Tconv cells may be co-cultured with Tregs, and a peptide of the corresponding antigen added to the co-culture to stimulate a response from the Tconv cells. The degree of proliferation of the Tconv cells and/or the quantity of the cytokine IL-2 they secrete in response to addition of the peptide may be used as indicators of the suppressive abilities of the co-cultured Tregs.

Antigen-specific Tconv cells co-cultured with Tregs as disclosed herein may proliferate 5%, 10%, 20%, 30%, 40%, 50%, 60%, 70%, 90%, 95% or 99% less than the same Tconv cells cultured in the absence of the Tregs. For example, antigen-specific Tconv cells co-cultured with the present Tregs may proliferate 5%, 10%, 20%, 30%, 40%, 50%, 60%, 70%, 90%, 95% or 99% less than the same Tconv cells cultured in the presence of non-engineered Tregs. The cells comprising the nucleic acid, expression construct or vector as defined herein, e.g., Tregs, may have an increased suppressive activity as compared to non-engineered Tregs (e.g., an increased suppressive activity of at least 5, 10, 20, 30, 40, 50, 60, 70, 80 or 90%).

Antigen-specific Tconv cells co-cultured with the Tregs herein may express at least 10%, at least 20%, at least 30%, at least 40%, at least 50%, or at least 60% less effector cytokine than corresponding Tconv cells cultured in the absence of the Tregs (e.g., in the presence of non-engineered Tregs).The effector cytokine may be selected from IL-2, IL-17, TNFα, GM-CSF, IFN-y, IL-4, IL-5, IL-9, IL-10 and IL-13. Suitably the effector cytokine may be selected from IL-2, IL-17, TNFα, GM-CSF and IFN-y.

Several different subpopulations of Tregs have been identified which may express different or different levels of particular markers. Tregs generally are T cells which express the markers CD4, CD25 and FOXP3 (CD4⁺CD25⁺FOXP3⁺).

Tregs may also express CTLA-4 (cytotoxic T-lymphocyte associated molecule-4) or GITR (glucocorticoid-induced TNF receptor).

Treg cells are present in the peripheral blood, lymph nodes, and tissues and Tregs for use herein include thymus-derived, natural Treg (nTreg) cells, peripherally generated Tregs, and induced Treg (iTreg) cells.

A Treg may be identified using the cell surface markers CD4 and CD25 in the absence of or in combination with low-level expression of the surface protein CD127 (CD4⁺CD25⁺CD127⁻ or CD4⁺CD25⁺CD127^{low}). The use of such markers to identify Tregs is known in the art and described in Liu et al. (JEM; 2006; 203; 7(10); 1701-1711), for example.

A Treg may be a CD4⁺CD25⁺FOXP3⁺ T cell, a CD4⁺CD25⁺CD127⁻ T cell, or a CD4⁺CD25⁺FOXP3⁺CD127^{-/low} T cell.

Suitably, the Treg may be a natural Treg (nTreg). As used herein, the term "natural T reg" means a thymus-derived Treg. Natural Tregs are CD4⁺CD25⁺FOXP3⁺ Helios⁺ Neuropilin 1⁺. Compared with iTregs, nTregs have higher expression of PD-1 (programmed cell death-1, pdcd1), neuropilin 1 (Nrp1), Helios (Ikzf2), and CD73. nTregs may be distinguished from iTregs on the basis of the expression of Helios protein or Neuropilin 1 (Nrp1) individually.

The Treg may have a demethylated Treg-specific demethylated region (TSDR). The TSDR is an important methylation-sensitive element regulating Foxp3 expression (Polansky, J.K., et al., 2008. European journal of immunology, 38(6), pp.1654-1663).

Further suitable Tregs include, but are not limited to, Tr1 cells (which do not express Foxp3, and have high IL-10 production); CD8⁺FOXP3⁺ T cells; and γδ FOXP3⁺ T cells.

Different subpopulations of Tregs are known to exist, including naive Tregs (CD45RA⁺FoxP3^{low}), effector/memory Tregs (CD45RA⁻FoxP3^{high}) and cytokine-producing Tregs (CD45RA FoxP3^{low}). "Memory Tregs" are Tregs which express CD45RO and which are considered to be CD45RO⁺. These cells have increased levels of CD45RO as compared to naive Tregs (e.g. at least 10, 20, 30, 40, 50, 60, 70, 80 or 90% more CD45RO) and which preferably do not express or have low levels of CD45RA (mRNA and/or protein) as compared to naive Tregs (e.g. at least 80, 90 or 95% less CD45RA as compared to naive Tregs). "Cytokine-producing Tregs" are Tregs which do not express or have very low levels of CD45RA (mRNA and/or protein) as compared to naive Tregs (e.g. at least 80, 90 or 95% less CD45RA as compared to naive Tregs), and which have low levels of FOXP3 as compared to Memory Tregs, e.g. less than 50, 60, 70, 80 or 90% of the FOXP3 as compared to Memory Tregs. Cytokine-producing Tregs may produce interferon gamma and may be less suppressive in vitro as compared to naive Tregs (e.g., less than 50, 60, 70, 80 or 90% suppressive than naive Tregs). Reference to expression levels herein may refer to mRNA or protein expression. Particularly, for cell surface markers such as CD45RA, CD25, CD4, CD45RO etc, expression may refer to cell surface expression, i.e., the amount or relative amount of a marker protein that is expressed on the cell surface. Expression levels may be determined by any known method of the art. For example, mRNA expression levels may be determined by Northern blotting/array analysis, and protein expression may be determined by Western blotting, or preferably by FACS using antibody staining for cell surface expression.

Particularly, the Treg may be a naive Treg. "A naive regulatory T cell, a naive T regulatory cell, or a naive Treg" as used interchangeably herein refers to a Treg cell which expresses CD45RA (particularly which expresses CD45RA on the cell surface). Naive Tregs are thus described as CD45RA⁺. Naive Tregs generally represent Tregs which have not been activated through their endogenous TCRs by peptide/MHC, whereas effector/memory Tregs relate to Tregs which have been activated by stimulation through their endogenous TCRs. Typically, a naive Treg may express at least 10, 20, 30, 40, 50, 60, 70, 80 or 90% more CD45RA than a Treg cell which is not naive (e.g., a memory Treg cell). Alternatively viewed, a naive Treg cell may express at least 2, 3, 4, 5, 10, 50 or 100-fold the amount of CD45RA as compared to a non-naive Treg cell (e.g., a memory Treg cell). The level of expression of CD45RA can be readily determined by methods of the art, e.g., by flow cytometry using commercially available antibodies. Typically, non-naive Treg cells do not express CD45RA or low levels of CD45RA.

Particularly, naive Tregs may not express CD45RO, and may be considered to be CD45RO. Thus, naive Tregs may express at least 10, 20, 30, 40, 50, 60, 70, 80 or 90% less CD45RO as compared to a memory Treg, or alternatively viewed at least 2, 3, 4, 5, 10, 50 or 100 fold less CD45RO than a memory Treg cell.

Although naive Tregs express CD25 as discussed above, CD25 expression levels may be lower than expression levels in memory Tregs, depending on the origin of the naive Tregs. For example, for naive Tregs isolated from peripheral blood, expression levels of CD25 may be at least 10, 20, 30, 40, 50, 60, 70, 80 or 90% lower than memory Tregs. Such naive Tregs may be considered to express intermediate to low levels of CD25. However, a skilled person will appreciate that naive Tregs isolated from cord blood may not show this difference.

Typically, a naive Treg as defined herein may be CD4⁺, CD25⁺, FOXP3⁺, CD127^{low}, CD45RA⁺.

Low expression of CD127 as used herein refers to a lower level of expression of CD127 as compared to a CD4⁺ non-regulatory or Tcon cell from the same subject or donor. Particularly, naive Tregs may express less than 90, 80, 70, 60, 50, 40, 30, 20 or 10% CD127 as compared to a CD4⁺ non-regulatory or Tcon cell from the same subject or donor. Levels of CD127 can be assessed by methods standard in the art, including by flow cytometry of cells stained with an anti-CD127 antibody.

Typically, naive Tregs do not express, or express low levels of CCR4, HLA-DR, CXCR3 and/or CCR6. Particularly, naive Tregs may express lower levels of CCR4, HLA-DR, CXCR3 and CCR6 than memory Tregs, e.g., at least 10, 20, 30, 40, 50, 60, 70, 80 or 90% lower level of expression. Naive Tregs may further express additional markers, including CCR7⁺ and CD31⁺.

Isolated naive Tregs may be identified by methods known in the art, including by determining the presence or absence of a panel of any one or more of the markers discussed above, on the cell surface of the isolated cells. For example, CD45RA, CD4, CD25 and CD127 low can be used to determine whether a cell is a naive Treg. Methods of determining whether isolated cells are naive Tregs or have a desired phenotype can be carried out as discussed below in relation to additional steps which may be carried out, and methods for determining the presence and/or levels of expression of cell markers are well-known in the art and include, for example, flow cytometry, using commercially available antibodies.

Suitably, the cell, such as a Treg, is isolated from peripheral blood mononuclear cells (PBMCs) obtained from a subject. Suitably the subject from whom the PBMCs are obtained is a mammal, preferably a human. Suitably the cell is matched (e.g. HLA matched) or is autologous to the subject to whom the engineered cell is to be administered. Suitably, the subject to be treated is a mammal, preferably a human. The cell may be generated *ex vivo* either from a patient's own peripheral blood (1st party), or in the setting of a haematopoietic stem cell transplant from donor peripheral blood (2nd party), or peripheral blood from an unconnected donor (3rd party). Suitably the cell is autologous to the subject to whom the engineered cell is to be administered.

Suitably, the Treg is part of a population of cells. Suitably, the population of Tregs comprises at least 70 % Tregs, such as at least 75, 85, 90, 95, 97, 98 or 99 % Tregs. Such a population may be referred to as an "enriched Treg population".

In some aspects, the Treg may be derived from *ex-vivo* differentiation of inducible progenitor cells (e.g. iPSCs) or embryonic progenitor cells to the Treg. A nucleic acid molecule or vector as described herein may be introduced into the inducible progenitor cells or embryonic progenitor cells prior to, or after, differentiation to a Treg. Suitable methods for differentiation are known in the art and include that disclosed in Haque et al, J Vis Exp., 2016, 117, 54720 (incorporated herein by reference).

As used herein, the term "conventional T cell" or Tcon or Tconv (used interchangeably herein) means a T lymphocyte cell which expresses an αβ T cell receptor (TCR) as well as a co-receptor which may be cluster of differentiation 4 (CD4) or cluster of differentiation 8 (CD8) and which does not have an immunosuppressive function. Conventional T cells are present in the peripheral blood, lymph nodes, and tissues. Suitably, the engineered Treg may be generated from a Tcon by introducing the nucleic acid which includes a sequence coding for FOXP3. Alternatively, the engineered Treg may be generated from a Tcon by *in vitro* culture of CD4+CD25-FOXP3- cells in the presence of IL-2 and TGF-β.

In another embodiment the target cell into which the nucleic acid molecule, construct or vector is introduced is not a cell intended for therapy. In an embodiment the cell is a production host cell. The cell may be for production of the nucleic acid, e.g., cloning, or vector, or polypeptides.

The invention also provides a cell population comprising a cell as defined or described herein. It will be appreciated that a cell population may comprise both cells of the invention comprising a nucleic acid molecule, expression construct or vector as defined herein, and cells which do not comprise a nucleic acid molecule, expression construct or vector of the invention, e.g., untransduced or untransfected cells. Although in a preferred embodiment, all the cells in a population may comprise a nucleic acid, expression construct or vector of the invention, cell populations having at least 10, 20, 30, 40, 50, 60, 70, 80, 90, 95 or 99% of cells comprising a nucleic acid, expression construct or vector of the invention are provided. Further, the population of cells may comprise more than one cell type, although in a preferred embodiment, at least 10, 20, 30, 40, 50, 60, 70, 80, 90, 95 or 99% of the cells are of the same type. Particularly, a cell population may comprise at least 70, 80, 90, 95 or 99% of T cells, more particularly Tregs. Additionally, at least 10, 20, 30, 40, 50, 60, 70, 80, 90, 95 or 99% of the T cells, particularly Tregs, may comprise a nucleic acid, expression construct or vector of the invention.

In particular, the invention provides a cell population comprising a plurality of cells comprising a CAR comprising an antigen recognition domain that specifically binds to DGCR2, or a nucleic acid molecule or vector encoding said CAR.

There is also provided a pharmaceutical composition comprising a cell or cell population as defined or described herein, or a vector as defined herein. The vector may be used for gene therapy. Thus, rather than administering a cell, a vector may be administered instead, to modify endogenous cells in the subject to express the introduced nucleic acid molecule. Vectors suitable for use in gene therapy are known in the art, and include viral vectors.

Thus, in a further aspect, the invention provides a cell, cell population or pharmaceutical composition as defined herein for use in therapy.

A pharmaceutical composition is a composition that comprises or consists of a therapeutically effective amount of a pharmaceutically active agent i.e., the cell (e.g., Treg), cell population or vector. It preferably includes a pharmaceutically acceptable carrier, diluent or excipient (including combinations thereof). Acceptable carriers or diluents for therapeutic use are well known in the pharmaceutical art, and are described, for example, in Remington's Pharmaceutical Sciences, Mack Publishing Co. (A. R. Gennaro edit. 1985). The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s) or solubilising agent(s).

By "pharmaceutically acceptable" it is included that the formulation is sterile and pyrogen free. The carrier, diluent, and/or excipient must be "acceptable" in the sense of being compatible with the cell or vector and not deleterious to the recipients thereof. Typically, the carriers, diluents, and excipients will be saline or infusion media which will be sterile and pyrogen free, however, other acceptable carriers, diluents, and excipients may be used.

Examples of pharmaceutically acceptable carriers include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethral fatty acid esters, hydroxymethyl-cellulose, polyvinylpyrrolidone, and the like.

The cells, cell population or pharmaceutical compositions may be administered in a manner appropriate for treating and/or preventing the desired disease or condition. The quantity and frequency of administration will be determined by such factors as the condition of the subject, and the type and severity of the subject's disease or condition, although appropriate dosages may be determined by clinical trials. The pharmaceutical composition may be formulated accordingly.

The cell, cell population or pharmaceutical composition as described herein can be administered parenterally, for example, intravenously or intrathecally, or they may be administered by infusion techniques. The cell, cell population or pharmaceutical composition may be administered in the form of a sterile aqueous solution which may contain other substances, for example, enough salts or glucose to make the solution isotonic with blood. The aqueous solution may be suitably buffered (preferably to a pH of from 3 to 9). The pharmaceutical composition may be formulated accordingly. The preparation of suitable parenteral formulations under sterile conditions is readily accomplished by standard pharmaceutical techniques well-known to those skilled in the art.

The pharmaceutical compositions may comprise cells in infusion media, for example sterile isotonic solution. The pharmaceutical composition may be enclosed in ampoules, disposable syringes or multiple dose vials made of glass or plastic.

The cell, cell population or pharmaceutical composition may be administered in a single or in multiple doses. Particularly, the cell, cell population or pharmaceutical composition may be administered in a single, one-off dose. The pharmaceutical composition may be formulated accordingly.

Depending upon the disease/condition and subject to be treated, as well as the route of administration, the cell, cell population or pharmaceutical composition may be administered at a specific stage of disease.

In type 1 diabetes, for example, pancreatic beta cells are destroyed such that they cannot produce insulin. Therefore, an optimum time to administer the cell, cell population or pharmaceutical composition of the invention may be at an early stage of the disease before all pancreatic beta cells have been destroyed, in order to maintain at least some functioning pancreatic beta cells (with residual pancreatic beta cell function) and maintain production of insulin. Particularly, at least 10, 20, 30, 40, 50, 60, 70, 80, 90 or 95% of pancreatic beta cells may be present at the time of administration of the cell, cell population or pharmaceutical composition described herein. Alternatively viewed, less than 5, 10, 20, 30, 40, 50, 60, 70, 80 or 90% of the pancreatic beta cells may have been destroyed before administration of the cell, cell population or pharmaceutical composition described herein.

The pharmaceutical composition may further comprise one or more active agents. The pharmaceutical composition may further comprise one or more other therapeutic agents, such as lympho-depletive agents (e.g. thymoglobulin, campath-1H, anti-CD2 antibodies, anti-CD3 antibodies, anti-CD20 antibodies, cyclophosphamide, fludarabine), inhibitors of mTOR (e.g. sirolimus, everolimus), drugs inhibiting costimulatory pathways (e.g. anti-CD40/CD40L, CTAL4Ig), and/or drugs inhibiting specific cytokines (IL-6, IL-17, TNFalpha, IL18).

Depending upon the disease/condition and subject to be treated, as well as the route of administration, the cell, cell population or pharmaceutical composition may be administered at varying doses (e.g. measured in cells/kg or cells/subject). The physician in any event will determine the actual dosage which will be most suitable for any individual subject and it will vary with the age, weight and response of the particular subject. Typically, however, for the cells herein, doses of 5×10⁷ to 3×10⁹ cells, or 10⁸ to 2×10⁹ cells per subject may be administered.

The cell may be appropriately modified for use in a pharmaceutical composition. For example, cells may be cryopreserved and thawed at an appropriate time, before being infused into a subject.

The invention further includes the use of kits comprising the cell, cell population and/or pharmaceutical composition herein. Preferably said kits are for use in the methods and uses as described herein, e.g., the therapeutic methods as described herein. Preferably said kits comprise instructions for use of the kit components.

The cell, cell population and pharmaceutical composition of the invention may find particular utility in the treatment of disorders associated with cells that express DGCR2, or with disorders where DGCR2 is localised at or near the site of disease, particularly disorders that would benefit from the immunosuppressive activity or target killing activity of the cells of the invention.

The cells, cell populations, compositions and vectors herein may be for use in treating, preventing or reducing the risk of a disease or condition in a subject, notably a disease or condition which may be treated by or with the CAR. The cells and compositions containing them are for adoptive cell therapy (ACT). Various conditions may be treated by administration of cells, including particularly Treg cells, expressing a CAR according to the present disclosure. As noted above, this may be conditions responsive to immunosuppression, and particularly the immunosuppressive effects of Tregs cells. The cells, cell populations, compositions and vectors described herein may thus be used for inducing, or achieving, immunosuppression in a subject. The Treg cells administered, or modified in vivo, may be targeted by expression of the CAR. Conditions suitable for such treatment include autoimmune or inflammatory diseases (e.g. type I diabetes), or more broadly a condition associated with any undesired or unwanted or deleterious immune response. Additionally, the cells, cell populations, compositions and vectors herein may be for use in promoting tissue repair and/or tissue regeneration.

Conditions to be treated or prevented include inflammation, or alternatively put, a condition associated with or involving inflammation. Inflammation may be chronic or acute. Furthermore, the inflammation may be low-level or systemic inflammation.

The term "target cell" refers to any cell expressing DGCR2 to which the cell of the invention is to be directed to exert its therapeutic effect. In some embodiments, the target cell functions as a marker of a disease site, i.e. to attract the cells of the invention to provide an immunosuppressive effect. In some embodiments, the target cell is killed or abrogated by the cells of the invention. As noted above, in some embodiments, the target cell will be a pancreatic beta cell.

In particular, the disease or disorder to be treated may be type 1 diabetes. Other diseases or disorders that may be treated using the CARs described herein include, for example, autoimmune pancreatitis, type 2 diabetes and insulinomas. Particularly the CARs may be expressed in cells having immunosuppressive function (e.g. CD4+ or CD8+ T-regulatory cells, tolerogenic NK or NKT cells, gamma-delta cells and immune regulatory 1 cells (Tr1) and other cells secreting immune modulatory cytokines such as IL-10, TGFbeta, IL-35 or amphiregulin) in order to treat type 1 diabetes, autoimmune pancreatitis or type 2 diabetes. The CARs may be expressed in cells having effector functions (e.g. T-effector cells, NK cells, NKT cells) in order to treat insulinomas, e.g. by eradicating or killing insulinoma cells.

Further, the CARs described herein may be used to protect or prevent rejection of transplanted cells expressing DGCR2 (e.g. beta cell replacements including allogeneic islet transplants, xenogeneic islet transplants and stem cell derived beta cells). Particularly, the CARs may be expressed in cells having immunosuppressive function (e.g. CD4+ or CD8+ T-regulatory cells, tolerogenic NK or NKT cells, gamma-delta cells and immune regulatory 1 cells (Tr1) and other cells secreting immune modulatory cytokines such as IL-10, TGFbeta, IL-35 or amphiregulin) in order to protect or prevent rejection of transplanted cells expressing DGCR2.

Further, the CARs described herein may be used for the targeting or homing of cells, e.g. cells having effector functions (e.g. T-effector cells, NK cells, NKT cells) or cells having immunosuppressive function (e.g. CD4+ or CD8+ T-regulatory cells, tolerogenic NK or NKT cells, gamma-delta cells and immune regulatory 1 cells (Tr1) and other cells secreting immune modulatory cytokines such as IL-10, TGFbeta, IL-35 or amphiregulin) to target cells or target sites which express DGCR2, e.g. to islet cells.

The engineered cells, e.g., Tregs, may be administered to a subject with a disease in order to lessen, reduce, or improve at least one symptom of disease such as hyperglycaemia. The at least one symptom may be lessened, reduced, or improved by at least 10%, at least 20%, at least 30%, at least 40%, or at least 50%, or the at least one symptom may be completely alleviated.

The engineered cells, e.g., Tregs may be administered to a subject with a disease in order to slow down, reduce, or block the progression of the disease. The progression of the disease may be slowed down, reduced, or blocked by at least 10%, at least 20%, at least 30%, at least 40%, or at least 50% compared to a subject in which the engineered cells are not administered, or progression of the disease may be completely stopped.

In particular, the disease to be treated may be type 1 diabetes. As mentioned above, CAR-Tregs specific for DGCR2 may be able to traffic to sites of DGCR2 expression and control inflammation, via their bystander effect, to slow down the rate of pancreatic beta cell destruction.

Type 1 diabetes is a chronic autoimmune disease in which pancreatic beta cells, which are responsible for the production of insulin, are destroyed by the immune system. This is triggered by genetic and environmental factors. Destruction of beta cells reduces or eliminates production of insulin by the body and results in inflammation in the pancreatic islets. Insulin is a hormone required to regulate glucose levels in the bloodstream and, before treatment, subjects with type 1 diabetes will have excessively high blood sugar levels (hyperglycaemia). Type 1 diabetes is a serious and lifelong condition. Patients with type 1 diabetes currently need to closely monitor their blood glucose levels and take an appropriate dose insulin (e.g. by injection or pump). This therapy is not a cure and must be continuously administered. Over time, irregularities in blood sugar levels, including large fluctuations in glycaemic level, can result in long-term complications such as damage to the heart, eyes, feet and kidneys, as well as a reduction in life expectancy. It is noted that glycaemic control is particularly poor in younger subjects, particularly subjects between the ages of approximately 16 and 25 years and the therapies described herein may therefore particularly be useful for this population.

Type 1 diabetes is a continuum that progresses sequentially at variable but predictable rates through distinct identifiable stages prior to the onset of symptoms. This is described in more detail in Insel et al, Diabetes Care. 2015; 38(10): 1964-1974. The ability to screen for risk and to stage type 1 diabetes prior to symptomatic type 1 diabetes provides an opportunity to intervene early to delay and ultimately prevent the onset of clinical symptoms.

Individuals at increased risk of developing type 1 diabetes can be identified by genetic screening. The HLA region on chromosome 6 accounts for about 30-50% of the genetic risk of type 1 diabetes, with the greatest association with HLA class II haplotypes *R81*0301-DQ81*0201* (DR3-DQ2) and *DRB1*0401-DQB1*0302* (DR4-DQ8)*.* The remaining genetic risk for type 1 diabetes can be attributed to approximately 50 non-HLA genes or loci identified by candidate gene and genome-wide association study approaches. The highest non-HLA genetic contribution arises from the INS, PTPN22, CTLA4 and IL2RA genes. Individuals who have been identified at increased risk of type 1 diabetes, but have not yet developed any signs of disease (i.e. pre-stage 1) may be prophylactically administered CAR-Tregs as described herein.

Stage 1 represents individuals who have developed two or more type 1 diabetes-associated islet autoantibodies (to insulin, GAD65, IA-2 and/or ZnT8) but have normal blood glucose levels.

Stage 2 represents individuals who have developed two or more type 1 diabetes-associated islet autoantibodies (to insulin, GAD65, IA-2 and/or ZnT8) but whose disease has progressed to the development of glucose intolerance, or dysglycemia, from loss of functional pancreatic beta cell mass. Dysglycemia may be defined as fasting blood glucose levels equal to or greater than 5.6 mmol/L, or 2 hour plasma glucose with a 75g oral glucose tolerance test (OGTT) equal to or greaterthan 7.8 mmol/L, high glucose levels at intermediate time points on OGTT (30, 60, 90 minute levels equal to or greater than 11.1 mmol/L), and/or HbA1c levels equal to or greater than 5.7% (39 mmol/mol).

Stage 3 represents individuals who have typical clinical symptoms and signs of diabetes, which include, for example, polyuria, polydipsia, weight loss, fatigue and diabetic ketoacidosis (DKA).

The CAR-Tregs described herein may, for example, be used to treat subjects at stage 1, stage 2 and/or stage 3 of disease. Alternatively, the CAR-Tregs described herein may be used to treat a subject at risk of type 1 diabetes, i.e. before stage 1.

Where the subject is at stage 3 of disease, CAR-Treg treatment should begin as soon as possible to minimize pancreatic beta cell destruction and maximize residual pancreatic beta cell function. This may, for example, be determined by measuring blood insulin levels or blood C-peptide levels in the subject.

It may, for example, be a requirement that the subject has a minimum stimulated C-peptide of 0.2 pmol/mL, or for example a minimum stimulated C-peptide of 0.4 pmol/mL from a mixed meal tolerance test at the time of CAR-Treg administration. These subjects may be considered to have "recent-onset type 1 diabetes".

For example, where the subject is at stage 3 of disease, CAR-Treg treatment may begin no more than about 24 weeks after the onset of symptoms or after diagnosis, for example no more than about 20 weeks or no more than about 16 weeks or no more than about 15 weeks or no more than about 14 weeks or no more than about 12 weeks or no more than about 8 weeks or no more than about 6 weeks after the onset of symptoms or after diagnosis. For example, CAR-Treg treatment may begin equal to or less than 100 days after the onset of symptoms or after diagnosis.

The subject may, for example, be at any age. For example, the subject may be under 30 years of age or under 25 years of age or under 20 years of age or under 18 years of age or under 16 years of age. Particularly, the subject may be from 8-30 years old, particularly from 8-25 years old, from 8-16 years old or from 16-25 years old.

Disease progression and pancreatic beta cell death may, for example, be monitored by a number of methods.

For example, imaging of pancreatic beta cells can be used to highlight beta cell integrity. The CAR-Tregs described herein may, for example, maintain or increase the number of pancreatic beta cells present in a subject after they are administered. This may, for example, be observed when the subject is receiving a reduced dose or no dose of exogenous insulin.

For example, insulin levels in a subject can be monitored. Normal fasting blood insulin levels (blood insulin levels after the subject has fasted (not eaten or drunk anything except water) for at least 8 hours) may be considered to be around 2 to 20 mIU/mL. The CAR-Tregs described herein may, for example, maintain or increase fasting blood insulin levels in a subject after they are administered to the subject, for example within the normal range for fasting blood insulin. This may, for example, be observed when the subject is receiving a reduced dose or no dose of exogenous insulin.

Insulin cell-free DNA (cfDNA) and unmethylated insulin may also be detected.

C-peptide levels in a subject can be monitored. The beta cells of the pancreas first produce a protein called "proinsulin". Each proinsulin breaks down to one molecule of insulin and one molecule of C-peptide. Both are released when blood sugar levels are raised. Insulin and C-peptide are released in equal amounts but are broken down differently. Therefore, C-peptide can be employed as a surrogate marker of beta cell function. The liver breaks down insulin at a variable rate, while the kidneys break down C-peptide at a fairly steady rate. C-peptide may therefore be a more reliable measure of insulin production and beta cell function. Normal fasting (i.e. after fasting for at least 8 hours) C-peptide levels may be considered to be around 0.8 to 3.85 ng/mL. The CAR-Tregs described herein may, for example, maintain or increase fasting C-peptide levels in a subject after they are administered to the subject, for example within the normal range for fasting C-peptide. This may, for example, be observed when the subject is receiving a reduced dose or no dose of exogenous insulin.

Further, blood glucose levels in a subject can be monitored. Normal fasting blood glucose levels (i.e. after fasting for at least 8 hours). Normal fasting blood glucose levels may be considered to be around 3.9 to 6.9 mmol/L. Alternatively or additionally, an oral glucose tolerance test could be performed and blood glucose levels measured (e.g. using 75g oral glucose). 2 hours following oral glucose, normal blood glucose levels may be considered to be less than around 7.8 mmol/L. At intermediate time points prior to the 2 hours (e.g. 30 minutes, 60 minutes, 90 minutes), normal blood glucose levels may be considered to be less than around 11.1 mmol/L. The CAR-Tregs described herein may, for example, maintain or reduce fasting blood glucose or OGTT blood glucose levels in a subject after they are administered to the subject, for example within the normal range for fasting blood glucose and/or OGTT blood glucose. This may, for example, be observed when the subject is receiving a reduced dose or no dose of exogenous insulin.

Haemoglobin (Hb) HbA1c levels in a subject can be monitored. HbA1c is created when glucose binds to haemoglobin and is a measure of average blood sugar levels over the preceding 2 to 3 months. Normal HbA1c values are considered to be approximately 4.0 to 5.6% (20 - 38 mmol/mol). The CAR-Tregs may, for example, maintain or reduce HbA1c levels in a subject after they are administered to the subject, for example within the normal range for HbA1c. This may, for example, be observed when the subject is receiving a reduced dose or no dose of exogenous insulin.

Suitably, the subject is a mammal. Suitably, the subject is a human.

Suitably, the cell may be an engineered Treg cell and the cell population may be a population of engineered Treg cells, which have been engineered to express a CAR as described herein.

Suitably, the CAR may comprise an antigen binding domain which is capable of specifically binding to DGCR2, i.e., the antigen is DGCR2.

A method for treating a disease or condition relates to the therapeutic use of the cells herein. In this respect, the cells may be administered to a subject having an existing disease or condition in order to lessen, reduce or improve at least one symptom associated with the disease or condition and/or to slow down, reduce or block the progression of the disease.

Suitably, treating and/or preventing an autoimmune or inflammatory disease may refer to administering an effective amount of the cells (e.g., Tregs) such that the amount of existing medication (e.g. exogenous insulin) that a subject with said disease requires is reduced, or may enable the discontinuation of the subject's existing medication.

Preventing a disease or condition relates to the prophylactic use of the cells herein. In this respect, the cells may be administered to a subject who has not yet contracted or developed the disease or condition and/or who is not showing any symptoms of the disease or condition to prevent the disease or condition or to reduce or prevent development of at least one symptom associated with the disease or condition. The subject may have a predisposition for, or be thought to be at risk of developing, the disease or condition (e.g. pre-stage 1 type 1 diabetes).

As used herein, the term "treatment" refers to clinical intervention designed to alter the natural course of the individual being treated during the course of clinical pathology. Desirable effects of treatment include decreasing the rate of progression, ameliorating or palliating the pathological state, and remission or improved prognosis of a particular disease, disorder, or condition. An individual is successfully "treated", for example, if one or more symptoms associated with a particular disease, disorder, or condition are mitigated or eliminated.

An "effective amount" refers to at least an amount effective, at dosages and for periods of time necessary, to achieve the desired therapeutic or prophylactic result. An effective amount can be provided in one or more administrations.

A "therapeutically effective amount" is at least the minimum concentration required to affect a measurable improvement of a particular disease, disorder, or condition. A therapeutically effective amount herein may vary according to factors such as the disease state, age, sex, and weight of the patient, and the ability of the chimeric receptors to elicit a desired response in the individual. A therapeutically effective amount is also one in which any toxic or detrimental effects of the cell, cell population or pharmaceutic compositions are outweighed by the therapeutically beneficial effects.

The terms "subject", "patient" and "individual" are used interchangeably herein and refer to a mammal, preferably a human. In particular, the terms subject, patient and individual refer to a human having a disease or disorder as defined herein in need of treatment.

In some embodiments of the invention, the patient may be subjected to other treatments prior to, contemporaneously with, or after the treatments of the present invention. For instance, in some embodiments, the patient may be treated with other procedures for the treatment of symptoms associated with the disease or disorder.

The medical use of or method herein may involve the steps of:
(i) isolating a cell-containing sample or providing a cell-containing sample;
(ii) introducing a nucleic acid molecule, construct or a vector as defined herein to the cell; and
(iii) administering the cells from (ii) to a subject.

The cell may be a Treg as defined herein. An enriched Treg population may be isolated and/or generated from the cell containing sample prior to, and/or after, step (ii) of the method. For example, isolation and/or generation may be performed prior to and/or after step (ii) to isolate and/or generate an enriched Treg sample. Enrichment may be performed after step (ii) to enrich for cells and/or Tregs comprising the CAR, the polynucleotide, and/or the vector as described herein.

Suitably, the cell may be autologous. Suitably, the cell may be allogenic.

Suitably, the cell (e.g., the engineered Treg) may be administered in combination with one or more other therapeutic agents, such as lympho-depletive agents (e.g., as discussed above). The engineered cell, e.g., Treg, may be administered simultaneously with or sequentially with (i.e., prior to or after) the one or more other therapeutic agents.

Cells, e.g., Tregs, may be activated and/or expanded prior to, or after, the introduction of a nucleic acid molecule as described herein, for example by treatment with an anti-CD3 monoclonal antibody or both anti-CD3 and anti-CD28 monoclonal antibodies. Expansion protocols are discussed above.

The cell, e.g., Tregs, may be washed after each step of the method, in particular after expansion.

The population of engineered cells, e.g., Treg cells may be further enriched by any method known to those of skill in the art, for example by FACS or magnetic bead sorting.

The steps of the method of production may be performed in a closed and sterile cell culture system.

The invention may also provide a method for increasing the stability and/or suppressive function of a cell comprising the step of introducing a nucleic acid molecule, an expression construct or vector as provided herein into the cell. An increase in suppressive function can be measured as discussed above, for example by co-culturing activated antigen-specific Tconv cells with cells of the invention, and for example measuring the levels the cytokines produced by the Tconv cells. An increase in suppressive function may be an increase of at least 10, 20, 30, 40, 50, 60, 70, 80, or 90% as compared to a non-engineered Treg.

An increase in stability of a cell, e.g., a Treg as defined herein, refers to an increase in the persistence or survival of those cells or to an increase in the proportion of cells retaining a Treg phenotype over a time period (e.g., to cells retaining Treg markers such as FOXP3 and Helios) as compared to a non-engineered Treg.

An increase in stability may be an increase in stability of at least 10, 20, 30, 40, 50, 60, 70, 80 or 90%, and may be measured by techniques known in the art, e.g., staining of Treg cell markers within a population of cells, and analysis by FACS.

The invention also provides the use of a CAR-Treg to reduce the rate or prevent death of pancreatic beta cells, for example in a subject having or at risk of developing type 1 diabetes, particularly recent-onset type 1 diabetes. This may, for example, be determined by measuring number of pancreatic beta cells, blood insulin levels and/or blood C-peptide levels in a subject. Any maintenance or improvement in number of pancreatic beta cells, blood insulin levels and/or blood C-peptide levels may be considered to be reducing the rate or preventing death of pancreatic beta cells. This may, for example, be observed when the subject is receiving a reduced dose or no dose of exogenous insulin.

The invention thus also provides the use of a CAR-Treg to maintain or increase fasting blood insulin levels and/or fasting blood C-peptide levels in a subject, for example in a subject having or at risk of developing type 1 diabetes, particularly recent-onset type 1 diabetes. This may, for example, be observed when the subject is receiving a reduced dose or no dose of exogenous insulin.

The invention further provides the use of a CAR-Treg to reduce or prevent hyperglycaemia, for example in a subject having or at risk of developing type 1 diabetes, particularly recent-onset type 1 diabetes. This may, for example, be determined by measuring fasting blood glucose and/or HbA1c levels in a subject. Any maintenance or improvement in blood glucose levels and/or HbA1c levels in a subject may be considered to be reducing or preventing hyperglycaemia in a subject. This may, for example, be observed when the subject is receiving a reduced dose or no dose of exogenous insulin.

The invention thus also provides the use of a CAR-Treg to maintain or decrease fasting blood glucose and/or HbA1c levels in a subject, for example in a subject having or at risk of developing type 1 diabetes, particularly recent-onset type 1 diabetes. This may, for example, be observed when the subject is receiving a reduced dose or no dose of exogenous insulin.

The CAR is the CAR of present invention, i.e., it comprises an antigen recognition domain that specifically binds to DGCR2 (e.g., to human DGCR2 and/or murine DGCR2), and it may have any of the features of the CAR as disclosed herein.

It will also be appreciated by a skilled person, that the scFvs as identified in the present invention can also be utilised in formats other than within a CAR structure. Thus, in a further aspect of the present invention there is provided an antibody, or an antibody fragment, which specifically binds to DGCR2. Said antibody, or antibody fragment may particularly be an scFv. Said antibody or antibody fragment, e.g. scFv may comprise the CDR sequences, the VH and VL sequences, or the scFv sequences as defined above with respect to the antigen binding domain of the CAR. Thus, all sequences are set out above in relation to CDRs, VH and VL and scFvs may equally be comprised within antibodies or antibody fragments in this embodiment. Particularly, the antibody or antibody fragment may comprise:
(a) VH CDRs as set out in SEQ ID Nos 1-3 and VL CDRs as set out in SEQ ID Nos 4-6;
(b) VH CDRs as set out in SEQ ID Nos 7-9 and VL CDRs as set out in SEQ ID Nos 10-12;
(c) VH CDRs as set out in SEQ ID Nos 13-15 and VL CDRs as set out in SEQ ID Nos 16-18;
(d) VH CDRs as set out in SEQ ID Nos 19-21 and VL CDRs as set out in SEQ ID Nos 22-24;
(e) VH CDRs as set out in SEQ ID Nos 25-27 and VL CDRs as set out in SEQ ID Nos 28-30;
(f) VH CDRs as set out in SEQ ID Nos 31-33 and VL CDRs as set out in SEQ ID Nos 34-36;
(g) VH CDRs as set out in SEQ ID Nos 37-39 and VL CDRs as set out in SEQ ID Nos 40-42;
(h) VH CDRs as set out in SEQ ID Nos 43-45 and VL CDRs as set out in SEQ ID Nos 46-48;
(i) VH CDRs as set out in SEQ ID Nos 49-51 and VL CDRs as set out in SEQ ID Nos 52-54;
or the CDRs may contain 1 to 3, or more particularly 1 or 2 amino acid sequence modifications in any of the aforementioned sequences.

An antibody or antibody fragment may be produced by any method known in the art, including by recombinant expression in a host cell transduced with a vector encoding said antibody or antibody fragment. Suitable host cells include a wide variety of eukaryotic cells (e.g. yeast or mammalian cells) or prokaryotic cells (e.g. E.coli). Antibodies or antibody fragments may alternatively be prepared by chemical synthesis using techniques well known in protein chemistry such as solid phase synthesis or synthesis in homogenous solution.

N-terminal or C-terminal fusion proteins comprising the antibody or antibody fragment as defined herein may be produced, or alternatively viewed, the antibody may be conjugated to another molecule, e.g., a therapeutic molecule or a detectable molecule. The invention therefore further encompasses an antibody or antibody fragment as defined above conjugated to one or more additional molecules, e.g. to an immunoglobulin, hormone, growth factor, lectin, insulin, low density lipoprotein, glucagon, endorphins, transferrin, a tag, fluorochrome, radioactive isotope or a therapeutic molecule (e.g. an immunosuppressive drug).

Nucleic acid molecules comprising a nucleotide sequence encoding the antibody, antibody fragment or fusion protein as defined herein are also provided, as are vectors and cells comprising said nucleic acid molecules.

These antibodies, antibody fragments and fusion proteins/conjugates may be used to detect cells expressing DGCR2 for identification and/or imaging (e.g. islet mass imaging or insulinoma detection). They may also be used to deliver therapeutic molecules to a target site (e.g. cells expressing DGCR2).

Thus, in this respect, the invention provides a method for detecting or imaging a cell expressing DGCR2, particularly an islet cell, comprising incubating a cell with an antibody, antibody fragment or fusion protein/conjugate comprising an antibody or antibody fragment as defined herein and determining whether said antibody, antibody fragment or fusion protein/conjugate is bound to said cell. Alternatively viewed, the invention provides a method for detecting or imaging a cell expressing DGCR2 in a subject comprising administering to said subject an antibody, antibody fragment or fusion protein/conjugate comprising an antibody or antibody fragment as defined herein and determining or detecting binding of said antibody, antibody fragment of fusion protein/conjugate to a cell. Particularly, detection may be carried out by fluorescence where the antibody, antibody fragment or fusion protein/conjugate may be fluorescently labelled. Other detectable labels may also be used.

Finally, the invention provides a method for delivering a therapeutic molecule to a target site where DGCR2 is present or expressed comprising administering to a subject a fusion protein or conjugate comprising an antibody or antibody fragment of the invention and a therapeutic molecule.

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range. Unless otherwise indicated, any nucleic acid sequences are written left to right in 5' to 3' orientation; amino acid sequences are written left to right in amino to carboxy orientation, respectively.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

It must be noted that as used herein and in the appended claims, the singular forms "a", "an", and "the" include plural referents unless the context clearly dictates otherwise.

The terms "comprising", "comprises" and "comprised of' as used herein are synonymous with "including", "includes" or "containing", "contains", and are inclusive or open-ended and do not exclude additional, non-recited members, elements or method steps. The terms "comprising", "comprises" and "comprised of' also include the term "consisting of'.

The publications discussed herein are provided solely for their disclosure prior to the filing date of the present application. Nothing herein is to be construed as an admission that such publications constitute prior art to the claims appended hereto.

Further embodiments of the invention are provided in embodiments 1 to 47 below:
1. A chimeric antigen receptor (CAR) comprising an antigen recognition domain that specifically binds to DGCR2.
2. The CAR according to embodiment 1, wherein the antigen recognition domain binds to human DGCR2.
3. The CAR according to embodiment 1 or 2, comprising:
   b. an exodomain comprising the antigen recognition domain;
   c. a transmembrane domain; and
   d. an endodomain comprising an intracellular signalling domain.
4. The CAR according to embodiment 3, further comprising a hinge domain and/or one or more co-stimulatory domains.
5. The CAR according to embodiment 4, wherein the hinge domain is selected from the hinge regions of CD28, CD8α, CD4, CD7, CH2CH3, an immunoglobulin, or a part or variant thereof, preferably wherein the CAR comprises a CD8α or CH2CH3 hinge region.
6. The CAR according to any of embodiments 3 to 5, wherein the CAR comprises one or more transmembrane domains selected from the transmembrane domains of CD28, ICOS, CD8α, CD4, CD134 (OX40), CD137 (4-1BB), CD3 zeta, CD45, CD9, CD16, CD22, CD33, CD64, CD80, CD86, CD154, or a part or variant thereof, preferably wherein the CAR comprises a CD4, CD28, or CD8α transmembrane domain.
7. The CAR according to any of embodiments 4 to 6, wherein the co-stimulatory domain is selected from the intracellular domains of CD28, ICOS, CD134 (OX40), CD137 (4-1BB), CD27, or TNFRSF25, or a part or variant thereof, preferably wherein the CAR comprises a CD28 co-stimulatory domain.
8. The CAR according to any of embodiments 3 to 7, wherein the CAR comprises one or more intracellular signalling domains selected from the group consisting of the CD3 zeta signalling domain or any of its homologs, a CD3 polypeptide, a syk family tyrosine kinase, a src family tyrosine kinase, CD2, CD5, CD28, or a part or variant thereof, preferably wherein the CAR comprises the CD3 zeta signalling domain.
9. The CAR according to any of embodiments 3 to 8, wherein the CAR comprises: a CD8α or CH2CH3 hinge domain, a CD28 or CD8α transmembrane domain, a CD28 co-stimulatory domain, and the CD3zeta signalling domain, wherein when the hinge domain is CD8α, the transmembrane domain is CD8α, and when the hinge domain is CH2CH3, the transmembrane domain is CD28.
10. The CAR according to any of embodiments 3 to 9, wherein the CAR comprises a signal peptide and/or a reporter peptide.
11. The CAR according to any preceding embodiment, wherein the antigen recognition domain is an antibody, an antibody fragment, or derived from an antibody.
12. The CAR according to any preceding embodiment, wherein the antigen recognition domain is a single chain antibody (scFv).
13. The CAR according to any preceding embodiment, wherein the antigen recognition domain comprises:
   (i) VH CDR1, 2 and 3 sequences set forth in SEQ ID NOs: 1, 2 and 3 respectively and VL CDR1, 2 and 3 sequences set forth in SEQ ID NOs: 4, 5 and 6 respectively;
   (ii) VH CDR1, 2 and 3 sequences set forth in SEQ ID NOs: 7, 8 and 9 respectively and VL CDR1, 2 and 3 sequences set forth in SEQ ID NOs: 10, 11 and 12 respectively;
   (iii) VH CDR1, 2 and 3 sequences set forth in SEQ ID NOs: 13, 14 and 15 respectively and VL CDR1, 2 and 3 sequences set forth in SEQ ID NOs: 16, 17 and 18 respectively;
   (iv) VH CDR1, 2 and 3 sequences set forth in SEQ ID NOs: 19. 20 and 21 respectively and VL CDR1, 2 and 3 sequences set forth in SEQ ID NOs: 22, 23 and 24 respectively;
   (v) VH CDR1, 2 and 3 sequences set forth in SEQ ID NOs: 25, 26 and 27 respectively and VL CDR1, 2 and 3 sequences set forth in SEQ ID NOs: 28, 29 and 30 respectively;
   (vi) VH CDR1, 2 and 3 sequences set forth in SEQ ID NOs: 31, 32 and 33 respectively and VL CDR1, 2 and 3 sequences set forth in SEQ ID NOs: 34, 35 and 36 respectively;
   (vii) VH CDR1, 2 and 3 sequences set forth in SEQ ID NOs: 37, 38 and 39 respectively and VL CDR1, 2 and 3 sequences set forth in SEQ ID NOs: 40, 41 and 42 respectively;
   (viii) VH CDR1, 2 and 3 sequences set forth in SEQ ID NOs: 43, 44 and 45 respectively and VL CDR1, 2 and 3 sequences set forth in SEQ ID NOs: 46, 47 and 48 respectively; or
   (ix) VH CDR1, 2 and 3 sequences set forth in SEQ ID NOs: 49, 50 and 51 respectively and VL CDR1, 2 and 3 sequences set forth in SEQ ID NOs: 52, 53 and 54 respectively;
   wherein one or more of said CDR sequences of (i) to (ix) may optionally comprise 1 to 3 amino acid modifications relative to an aforementioned CDR sequence, particularly wherein one or more of said CDR sequences may optionally be modified by substitution, addition or deletion of 1 to 3 amino acids.
14. The CAR according to any preceding embodiment, wherein the antigen recognition domain comprises:
   (i) a VH domain comprising the sequence set forth in SEQ ID NO: 55, or a sequence having at least 70% sequence identity thereto, and a VL domain comprising the sequence as set forth in SEQ ID NO: 56, or a sequence having at least 70% identity thereto;
   (ii) a VH domain comprising the sequence set forth in SEQ ID NO: 57, or a sequence having at least 70% sequence identity thereto, and a VL domain comprising the sequence as set forth in SEQ ID NO: 58, or a sequence having at least 70% identity thereto;
   (iii) a VH domain comprising the sequence set forth in SEQ ID NO: 59, or a sequence having at least 70% sequence identity thereto, and a VL domain comprising the sequence as set forth in SEQ ID NO: 60, or a sequence having at least 70% identity thereto;
   (iv) a VH domain comprising the sequence set forth in SEQ ID NO: 61, or a sequence having at least 70% sequence identity thereto, and a VL domain comprising the sequence as set forth in SEQ ID NO: 62, or a sequence having at least 70% identity thereto;
   (v) a VH domain comprising the sequence set forth in SEQ ID NO: 63, or a sequence having at least 70% sequence identity thereto, and a VL domain comprising the sequence as set forth in SEQ ID NO: 64, or a sequence having at least 70% identity thereto;
   (vi) a VH domain comprising the sequence set forth in SEQ ID NO: 65, or a sequence having at least 70% sequence identity thereto, and a VL domain comprising the sequence as set forth in SEQ ID NO: 66, or a sequence having at least 70% identity thereto;
   (vii) a VH domain comprising the sequence set forth in SEQ ID NO: 67, or a sequence having at least 70% sequence identity thereto, and a VL domain comprising the sequence as set forth in SEQ ID NO: 68, or a sequence having at least 70% identity thereto;
   (viii) a VH domain comprising the sequence set forth in SEQ ID NO: 69, or a sequence having at least 70% sequence identity thereto, and a VL domain comprising the sequence as set forth in SEQ ID NO: 70, or a sequence having at least 70% identity thereto; or
   (ix) a VH domain comprising the sequence set forth in SEQ ID NO: 71, or a sequence having at least 70% sequence identity thereto, and a VL domain comprising the sequence as set forth in SEQ ID NO: 72, or a sequence having at least 70% identity thereto.
15. The CAR according to any preceding embodiment, wherein the antigen recognition domain comprises:
   (i) a VH domain comprising the sequence encoded by SEQ ID NO: 73, or a sequence having at least 70% identity to the sequence encoded by SEQ ID NO: 73, and a VL domain comprising the sequence encoded by SEQ ID NO: 74, or a sequence having at least 70% identity to the sequence encoded by SEQ ID NO: 74;
   (ii) a VH domain comprising the sequence encoded by SEQ ID NO: 75, or a sequence having at least 70% identity to the sequence encoded by SEQ ID NO: 75, and a VL domain comprising the sequence encoded by SEQ ID NO: 76, or a sequence having at least 70% identity to the sequence encoded by SEQ ID NO: 76;
   (iii) a VH domain comprising the sequence encoded by SEQ ID NO: 77, or a sequence having at least 70% identity to the sequence encoded by SEQ ID NO: 77, and a VL domain comprising the sequence encoded by SEQ ID NO: 78, or a sequence having at least 70% identity to the sequence encoded by SEQ ID NO: 78;
   (iv) a VH domain comprising the sequence encoded by SEQ ID NO: 79, or a sequence having at least 70% identity to the sequence encoded by SEQ ID NO: 79, and a VL domain comprising the sequence encoded by SEQ ID NO: 80, or a sequence having at least 70% identity to the sequence encoded by SEQ ID NO: 80;
   (v) a VH domain comprising the sequence encoded by SEQ ID NO: 81, or a sequence having at least 70% identity to the sequence encoded by SEQ ID NO: 81, and a VL domain comprising the sequence encoded by SEQ ID NO: 82, or a sequence having at least 70% identity to the sequence encoded by SEQ ID NO: 82;
   (vi) a VH domain comprising the sequence encoded by SEQ ID NO: 83, or a sequence having at least 70% identity to the sequence encoded by SEQ ID NO: 83, and a VL domain comprising the sequence encoded by SEQ ID NO: 84, or a sequence having at least 70% identity to the sequence encoded by SEQ ID NO: 84;
   (vii) a VH domain comprising the sequence encoded by SEQ ID NO: 85, or a sequence having at least 70% identity to the sequence encoded by SEQ ID NO: 85, and a VL domain comprising the sequence encoded by SEQ ID NO: 86, or a sequence having at least 70% identity to the sequence encoded by SEQ ID NO: 86;
   (viii) a VH domain comprising the sequence encoded by SEQ ID NO: 87, or a sequence having at least 70% identity to the sequence encoded by SEQ ID NO: 87, and a VL domain comprising the sequence encoded by SEQ ID NO: 88, or a sequence having at least 70% identity to the sequence encoded by SEQ ID NO: 88; or
   (ix) a VH domain comprising the sequence encoded by SEQ ID NO: 89, or a sequence having at least 70% identity to the sequence encoded by SEQ ID NO: 89, and a VL domain comprising the sequence encoded by SEQ ID NO: 90, or a sequence having at least 70% identity to the sequence encoded by SEQ ID NO: 90.
16. The CAR according to any preceding embodiment, wherein the antigen recognition domain comprises or consists of:
   (i) the sequence set forth in SEQ ID NO: 91 or a sequence having at least 80% sequence identity thereto;
   (ii) the sequence set forth in SEQ ID NO: 92 or a sequence having at least 80% sequence identity thereto;
   (iii) the sequence set forth in SEQ ID NO: 93 or a sequence having at least 80% sequence identity thereto;
   (iv) the sequence set forth in SEQ ID NO: 94 or a sequence having at least 80% sequence identity thereto;
   (v) the sequence set forth in SEQ ID NO: 95 or a sequence having at least 80% sequence identity thereto;
   (vi) the sequence set forth in SEQ ID NO: 96 or a sequence having at least 80% sequence identity thereto;
   (vii) the sequence set forth in SEQ ID NO: 97 or a sequence having at least 80% sequence identity thereto;
   (viii) the sequence set forth in SEQ ID NO: 98 or a sequence having at least 80% sequence identity thereto; or
   (ix) the sequence set forth in SEQ ID NO: 99 or a sequence having at least 80% sequence identity thereto.
17. The CAR according to any preceding embodiment, wherein the antigen recognition domain comprises or consists of:
   (i) the sequence encoded by the sequence set forth in SEQ ID NO: 100 or a sequence having at least 80% identity to the sequence encoded by the sequence set forth in SEQ ID NO: 100;
   (ii) the sequence encoded by the sequence set forth in SEQ ID NO: 101 or a sequence having at least 80% identity to the sequence encoded by the sequence set forth in SEQ ID NO: 101;
   (iii) the sequence encoded by the sequence set forth in SEQ ID NO: 102 or a sequence having at least 80% identity to the sequence encoded by the sequence set forth in SEQ ID NO: 102;
   (iv) the sequence encoded by the sequence set forth in SEQ ID NO: 103 or a sequence having at least 80% identity to the sequence encoded by the sequence set forth in SEQ ID NO: 103;
   (v) the sequence encoded by the sequence set forth in SEQ ID NO: 104 or a sequence having at least 80% identity to the sequence encoded by the sequence set forth in SEQ ID NO: 104;
   (vi) the sequence encoded by the sequence set forth in SEQ ID NO: 105 or a sequence having at least 80% identity to the sequence encoded by the sequence set forth in SEQ ID NO: 105;
   (vii) the sequence encoded by the sequence set forth in SEQ ID NO: 106 or a sequence having at least 80% identity to the sequence encoded by the sequence set forth in SEQ ID NO: 106;
   (viii) the sequence encoded by the sequence set forth in SEQ ID NO: 107 or a sequence having at least 80% identity to the sequence encoded by the sequence set forth in SEQ ID NO: 107; or
   (ix) the sequence encoded by the sequence set forth in SEQ ID NO: 108 or a sequence having at least 80% identity to the sequence encoded by the sequence set forth in SEQ ID NO: 108.
18. The CAR according to any preceding embodiment, wherein the CAR comprises or consists of the sequence set forth in any one of SEQ ID Nos: 178 to 195, or a sequence having at least 80% identity thereto.
19. A nucleic acid molecule comprising a nucleotide sequence encoding the CAR of any preceding embodiment.
20. A vector comprising the nucleic acid molecule of embodiment 19.
21. The vector of embodiment 20, further comprising a nucleic acid molecule encoding a FOXP3 polypeptide.
22. A cell comprising the CAR of any of embodiments 1 to 18, the nucleic acid molecule of embodiment 19, or the vector of embodiment 20 or 21.
23. The cell of embodiment 22, wherein the cell is a production host cell.
24. The cell of embodiment 22, wherein the cell is an immune cell or a progenitor or precursor thereof, preferably a T cell, or a precursor thereof, or a stem cell.
25. The cell of embodiment 22 or 24, wherein the cell is a regulatory T cell (Treg), or a precursor thereof, or an iPSC cell, particularly wherein the cell further comprises an exogenous nucleic acid comprising a nucleotide sequence encoding a FOXP3 polypeptide.
26. A cell population comprising a cell of any one of embodiments 22, 24 or 25.
27. The cell population of embodiment 26, comprising a plurality of cells of any one of embodiments 22, 24 or 25, particularly a plurality of T cells of embodiment 24, more particularly a plurality of Tregs of embodiment 25.
28. A pharmaceutical composition comprising a cell of any one of embodiments 22, 24 or 25, a cell population of embodiment 26 or 27, or a vector of embodiment 20 or 21.
29. The cell of any one of embodiments 22, 24 or 25, a cell population of embodiment 26 or 27, or the pharmaceutical composition of embodiment 28, for use in therapy.
30. The cell, cell population or pharmaceutical composition for use of embodiment 29, wherein the therapy is adoptive cell transfer therapy.
31. The cell of any one of embodiments 22, 24 or 25, a cell population of embodiment 26 or 27, or pharmaceutical composition of embodiment 28, for use in treating or preventing an autoimmune or inflammatory disease, or for use in inducing immunosuppression, or for use in promoting tissue repair and/or tissue regeneration, particularly wherein the cell is a Treg.
32. The cell, cell population or pharmaceutical composition for use of embodiment 31, wherein the autoimmune or inflammatory disease is type 1 diabetes, for example recent-onset type 1 diabetes.
33. A method of treating and/or preventing an autoimmune or inflammatory disease such as type 1 diabetes, or inducing immunosuppression, or promoting tissue repair and/or tissue regeneration, wherein the method comprises administering a cell of any one of embodiments 22, 24 or 25, particularly a Treg, a cell population of embodiment 26 or 27, or a pharmaceutical composition of embodiment 28, particularly comprising a Treg.
34. The method according to embodiment 33, which comprises the following steps:
   i. isolation or provision of a Treg-enriched cell sample from a subject;
   ii. introduction into the Treg cells of a nucleic acid molecule of embodiment 19 or a vector of embodiment 20 or 21; and
   iii. administering the Treg cells from (ii) to the subject.
35. Use of a cell of any one of embodiments 22, 24 or 25, a cell population of embodiment 26 or 27, or a pharmaceutical composition of embodiment 28, in the manufacture of a medicament for treating and/or preventing an autoimmune or inflammatory disease such as type 1 diabetes, or inducing immunosuppression, or promoting tissue repair and/or tissue regeneration in a subject, particularly wherein the cell is a Treg.
36. A method of making a cell of any one of embodiments 22, 24 or 25, which comprises the step of introducing into the cell (e.g. transducing or transfecting a cell with) the nucleic acid molecule of embodiment 19 or the vector of embodiment 20 or 21.
37. The method of embodiment 36, wherein the cell is a Treg cell, and the method comprises isolating or providing a cell-containing sample comprising Tregs, and/or Tregs are enriched and/or generated from the cell-containing sample prior to or after the step of introducing the nucleic acid molecule or vector into the cell.
38. A cell obtainable by the method of embodiment 36 or 37.
39. The cell of any one of embodiments 22, 24 or 25, a cell population of embodiment 26 or 27, or pharmaceutical composition of embodiment 28, for use to prevent or reduce the rate of death of pancreatic beta cells in a subject.
40. The cell of any one of embodiments 22, 24 or 25, a cell population of embodiment 26 or 27, or pharmaceutical composition of embodiment 28, for use to maintain or increase fasting blood insulin levels in a subject.
41. The cell of any one of embodiments 22, 24 or 25, a cell population of embodiment 26 or 27, or pharmaceutical composition of embodiment 28, for use to maintain or increase fasting C-peptide levels in a subject.
42. The cell of any one of embodiments 22, 24 or 25, a cell population of embodiment 26 or 27, or pharmaceutical composition of embodiment 28, for use to reduce or prevent hyperglycemia in a subject.
43. The cell of any one of embodiments 22, 24 or 25, a cell population of embodiment 26 or 27, or pharmaceutical composition of embodiment 28, for use to maintain or decrease fasting blood glucose levels in a subject.
44. The cell of any one of embodiments 22, 24 or 25, a cell population of embodiment 26 or 27, or pharmaceutical composition of embodiment 28, for use to maintain or reduce HbA1c levels in a subject.
45. The cell, cell population, or pharmaceutical composition for use of any of embodiments 39 to 44, wherein the subject has or is at risk of developing type 1 diabetes, for example wherein the subject has recent-onset type 1 diabetes.
46. The cell, cell population, or pharmaceutical composition for use of any of embodiments 39 to 45, wherein the subject is not being administered exogenous insulin.
47. The cell, cell population, or pharmaceutical composition for use of any of embodiments 39 to 46, wherein the subject is being administered a reduced dose of insulin compared to the dose of insulin administered prior to administration of the CAR-Tregs.

The invention will now be further described by way of Examples, which are meant to serve to assist one of ordinary skill in the art in carrying out the invention and are not intended in any way to limit the scope of the invention.

### SEQUENCE LISTING

**SEQ ID NO: 1** - amino acid sequence of VH CDR1 of G3
   GFTFSSYG
**SEQ ID NO: 2 -** amino acid sequence of VH CDR2 of G3
   ISYDGSNK
**SEQ ID NO: 3** - amino acid sequence of VH CDR3 of G3
   AKDRGAVAGTDWYFDL
**SEQ ID NO: 4** - amino acid sequence of VL CDR1 of G3
   SLRSYY
**SEQ ID NO:** 5 - amino acid sequence of VL CDR2 of G3
   GKN
**SEQ ID NO:** 6 - amino acid sequence of VL CDR3 of G3
   NSRDSSGNHVV
**SEQ ID NO:** 7 - amino acid sequence of VH CDR1 of G9
   GFTFSSYS
**SEQ ID NO: 8** - amino acid sequence of VH CDR2 of G9
   ITRGGNT
**SEQ ID NO: 9** - amino acid sequence of VH CDR3 of G9
   AKDWTMSGINVFDT
**SEQ ID NO: 10** - amino acid sequence of VL CDR1 of G9
   SSDVGAYNR
**SEQ ID NO: 11** - amino acid sequence of VL CDR2 of G9
   EVN
**SEQ ID NO: 12** - amino acid sequence of VL CDR3 of G9
   SSSTTSSTYV
**SEQ ID NO: 13** - amino acid sequence of VH CDR1 of G11
   EFPFSSYA
**SEQ ID NO: 14** - amino acid sequence of VH CDR2 of G11
   ISSSGGDT
**SEQ ID NO: 15** - amino acid sequence of VH CDR3 of G11
   AKNWNLDY
**SEQ ID NO: 16** - amino acid sequence of VL CDR1 of G11
   SLRSYY
**SEQ ID NO: 17** - amino acid sequence of VL CDR2 of G11
   GDN
**SEQ ID NO: 18** - amino acid sequence of VL CDR3 of G11
   NSRDSSGHHNVL
**SEQ ID NO: 19** - amino acid sequence of VH CDR1 of A5
   GFTVSSNY
**SEQ ID NO: 20** - amino acid sequence of VH CDR2 of A5
   IYSGGST
**SEQ ID NO: 21** - amino acid sequence of VH CDR3 of A5
   ARGWTTFDY
**SEQ ID NO: 22** - amino acid sequence of VL CDR1 of A5
   QLGDRY
**SEQ ID NO: 23** - amino acid sequence of VL CDR2 of A5
   QDD
**SEQ ID NO: 24** - amino acid sequence of VL CDR3 of A5
   QTWDNSPVV
**SEQ ID NO: 25** - amino acid sequence of VH CDR1 of F5
   GFTFSSYG
**SEQ ID NO: 26** - amino acid sequence of VH CDR2 of F5
   ISYDGSNK
**SEQ ID NO: 27** - amino acid sequence of VH CDR3 of F5
   AKDLTRSGYPSYYFDY
**SEQ ID NO: 28** - amino acid sequence of VL CDR1 of F5
   SLRSYY
**SEQ ID NO: 29** - amino acid sequence of VL CDR2 of F5
   GKN
**SEQ ID NO: 30** - amino acid sequence of VL CDR3 of F5
   NSRGSSGNNWV
**SEQ ID NO: 31** - amino acid sequence of VH CDR1 of D3
   GFTFSSNA
**SEQ ID NO: 32 -** amino acid sequence of VH CDR2 of D3
   IGTGGTT
**SEQ ID NO: 33 -** amino acid sequence of VH CDR3 of D3
   AKTGQLDY
**SEQ ID NO: 34 -** amino acid sequence of VL CDR1 of D3
   SSDVGGYNY
**SEQ ID NO: 35 -** amino acid sequence of VL CDR2 of D3
   DVS
**SEQ ID NO: 36 -** amino acid sequence of VL CDR3 of D3
   AAWDDSLSGWV
**SEQ ID NO: 37 -** amino acid sequence of VH CDR1 of D4
   GFTFSNYA
**SEQ ID NO: 38** - amino acid sequence of VH CDR2 of D4
   ISGGGDNT
**SEQ ID NO: 39** - amino acid sequence of VH CDR3 of D4
   AKKQSGGSGWSLWGFDY
**SEQ ID NO: 40** - amino acid sequence of VL CDR1 of D4
   TGAVTSGYY
**SEQ ID NO: 41** - amino acid sequence of VL CDR2 of D4
   NTN
**SEQ ID NO: 42 -** amino acid sequence of VL CDR3 of D4
   LLYYGGRGV
**SEQ ID NO: 43 -** amino acid sequence of VH CDR1 of F7
   GFTFSSYA
**SEQ ID NO: 44 -** amino acid sequence of VH CDR2 of F7
   ISGSGGST
**SEQ ID NO: 45** - amino acid sequence of VH CDR3 of F7
   AKNPIQLWSKIKNWFDP
**SEQ ID NO: 46** - amino acid sequence of VL CDR1 of F7
   AGDVTYGHF
**SEQ ID NO: 47** - amino acid sequence of VL CDR2 of F7
   DVN
**SEQ ID NO: 48** - amino acid sequence of VL CDR3 of F7
   ALSYFGSMF
**SEQ ID NO: 49** - amino acid sequence of VH CDR1 of D2
   GFTFSSYG
**SEQ ID NO: 50** - amino acid sequence of VH CDR2 of D2
   ISYDGSNK
**SEQ ID NO: 51** - amino acid sequence of VH CDR3 of D2
   AKDLTRSGYPSYYFDY
**SEQ ID NO: 52** - amino acid sequence of VL CDR1 of D2
   SLRSYY
**SEQ ID NO: 53** - amino acid sequence of VL CDR2 of D2
   GKN
**SEQ ID NO: 54** - amino acid sequence of VL CDR3 of D2
   NSRDSSGNLV
**SEQ ID NO: 55** - amino acid sequence of the VH domain of G3 (CDRs are underlined) 
QVQLVQSGGGVVQPGRSLRLSCAASGFTFSSYGMHWVRQAPGKGLEWVAVISYDGSNKYYADSVK
GRFTISRDNSKNTLYLQMNSLRAEDTAVYYCAKDRGAVAGTDWYFDLWGRGTLVTVSS
**SEQ ID NO: 56** - amino acid sequence of the VL domain of G3 (CDRs are underlined) 
SSELTQDPAVSVALGQTVRITCQGDSLRSYYASWYQQKPGQAPVLVIYGKNNRPSGIPDRFSGSSS
GNTASLTITGAQAEDEADYYCNSRDSSGNHVVFGGGTKLTVL
**SEQ ID NO: 57** - amino acid sequence of the VH domain of G9 (CDRs are underlined) 
QVQLVQSGGGLVQPGGSLRLSCAASGFTFSSYSMSWVRQAPGKGLEWVLGITRGGNTFYADSVTG
RFTISRDISKNTVFLQMDSLRVEDTAIYYCAKDWTMSGINVFDTWGQGTLVTVSS
**SEQ ID NO: 58** - amino acid sequence of the VL domain of G9 (CDRs are underlined) 
QAVLTQPPSVSGSPGQSVTISCTGTSSDVGAYNRVSWYQQPPGTAPKLMIYEVNNRPSGVPDRFSG
SKSGNTASLTISGLQAEDE ADYYCSSSTTSSTYVFGTGTKVTVL
**SEQ ID NO: 59** - amino acid sequence of the VH domain of G11 (CDRs are underlined) 
EVQLQQSGGGLVQPGGSLRLSCAASEFPFSSYAMSWVRQAPGKGLAWVSAISSSGGDTYYADSVK
GRFTISRDNSKNTLYLQMNSLRAEDTAVYYCAKNWNLDYWGQGTLVTVSS
**SEQ ID NO: 60** - amino acid sequence of the VL domain of G11 (CDRs are underlined) 
SSELTQDPAVSVALGQTVMITCQGHSLRSYYASWYQQKPGQAPLLVIYGDNRRPSGIPDRFSGSNS
GNTASLTITGTQAEDEADYYCNSRDSSGHHNVLFGGGTKLTVL
**SEQ ID NO: 61** - amino acid sequence of the VH domain of A5 (CDRs are underlined) 
EVQLVESGGGLVQPGGSLRLSCAASGFTVSSNYMSWVRQAPGKGLEWVSVIYSGGSTYYADSVKG
RFTISRDNAKNSLYLQMNSLRAEDTAVYYCARGWTTFDYWGQGTLVTVSS
**SEQ ID NO: 62** - amino acid sequence of the VL domain of A5 (CDRs are underlined) 
QAVLTQPPSLSVSPRQTVTITCSGDQLGDRYVSWYQQKPGQSPMLVIYQDDRRPSGVPERFSGSNS
GNTATLTIGGTQAMDQADYYCQTWDNSPVVFGGGTKLTVL
**SEQ ID NO: 63** - amino acid sequence of the VH domain of F5 (CDRs are underlined) 
EVQLVQSGGGVVQPGRSLRLSCAASGFTFSSYGMHWVRQAPGKGLEWVAVISYDGSNKYYADSVK
GRFTISRDNSKNTLYLQMNSLRAEDTAVYYCAKDLTRSGYPSYYFDYWGQGTLVTVSS
**SEQ ID NO: 64 -** amino acid sequence of the VL domain of F5 (CDRs are underlined) 
SSELTQDPAVSVALGQTVRITCQGDSLRSYYASWYQQKPGQAPVLVIYGKNNRPSGIPDRFSGSSS
GNTASLTITGAQAEDEADYYCNSRGSSGNNWVFGGGTKLTVL
**SEQ ID NO: 65** - amino acid sequence of the VH domain of D3 (CDRs are underlined) 
QVQLVESGGGLVQPGGSLRLSCAASGFTFSSNAMSWVRQAPGKGLEWVSAIGTGGTTYYADSVKG
RFTISRDNSKNTLYLQMNSLRAEDTAVYYCAKTGQLDYWGQGTLVTVSS
**SEQ ID NO: 66** - amino acid sequence of the VL domain of D3 (CDRs are underlined) 
QSALTQPASVSGSPGQSITISCTGTSSDVGGYNYVSWFQQHPDKAPKLMIYDVSNRPSGVSNRFSG
SKSGTSASLAISGLRSEDEADYYCAAWDDSLSGWVFGGGTKLTVL
**SEQ ID NO: 67** - amino acid sequence of the VH domain of D4 (CDRs are underlined) 
QVQLVQSGGGLVQPGGSLRLSCAASGFTFSNYAMSWVRQAPGKGLEWVSGISGGGDNTYYTDSV
KGRFTISRDNSKNTLYLQMNSLTAEDTALYYCAKKQSGGSGWSLWGFDYWGQGTLVTVSS
**SEQ ID NO: 68** - amino acid sequence of the VL domain of D4 (CDRs are underlined) 
QAVVTQEPSLTVSPGGTVTLTCASSTGAVTSGYYPSWFQQKPGQAPRPLIYNTNNKHSWSPARFSG
SLLGGKAALTLSGVQPEDEADYYCLLYYGGRGVFGGGTRLTVL
**SEQ ID NO: 69** - amino acid sequence of the VH domain of F7 (CDRs are underlined) 
EVQLLESGGGLVQPGGSLRLSCAASGFTFSSYAMSWVRQAPGKGLEWVSAISGSGGSTYYADSVK
GRFTISRDNSKNTLYLQMNSLRAEDTAVYYCAKNPIQLWSKIKNWFDPWGQGTLVTVSS
**SEQ ID NO: 70** - amino acid sequence of the VL domain of F7 (CDRs are underlined) 
QAVVTQEPSLSVSPGGTVSLTCGSSAGDVTYGHFPYWYQQKPGQAPRMLIYDVNNRASWTPARFS
GSLVGGKAALTLSGAQPGDEADYYCALSYFGSMFFGGGTKLTVL
**SEQ ID NO: 71** - amino acid sequence of the VH domain of D2 (CDRs are underlined) 
EVQLVQSGGGVVQPGRSLRLSCAASGFTFSSYGMHWVRQAPGKGLEWVAVISYDGSNKYYADSVK
GRFTISRDNSKNTLYLQMNSLRAEDTAVYYCAKDLTRSGYPSYYFDYWGQGTLVTVSS
**SEQ ID NO: 72** - amino acid sequence of the VL domain of D2 (CDRs are underlined) 
SSELTQDPAVSVALGRTVRITCQGDSLRSYYASWYQQKPGQAPVLVIYGKNNRPSGIPDRFSGSSS
GNTASLTITGAQAEDEADYYCNSRDSSGNLVFGGGTKLTVL
**SEQ ID NO: 73** - nucleotide sequence encoding the VH domain of G3 
CAGGTGCAGCTGGTGCAGTCTGGGGGAGGCGTGGTCCAGCCTGGGAGGTCCCTGAGACTCTC
CTGTGCAGCCTCTGGATTCACCTTCAGTAGCTATGGCATGCACTGGGTCCGCCAGGCTCCAGGC
AAGGGGCTGGAGTGGGTGGCAGTTATATCATATGATGGAAGTAATAAATACTATGCAGACTCCGT
GAAGGGCCGATTCACCATCTCCAGAGACAATTCCAAGAACACGCTGTATCTGCAAATGAACAGC
CTGAGAGCTGAGGACACGGCTGTGTATTACTGTGCGAAAGATCGGGGAGCAGTGGCTGGGACT
GACTGGTACTTCGATCTCTGGGGCCGTGGCACCCTGGTCACTGTCTCCTCAG
**SEQ ID NO: 74** - nucleotide sequence encoding the VL domain of G3 
TCTTCTGAGCTGACTCAGGACCCTGCTGTGTCTGTGGCCTTGGGACAGACAGTCAGGATCACAT
GCCAAGGAGACAGCCTCAGAAGCTATTATGCAAGCTGGTACCAGCAGAAGCCAGGACAGGCCC
CTGTACTTGTCATCTATGGTAAAAACAACCGGCCCTCAGGGATCCCAGACCGATTCTCTGGCTCC
AGCTCAGGAAACACAGCTTCCTTGACCATCACTGGGGCTCAGGCGGAAGATGAGGCTGACTATT
ACTGTAACTCCCGGGACAGCAGTGGTAACCATGTGGTATTCGGCGGAGGGACCAAGCTGACCG
TCCTAG
**SEQ ID NO: 75** - nucleotide sequence encoding the VH domain of G9 
CAGGTCCAGCTGGTGCAATCTGGGGGAGGCTTGGTACAGCCGGGGGGGTCCCTGAGACTCTCC
TGTGCAGCCTCTGGATTCACCTTCAGTAGCTATAGCATGAGCTGGGTCCGCCAGGCCCCAGGGA
AGGGGCTGGAGTGGGTCTTAGGTATTACTCGTGGTGGTAACACATTCTACGCAGACTCCGTGAC
GGGCCGGTTCACCATCTCCAGAGACATTTCCAAGAACACGGTGTTTCTGCAAATGGACAGCCTG
AGAGTCGAGGACACGGCCATTTATTACTGTGCGAAAGACTGGACCATGAGTGGAATCAACGTCT
TCGACACCTGGGGCCAGGGGACCCTGGTCACCGTCTCCTCCG
**SEQ ID NO: 76** - nucleotide sequence encoding the VL domain of G9 
CAGGCAGTGCTGACTCAGCCACCCTCCGTGTCCGGGTCTCCTGGACAGTCAGTCACCATCTCCT
GCACTGGAACCAGCAGTGACGTTGGTGCTTATAACCGTGTCTCCTGGTACCAGCAGCCCCCAGG
CACAGCCCCCAAACTTATGATTTATGAGGTCAATAATCGGCCCTCAGGGGTCCCTGATCGCTTCT
CTGGGTCCAAGTCTGGCAACACGGCCTCCCTGACCATCTCTGGGCTCCAGGCTGAGGACGAGG
CTGATTATTACTGCAGCTCATCTACAACCAGTAGCACTTATGTCTTCGGAACTGGGACCAAGGTC
ACCGTCCTAG
**SEQ ID NO: 77** - nucleotide sequence encoding the VH domain of G11 
GAAGTGCAGCTACAGCAGTCCGGGGGAGGCTTGGTACAGCCTGGGGGGTCCCTGAGACTCTCC
TGTGCAGCCTCTGAATTCCCCTTTAGCAGCTATGCCATGAGCTGGGTCCGCCAGGCTCCAGGGA
AGGGGCTGGCGTGGGTCTCAGCTATTAGTAGTAGTGGTGGTGACACATACTACGCAGACTCCGT
GAAGGGCCGGTTCACCATCTCCAGAGACAATTCCAAGAACACGCTGTATCTGCAAATGAACAGC
CTGAGAGCCGAGGACACGGCCGTATATTACTGTGCGAAAAACTGGAACCTTGACTACTGGGGCC
AGGGAACCCTGGTCACCGTCTCCTCAG
**SEQ ID NO: 78-** nucleotide sequence encoding the VL domain of G11 
TCTTCTGAGCTGACTCAGGACCCTGCTGTGTCTGTGGCCTTGGGACAGACAGTCATGATAACAT
GCCAAGGACACAGCCTCCGAAGCTATTATGCAAGCTGGTACCAGCAGAAGCCAGGACAGGCCC
CTCTACTTGTCATCTATGGTGACAACAGGCGGCCCTCAGGGATCCCAGACCGATTCTCTGGCTC
CAACTCAGGAAACACAGCTTCCTTGACCATCACTGGGACTCAGGCGGAAGATGAGGCTGACTAC
TATTGTAATTCTCGGGACAGTAGTGGTCACCATAATGTGCTATTCGGCGGGGGGACCAAGCTGA
CCGTCCTA
**SEQ ID NO: 79** - nucleotide sequence encoding the VH domain of A5 
GAGGTGCAGCTGGTGGAGTCTGGGGGAGGCTTGGTCCAGCCTGGGGGGTCCCTGAGACTCTC
CTGTGCAGCCTCTGGATTCACCGTCAGTAGCAACTACATGAGCTGGGTCCGCCAGGCTCCAGG
GAAGGGGCTGGAGTGGGTCTCAGTTATTTATAGCGGTGGTAGCACATACTACGCAGACTCCGTG
AAGGGCCGATTCACCATCTCCAGAGACAACGCCAAGAACTCACTGTATCTGCAAATGAACAGCC
TGAGAGCTGAGGACACGGCTGTGTATTACTGTGCGAGGGGTTGGACGACTTTTGACTACTGGGG
CCAGGGAACCCTGGT CACCGTCTCCTCAG
**SEQ ID NO: 80** - nucleotide sequence encoding the VL domain of A5 
CAGGCTGTGCTGACTCAGCCCCCCTCACTGTCTGTGTCCCCAAGACAGACAGTCACCATCACCT
GCTCTGGAGATCAACTGGGAGATAGATATGTTTCCTGGTATCAGCAGAAGCCAGGCCAGTCCCC
TATGTTGGTCATCTATCAAGATGACAGGCGGCCCTCAGGGGTCCCTGAGCGATTCTCTGGCTCC
AACTCTGGGAACACAGCCACTCTGACCATCGGCGGGACCCAGGCTATGGATCAGGCTGATTATT
ATTGTCAAACGTGGGACAACAGTCCTGTGGTGTTTGGCGGGGGGACCAA ATTGACCGTCCTAG
**SEQ ID NO: 81** - nucleotide sequence encoding the VH domain of F5 
GAAGTGCAGCTGGTGCAGTCTGGGGGAGGCGTGGTCCAGCCTGGGAGGTCCCTGAGACTCTCC
TGTGCAGCCTCTGGATTCACCTTCAGTAGCTATGGCATGCACTGGGTCCGCCAGGCTCCAGGCA
AGGGGCTGGAGTGGGTGGCAGTTATTTCATATGATGGAAGTAATAAATACTATGCAGACTCCGTG
AAGGGCCGATTCACCATCTCCAGAGACAATTCCAAGAACACGCTGTATCTGCAAATGAACAGCCT
GAGAGCTGAGGACACGGCTGTGTATTATTGTGCGAAAGATCTTACCCGAAGTGGTTATCCTTCCT
ACTACTTTGACTACTGGGGCCAGGGAACCCTGGTCACC GTCTCCTCAG
**SEQ ID NO: 82** - nucleotide sequence encoding the VL domain of F5 
TCTTCTGAGCTGACTCAGGACCCTGCTGTGTCTGTGGCCTTGGGACAGACAGTCAGGATCACAT
GCCAAGGAGACAGCCTCAGAAGCTATTATGCAAGCTGGTACCAGCAGAAGCCAGGACAGGCCC
CTGTACTTGTCATCTATGGTAAAAACAACCGGCCCTCAGGGATCCCAGACCGATTCTCTGGCTCC
AGCTCAGGAAACACAGCTTCCTTGACCATCACTGGGGCTCAGGCGGAAGATGAGGCTGACTATT
ACTGTAACTCCCGGGGCAGCAGTGGTAACAATTGGGTGTTCGGCGGAGG
GACCAAGCTGACCGTCCTAG
**SEQ ID NO: 83** - nucleotide sequence encoding the VH domain of D3 
CAAGTGCAGCTGGTGGAGTCTGGGGGAGGCTTGGTACAGCCTGGGGGGTCCCTGAGACTCTCC
TGTGCAGCCTCTGGATTCACCTTTAGCAGCAATGCCATGAGTTGGGTCCGCCAGGCTCCAGGGA
AGGGGCTGGAGTGGGTCTCAGCTATTGGTACTGGTGGTACCACATACTACGCAGACTCCGTGAA
GGGCCGGTTCACCATCTCCAGAGACAATTCCAAGAACACGCTGTATCTGCAAATGAACAGCCTG
AGAGCCGAGGACACGGCCGTATATTACTGTGCGAAAACGGGCCAACTTGACTACTGGGGCCAG
GGAACCCTGGTCACCGTCTCCTCAG
**SEQ ID NO: 84** - nucleotide sequence encoding the VL domain of D3 
CAGTCTGCCCTGACTCAGCCTGCCTCCGTGTCTGGGTCTCCTGGACAGTCGATCACCATCTCCT
GCACTGGAACCAGCAGTGACGTTGGTGGTTATAACTATGTCTCCTGGTTCCAACAGCACCCAGA
CAAAGCCCCCAAACTCATGATTTATGATGTCAGTAATCGGCCCTCAGGGGTTTCTAATCGCTTCT
CTGGCTCCAAGTCTGGCACCTCAGCCTCCCTGGCCATCAGTGGGCTCCGGTCCGAGGATGAGG
CTGATTATTACTGTGCAGCATGGGATGACAGCCTGAGTGGGTGGGTGTTCGGC
GGAGGGACCAAGCTGACCGTCCTAG
**SEQ ID NO: 85 -** nucleotide sequence encoding the VH domain of D4 
CAGGTGCAGCTGGTACAGTCTGGGGGAGGCTTAGTACAGCCTGGGGGGTCCCTGAGACTCTCC
TGTGCAGCCTCTGGATTCACCTTTAGTAACTATGCCATGAGCTGGGTCCGCCAGGCTCCAGGGA
AGGGGCTGGAGTGGGTCTCAGGTATTAGTGGTGGTGATAACACATACTACACAGACTCCGT
GAAGGGCCGCTTCACCATCTCCAGAGACAATTCCAAGAACACGCTGTATCTGCAAATGAACAGC
CTGACAGCCGAGGACACGGCCCTATATTACTGTGCGAAAAAACAAAGCGGAGGTAGTGGCTGGT
CCCTCTGGGGATTCGACTACTGGGGCCAGGGAACCCTGGTCACCGTCTCCTCAG
**SEQ ID NO: 86** - nucleotide sequence encoding the VL domain of D4 
CAGGCTGTGGTGACCCAGGAGCCCTCACTGACTGTGTCCCCAGGAGGGACAGTCACTCTCACC
TGTGCTTCCAGCACTGGAGCAGTCACCAGTGGTTACTATCCAAGCTGGTTCCAGCAGAAACCTG
GACAAGCACCCAGGCCACTAATTTATAATACAAACAACAAACACTCCTGGAGTCCTGCCCGGTTC
TCAGGCTCCCTCCTTGGGGGCAAAGCTGCCCTGACACTGTCAGGTGTGCAGCCTGAGGACGAG
GCTGACTATTACTGCCTGCTCTACTATGGTGGGCGGGGAGTATTCGGCGGAGGGACCAGGCTG
ACCGTCCTA
**SEQ ID NO: 87** - nucleotide sequence encoding the VH domain of F7 
GAGGTGCAGCTGTTGGAGTCTGGGGGAGGCTTGGTACAGCCTGGGGGGTCCCTGAGACTCTCC
TGTGCAGCCTCTGGATTCACCTTTAGCAGCTATGCCATGAGCTGGGTCCGCCAGGCTCCAGGGA
AGGGGCTGGA
GTGGGTCTCAGCTATTAGTGGTAGTGGTGGTAGCACATACTACGCAGACTCCGTGAAGGGCCGG
TTCACCATCTCCAGAGACAATTCCAAGAACACGCTGTATCTGCAAATGAACAGCCTGAGAGCCGA
GGACACGGCCGTATATTACTGTGCGAAAAATCCCATACAGCTATGGTCTAAAATAAAGAACTGGT
TCGACCCCTGGGGCCAGGGAACCCTGGT CACCGTCTCCTCAG
**SEQ ID NO: 88** - nucleotide sequence encoding the VL domain of F7 
CAGGCTGTGGTGACTCAAGAGCCCTCACTAAGTGTGTCCCCAGGAGGGACAGTCAGTCTCACCT
GTGGCTCCAGCGCTGGAGATGTCACCTATGGTCACTTTCCCTACTGGTACCAGCAGAAGCCTGG
CCAAGCCCCCAGAATGCTCATTTATGATGTAAACAACAGAGCGTCCTGGACCCCTGCCCGGTTC
TCAGGCTCCCTCGTTGGGGGCAAGGCTGCCCTGACCCTTTCGGGTGCGCAGCCTGGGGATGAG
GCTGACTATTACTGCGCCCTCTCCTATTTTGGTTCTATGTTTTTCGGCGGAGG
GACCAAGCTGACCGTCCT
**SEQ ID NO: 89** - nucleotide sequence encoding the VH domain of D2 
GAGGTGCAGCTGGTGCAGTCTGGGGGAGGCGTGGTCCAGCCTGGGAGGTCCCTGAGACTCTC
CTGTGCAGCCTCTGGATTCACCTTCAGTAGCTATGGCATGCACTGGGTCCGCCAGGCTCCAGGC
AAGGGGCTGGAGTGGGTGGCAGTTATTTCATATGATGGAAGTAATAAATACTATGCAGACTCCGT
GAAGGGCCGATTCACCATCTCCAGAGACAATTCCAAGAACACGCTGTATCTGCAAATGAACAGC
CTGAGAGCTGAGGACACGGCTGTGTATTATTGTGCGAAAGATCTTACCCGAAGTGGTTATCCTTC
CTACTACTTTGACTACTGGGGCCAGGGAACCCTGGTCACCGTCTCCTCA
**SEQ ID NO: 90** - nucleotide sequence encoding the VL domain of D2 
TCTTCTGAGCTGACTCAGGACCCTGCTGTGTCTGTGGCCTTGGGACGGACAGTCAGGATCACAT
GCCAAGGAGACAGCCTCAGAAGCTATTATGCAAGCTGGTACCAGCAGAAGCCAGGACAGGCCC
CTGTACTTGTCATCTATGGTAAAAACAACCGGCCCTCAGGGATCCCAGACCGATTCTCTGGCTCC
AGCTCAGGAAACACAGCTTCCTTGACCATCACTGGGGCTCAGGCGGAAGATGAGGCTGACTATT
ACTGTAACTCCCGGGACAGCAGTGGTAACCTAGTATTCGGCGGAGGGACCAAGCTGACCGTCCT
A
**SEQ ID NO: 91** - amino acid sequence of an antigen recognition domain including the VH and VL sequences of G3 
QVQLVQSGGGVVQPGRSLRLSCAASGFTFSSYGMHWVRQAPGKGLEWVAVISYDGSNKYYADSVK
GRFTISRDNSKNTLYLQMNSLRAEDTAVYYCAKDRGAVAGTDWYFDLWGRGTLVTVSSGGGGSGG
GGSGGGGSSSELTQDPAVSVALGQTVRITCQGDSLRSYYASWYQQKPGQAPVLVIYGKNNRPSGIP
DRFSGSSSGNTASLTITGAQAEDEADYYCNSRDSSGNHVVFGGGTKLTVL
**SEQ ID NO: 92** - amino acid sequence of an antigen recognition domain including the VH and VL sequences of G9 
QVQLVQSGGGLVQPGGSLRLSCAASGFTFSSYSMSWVRQAPGKGLEWVLGITRGGNTFYADSVTG
RFTISRDISKNTVFLQMDSLRVEDTAIYYCAKDWTMSGINVFDTWGQGTLVTVSSGGGGSGGGGSG
GGGSQAVLTQPPSVSGSPGQSVTISCTGTSSDVGAYNRVSWYQQPPGTAPKLMIYEVNNRPSGVP
DRFSGSKSGNTASLTISGLQAEDEADYYCSSSTTSSTYVFGTGTKVTVL
**SEQ ID NO: 93** - amino acid sequence of an antigen recognition domain including the VH and VL sequences of G11 
EVQLQQSGGGLVQPGGSLRLSCAASEFPFSSYAMSWVRQAPGKGLAWVSAISSSGGDTYYADSVK
GRFTISRDNSKNTLYLQMNSLRAEDTAVYYCAKNWNLDYWGQGTLVTVSSGGGGSGGGGSGGGG
SSSELTQDPAVSVALGQTVMITCQGHSLRSYYASWYQQKPGQAPLLVIYGDNRRPSGIPDRFSGSNS
GNTASLTITGTQAEDEADYYCNSRDSSGHHNVLFGGGTKLTVL
**SEQ ID NO: 94** - amino acid sequence of an antigen recognition domain including the VH and VL sequences of A5 
EVQLVESGGGLVQPGGSLRLSCAASGFTVSSNYMSWVRQAPGKGLEWVSVIYSGGSTYYADSVKG
RFTISRDNAKNSLYLQMNSLRAEDTAVYYCARGWTTFDYWGQGTLVTVSSGGGGSGGGGSGGGG
SQAVLTQPPSLSVSPRQTVTITCSGDQLGDRYVSWYQQKPGQSPMLVIYQDDRRPSGVPERFSGSN
SGNTATLTIGGTQAMDQADYYCQTWDNSPVVFGGGTKLTVL
**SEQ ID NO: 95** - amino acid sequence of an antigen recognition domain including the VH and VL sequences of F5 
EVQLVQSGGGVVQPGRSLRLSCAASGFTFSSYGMHWVRQAPGKGLEWVAVISYDGSNKYYADSVK
GRFTISRDNSKNTLYLQMNSLRAEDTAVYYCAKDLTRSGYPSYYFDYWGQGTLVTVSSGGGGSGG
 
GGSGGGGSSSELTQDPAVSVALGQTVRITCQGDSLRSYYASWYQQKPGQAPVLVIYGKNNRPSGIP
DRFSGSSSGNTASLTITGAQAEDEADYYCNSRGSSGNNWVFGGGTKLTVL
**SEQ ID NO: 96** - amino acid sequence of an antigen recognition domain including the VH and VL sequences of D3 
QVQLVESGGGLVQPGGSLRLSCAASGFTFSSNAMSWVRQAPGKGLEWVSAIGTGGTTYYADSVKG
RFTISRDNSKNTLYLQMNSLRAEDTAVYYCAKTGQLDYWGQGTLVTVSSGGGGSGGGGSGGGGS
QSALTQPASVSGSPGQSITISCTGTSSDVGGYNYVSWFQQHPDKAPKLMIYDVSNRPSGVSNRFSG
SKSGTSASLAISGLRSEDEADYYCAAWDDSLSGWVFGGGTKLTVL
**SEQ ID NO: 97** - amino acid sequence of an antigen recognition domain including the VH and VL sequences of D4 
QVQLVQSGGGLVQPGGSLRLSCAASGFTFSNYAMSWVRQAPGKGLEWVSGISGGGDNTYYTDSVK
GRFTISRDNSKNTLYLQMNSLTAEDTALYYCAKKQSGGSGWSLWGFDYWGQGTLVTVSSGGGGSG
GGGSGGGGSQAVVTQEPSLTVSPGGTVTLTCASSTGAVTSGYYPSWFQQKPGQAPRPLIYNTNNK
HSWSPARFSGSLLGGKAALTLSGVQPEDEADYYCLLYYGGRGVFGGGTRLTVL
**SEQ ID NO: 98** - amino acid sequence of an antigen recognition domain including the VH and VL sequences of F7 
EVQLLESGGGLVQPGGSLRLSCAASGFTFSSYAMSWVRQAPGKGLEWVSAISGSGGSTYYADSVK
GRFTISRDNSKNTLYLQMNSLRAEDTAVYYCAKNPIQLWSKIKNWFDPWGQGTLVTVSSGGGGSGG
GGSGGGGSQAVVTQEPSLSVSPGGTVSLTCGSSAGDVTYGHFPYWYQQKPGQAPRMLIYDVNNRA
SWTPARFSGSLVGGKAALTLSGAQPGDEADYYCALSYFGSMFFGGGTKLTVL
**SEQ ID NO: 99** - amino acid sequence of an antigen recognition domain including the VH and VL sequences of D2 
EVQLVQSGGGVVQPGRSLRLSCAASGFTFSSYGMHWVRQAPGKGLEWVAVISYDGSNKYYADSVK
GRFTISRDNSKNTLYLQMNSLRAEDTAVYYCAKDLTRSGYPSYYFDYWGQGTLVTVSSGGGGSGG
GGSGGGGSSSELTQDPAVSVALGRTVRITCQGDSLRSYYASWYQQKPGQAPVLVIYGKNNRPSGIP
DRFSGSSSGNTASLTITGAQAEDEADYYCNSRDSSGNLVFGGGTKLTVL
**SEQ ID NO: 100** - nucleotide sequence encoding G3 plus a signal sequence and a tag (the sequence encoding the signal sequence and tag are underlined) 
ATGAAATACCTATTGCCTACGGCAGCCGCTGGCTTGCTGCTGCTGGCAGCTCAGCCGGCCATGG
CCCAGGTGCAGCTGGTGCAGTCTGGGGGAGGCGTGGTCCAGCCTGGGAGGTCCCTGAGACTC
TCCTGTGCAGCCTCTGGATTCACCTTCAGTAGCTATGGCATGCACTGGGTCCGCCAGGCTCCAG
GCAAGGGGCTGGAGTGGGTGGCAGTTATATCATATGATGGAAGTAATAAATACTATGCAGACTC
CGTGAAGGGCCGATTCACCATCTCCAGAGACAATTCCAAGAACACGCTGTATCTGCAAATGAACA
GCCTGAGAGCTGAGGACACGGCTGTGTATTACTGTGCGAAAGATCGGGGAGCAGTGGCTGGGA
CTGACTGGTACTTCGATCTCTGGGGCCGTGGCACCCTGGTCACTGTCTCCTCAGGGAGTGCATC
 
CGCCCCAAAGCTTGAAGAAGGTGAATTTTCAGAAGCACGCGTATCTTCTGAGCTGACTCAGGAC
CCTGCTGTGTCTGTGGCCTTGGGACAGACAGTCAGGATCACATGCCAAGGAGACAGCCTCAGAA
GCTATTATGCAAGCTGGTACCAGCAGAAGCCAGGACAGGCCCCTGTACTTGTCATCTATGGTAA
AAACAACCGGCCCTCAGGGATCCCAGACCGATTCTCTGGCTCCAGCTCAGGAAACACAGCTTCC
TTGACCATCACTGGGGCTCAGGCGGAAGATGAGGCTGACTATTACTGTAACTCCCGGGACAGCA
GTGGTAACCATGTGGTATTCGGCGGAGGGACCAAGCTGACCGTCCTAGGTCAGCCCAAGGCTG
CCCCCTCGGTCACTCTGTTCCCACCCTCCTCTGCGGCCGCAGGTTCTGAACAAAAGCTGATCTC
AGAAGAAGATCTATCCCATCATCACCATCATCAT
**SEQ ID NO: 101** - nucleotide sequence encoding G9 plus a signal sequence and a tag (the sequence encoding the signal sequence and tag are underlined) 
ATGAAATACCTATTGCCTACGGCAGCCGCTGGCTTGCTGCTGCTGGCAGCTCAGCCGGCCATGG
CCCAGGTCCAGCTGGTGCAATCTGGGGGAGGCTTGGTACAGCCGGGGGGGTCCCTGAGACTCT
CCTGTGCAGCCTCTGGATTCACCTTCAGTAGCTATAGCATGAGCTGGGTCCGCCAGGCCCCAGG
GAAGGGGCTGGAGTGGGTCTTAGGTATTACTCGTGGTGGTAACACATTCTACGCAGACTCCGTG
ACGGGCCGGTTCACCATCTCCAGAGACATTTCCAAGAACACGGTGTTTCTGCAAATGGACAGCC
TGAGAGTCGAGGACACGGCCATTTATTACTGTGCGAAAGACTGGACCATGAGTGGAATCAACGT
CTTCGACACCTGGGGCCAGGGGACCCTGGTCACCGTCTCCTCCGGGAGTGCATCCGCCCCAAA
GCTTGAAGAAGGTGAATTTTCAGAAGCACGCGTACAGGCAGTGCTGACTCAGCCACCCTCCGTG
TCCGGGTCTCCTGGACAGTCAGTCACCATCTCCTGCACTGGAACCAGCAGTGACGTTGGTGCTT
ATAACCGTGTCTCCTGGTACCAGCAGCCCCCAGGCACAGCCCCCAAACTTATGATTTATGAGGT
CAATAATCGGCCCTCAGGGGTCCCTGATCGCTTCTCTGGGTCCAAGTCTGGCAACACGGCCTCC
CTGACCATCTCTGGGCTCCAGGCTGAGGACGAGGCTGATTATTACTGCAGCTCATCTACAACCA
GTAGCACTTATGTCTTCGGAACTGGGACCAAGGTCACCGTCCTAGGTCAGCCCAAGGCCAACCC
CACTGTCACTCTGTTCCCACCCTCCTCTGCGGCCGCAGGTTCTGAACAAAAGCTGATCTCAGAA
GA AGATCTATCCCATCATCACC ATCATCAT
**SEQ ID NO: 102** - nucleotide sequence encoding G11 plus a signal sequence and a tag (the sequence encoding the signal sequence and tag are underlined) 
ATGAAATACCTATTGCCTACGGCAGCCGCTGGCTTGCTGCTGCTGGCAGCTCAGCCGGCCATGG
CCGAAGTGCAGCTACAGCAGTCCGGGGGAGGCTTGGTACAGCCTGGGGGGTCCCTGAGACTCT
CCTGTGCAGCCTCTGAATTCCCCTTTAGCAGCTATGCCATGAGCTGGGTCCGCCAGGCTCCAGG
GAAGGGGCTGGCGTGGGTCTCAGCTATTAGTAGTAGTGGTGGTGACACATACTACGCAGACTCC
GTGAAGGGCCGGTTCACCATCTCCAGAGACAATTCCAAGAACACGCTGTATCTGCAAATGAACA
GCCTGAGAGCCGAGGACACGGCCGTATATTACTGTGCGAAAAACTGGAACCTTGACTACTGGGG
CCAGGGAACCCTGGTCACCGTCTCCTCAGGGAGTGCATCCGCCCCAAAGCTTGAAGAAGGTGA
ATTTTCAGAAGCACGCGTATCTTCTGAGCTGACTCAGGACCCTGCTGTGTCTGTGGCCTTGGGA
CAGACAGTCATGATAACATGCCAAGGACACAGCCTCCGAAGCTATTATGCAAGCTGGTACCAGC
AGAAGCCAGGACAGGCCCCTCTACTTGTCATCTATGGTGACAACAGGCGGCCCTCAGGGATCCC
AGACCGATTCTCTGGCTCCAACTCAGGAAACACAGCTTCCTTGACCATCACTGGGACTCAGGCG
 
GAAGATGAGGCTGACTACTATTGTAATTCTCGGGACAGTAGTGGTCACCATAATGTGCTATTCGG
CGGGGGGACCAAGCTGACCGTCCTAAGTCAGCCCAAGGCTGCCCCCTCGGTCACTCTGTTCCC
ACCCTCCTCTGCGGCCGCAGGTTCTGAACAAAAGCTGATCTCAGAAGAAGATCTATCCCATCATC
ACCATCATCAT
**SEQ ID NO: 103** - nucleotide sequence encoding A5 plus a signal sequence and a tag (the sequence encoding the signal sequence and tag are underlined) 
ATGAAATACCTATTGCCTACGGCAGCCGCTGGCTTGCTGCTGCTGGCAGCTCAGCCGGCCATGG
CCGAGGTGCAGCTGGTGGAGTCTGGGGGAGGCTTGGTCCAGCCTGGGGGGTCCCTGAGACTC
TCCTGTGCAGCCTCTGGATTCACCGTCAGTAGCAACTACATGAGCTGGGTCCGCCAGGCTCCAG
GGAAGGGGCTGGAGTGGGTCTCAGTTATTTATAGCGGTGGTAGCACATACTACGCAGACTCCGT
GAAGGGCCGATTCACCATCTCCAGAGACAACGCCAAGAACTCACTGTATCTGCAAATGAACAGC
CTGAGAGCTGAGGACACGGCTGTGTATTACTGTGCGAGGGGTTGGACGACTTTTGACTACTGGG
GCCAGGGAACCCTGGTCACCGTCTCCTCAGGGAGTGCATCCGCCCCAAAGCTTGAAGAAGGTG
AATTTTCAGAAGCACGCGTACAGGCTGTGCTGACTCAGCCCCCCTCACTGTCTGTGTCCCCAAG
ACAGACAGTCACCATCACCTGCTCTGGAGATCAACTGGGAGATAGATATGTTTCCTGGTATCAGC
AGAAGCCAGGCCAGTCCCCTATGTTGGTCATCTATCAAGATGACAGGCGGCCCTCAGGGGTCCC
TGAGCGATTCTCTGGCTCCAACTCTGGGAACACAGCCACTCTGACCATCGGCGGGACCCAGGCT
ATGGATCAGGCTGATTATTATTGTCAAACGTGGGACAACAGTCCTGTGGTGTTTGGCGGGGGGA
CCAAATTGACCGTCCTAGGTCAGCCCAAGGCTGCCCCCTCGGTCACTCTGTTCCCACCCTCCTC
TGCGGCCGCAGGTTCTGAACAAAAGCTGATCTCAGAAGAAGATCTATCCCATCATCACCATCATC
AT
**SEQ ID NO: 104** - nucleotide sequence encoding F5 plus a signal sequence and a tag (the sequence encoding the signal sequence and tag are underlined) 
ATGAAATACCTATTGCCTACGGCAGCCGCTGGCTTGCTGCTGCTGGCAGCTCAGCCGGCCATGG
CCGAAGTGCAGCTGGTGCAGTCTGGGGGAGGCGTGGTCCAGCCTGGGAGGTCCCTGAGACTCT
CCTGTGCAGCCTCTGGATTCACCTTCAGTAGCTATGGCATGCACTGGGTCCGCCAGGCTCCAGG
CAAGGGGCTGGAGTGGGTGGCAGTTATTTCATATGATGGAAGTAATAAATACTATGCAGACTCCG
TGAAGGGCCGATTCACCATCTCCAGAGACAATTCCAAGAACACGCTGTATCTGCAAATGAACAGC
CTGAGAGCTGAGGACACGGCTGTGTATTATTGTGCGAAAGATCTTACCCGAAGTGGTTATCCTTC
CTACTACTTTGACTACTGGGGCCAGGGAACCCTGGTCACCGTCTCCTCAGGGAGTGCATCCGCC
CCAAAGCTTGAAGAAGGTGAATTTTCAGAAGCACGCGTATCTTCTGAGCTGACTCAGGACCCTG
CTGTGTCTGTGGCCTTGGGACAGACAGTCAGGATCACATGCCAAGGAGACAGCCTCAGAAGCTA
TTATGCAAGCTGGTACCAGCAGAAGCCAGGACAGGCCCCTGTACTTGTCATCTATGGTAAAAACA
ACCGGCCCTCAGGGATCCCAGACCGATTCTCTGGCTCCAGCTCAGGAAACACAGCTTCCTTGAC
CATCACTGGGGCTCAGGCGGAAGATGAGGCTGACTATTACTGTAACTCCCGGGGCAGCAGTGG
TAACAATTGGGTGTTCGGCGGAGGGACCAAGCTGACCGTCCTAGGTCAGCCCAAGGCTGCCCC
CTCGGTCACTCTGTTCCCGCCCTCCTCTGCGGCCGCAGGTTCTGAACAAAAGCTGATCTCAGA
AGAAGATCTATCCCATCATC ACCATCATCAT
**SEQ ID NO: 105** - nucleotide sequence encoding D3 plus a signal sequence and a tag (the sequence encoding the signal sequence and tag are underlined) 
ATGAAATACCTATTGCCTACGGCAGCCGCTGGCTTGCTGCTGCTGGCAGCTCAGCCGGCCATGG
CCCAAGTGCAGCTGGTGGAGTCTGGGGGAGGCTTGGTACAGCCTGGGGGGTCCCTGAGACTCT
CCTGTGCAGCCTCTGGATTCACCTTTAGCAGCAATGCCATGAGTTGGGTCCGCCAGGCTCCAGG
GAAGGGGCTGGAGTGGGTCTCAGCTATTGGTACTGGTGGTACCACATACTACGCAGACTCCGTG
AAGGGCCGGTTCACCATCTCCAGAGACAATTCCAAGAACACGCTGTATCTGCAAATGAACAGCC
TGAGAGCCGAGGACACGGCCGTATATTACTGTGCGAAAACGGGCCAACTTGACTACTGGGGCC
AGGGAACCCTGGTCACCGTCTCCTCAGGGAGTGCATCCGCCCCAAAGCTTGAAGAAGGTGAATT
TTCAGAAGCACGCGTACAGTCTGCCCTGACTCAGCCTGCCTCCGTGTCTGGGTCTCCTGGACAG
TCGATCACCATCTCCTGCACTGGAACCAGCAGTGACGTTGGTGGTTATAACTATGTCTCCTGGTT
CCAACAGCACCCAGACAAAGCCCCCAAACTCATGATTTATGATGTCAGTAATCGGCCCTCAGGG
GTTTCTAATCGCTTCTCTGGCTCCAAGTCTGGCACCTCAGCCTCCCTGGCCATCAGTGGGCTCC
GGTCCGAGGATGAGGCTGATTATTACTGTGCAGCATGGGATGACAGCCTGAGTGGGTGGGTGTT
CGGCGGAGGGACCAAGCTGACCGTCCTAGGTCAGCCCAAGGCTGCCCCCTCGGTCACTCTGTT
CCCACCCTCCTCTGCGGCCGCAGGTTCTGAACAAAAGCTGATCTCAGAAGAAGATCTATCCCAT
CA TCACCATCATCAT
**SEQ ID NO: 106 -** nucleotide sequence encoding D4 plus a signal sequence and a tag (the sequence encoding the signal sequence and tag are underlined) 
ATGAAATACCTATTGCCTACGGCAGCCGCTGGCTTGCTGCTGCTGGCAGCTCAGCCGGCCATGG
CCCAGGTGCAGCTGGTACAGTCTGGGGGAGGCTTAGTACAGCCTGGGGGGTCCCTGAGACTCT
CCTGTGCAGCCTCTGGATTCACCTTTAGTAACTATGCCATGAGCTGGGTCCGCCAGGCTCCAGG
GAAGGGGCTGGAGTGGGTCTCAGGTATTAGTGGTGGTGATAACACATACTACACAGACTCC
GTGAAGGGCCGCTTCACCATCTCCAGAGACAATTCCAAGAACACGCTGTATCTGCAAATGAACA
GCCTGACAGCCGAGGACACGGCCCTATATTACTGTGCGAAAAAACAAAGCGGAGGTAGTGGCT
GGTCCCTCTGGGGATTCGACTACTGGGGCCAGGGAACCCTGGTCACCGTCTCCTCAGGGAGTG
CATCCGCCCTAAAGCTTGAAGAAGGTGAATTTTCAGAAGCACGCGTACAGGCTGTGGTGACCCA
GGAGCCCTCACTGACTGTGTCCCCAGGAGGGACAGTCACTCTCACCTGTGCTTCCAGCACTGGA
GCAGTCACCAGTGGTTACTATCCAAGCTGGTTCCAGCAGAAACCTGGACAAGCACCCAGGCCAC
TAATTTATAATACAAACAACAAACACTCCTGGAGTCCTGCCCGGTTCTCAGGCTCCCTCCTTGGG
GGCAAAGCTGCCCTGACACTGTCAGGTGTGCAGCCTGAGGACGAGGCTGACTATTACTGCCTG
CTCTACTATGGTGGGCGGGGAGTATTCGGCGGAGGGACCAGGCTGACCGTCCTAAGTCAGCCC
AAGGCTGCCCCCTCGGTCACTCTGTTCCCACCCTCCTCTGCGGCCGCAGGTTCTGAACAAAAGC
T GATCTCAGAAGAAGATCTAT CCCATCATCACCATCATCAT
**SEQ ID NO: 107** - nucleotide sequence encoding F7 plus a signal sequence and a tag (the sequence encoding the signal sequence and tag are underlined) 
ATGAAATACCTATTGCCTACGGCAGCCGCTGGCTTGCTGCTGCTGGCAGCTCAGCCGGCCATGG
CCGAGGTGCAGCTGTTGGAGTCTGGGGGAGGCTTGGTACAGCCTGGGGGGTCCCTGAGACTCT
CCTGTGCAGCCTCTGGATTCACCTTTAGCAGCTATGCCATGAGCTGGGTCCGCCAGGCTCCAGG
GAAGGGGCTGGAGTGGGTCTCAGCTATTAGTGGTAGTGGTGGTAGCACATACTACGCAGACTCC
GTGAAGGGCCGGTTCACCATCTCCAGAGACAATTCCAAGAACACGCTGTATCTGCAAATGAACA
GCCTGAGAGCCGAGGACACGGCCGTATATTACTGTGCGAAAAATCCCATACAGCTATGGTCTAA
AATAAAGAACTGGTTCGACCCCTGGGGCCAGGGAACCCTGGTCACCGTCTCCTCAGGGAGTGC
ATCCGCCCCAAAGCTTGAAGAAGGTGAATTTTCAGAAGCACGCGTACAGGCTGTGGTGACTCAA
GAGCCCTCACTAAGTGTGTCCCCAGGAGGGACAGTCAGTCTCACCTGTGGCTCCAGCGCTGGA
GATGTCACCTATGGTCACTTTCCCTACTGGTACCAGCAGAAGCCTGGCCAAGCCCCCAGAATGC
TCATTTATGATGTAAACAACAGAGCGTCCTGGACCCCTGCCCGGTTCTCAGGCTCCCTCGTTGG
GGGCAAGGCTGCCCTGACCCTTTCGGGTGCGCAGCCTGGGGATGAGGCTGACTATTACTGCGC
CCTCTCCTATTTTGGTTCTATGTTTTTCGGCGGAGGGACCAAGCTGACCGTCCTCAGTCAGCCCA
AGGCTGCCCCCTCGGTCACTCTGTTCCCACCCTCCTCTGCGGCCGCAGGTTCTGAACAAAAGCT
GATC TCAGAAGAAGATCTATCCCA TCATCACCATCATCAT
**SEQ ID NO: 108** - nucleotide sequence encoding D2 plus a signal sequence and a tag (the sequence encoding the signal sequence and tag are underlined) 
ATGAAATACCTATTGCCTACGGCAGCCGCTGGCTTGCTGCTGCTGGCAGCTCAGCCGGCCATGG
CCGAGGTGCAGCTGGTGCAGTCTGGGGGAGGCGTGGTCCAGCCTGGGAGGTCCCTGAGACTC
TCCTGTGCAGCCTCTGGATTCACCTTCAGTAGCTATGGCATGCACTGGGTCCGCCAGGCTCCAG
GCAAGGGGCTGGAGTGGGTGGCAGTTATTTCATATGATGGAAGTAATAAATACTATGCAGACTCC
GTGAAGGGCCGATTCACCATCTCCAGAGACAATTCCAAGAACACGCTGTATCTGCAAATGAACA
GCCTGAGAGCTGAGGACACGGCTGTGTATTATTGTGCGAAAGATCTTACCCGAAGTGGTTATCC
TTCCTACTACTTTGACTACTGGGGCCAGGGAACCCTGGTCACCGTCTCCTCAGGGAGTGCATCC
GCCCCAAAGCTTGAAGAAGGTGAATTTTCAGAAGCACGCGTATCTTCTGAGCTGACTCAGGACC
CTGCTGTGTCTGTGGCCTTGGGACGGACAGTCAGGATCACATGCCAAGGAGACAGCCTCAGAA
GCTATTATGCAAGCTGGTACCAGCAGAAGCCAGGACAGGCCCCTGTACTTGTCATCTATGGTAA
AAACAACCGGCCCTCAGGGATCCCAGACCGATTCTCTGGCTCCAGCTCAGGAAACACAGCTTCC
TTGACCATCACTGGGGCTCAGGCGGAAGATGAGGCTGACTATTACTGTAACTCCCGGGACAGCA
GTGGTAACCTAGTATTCGGCGGAGGGACCAAGCTGACCGTCCTAGGTCAGCCCAAGGCTGCCC
CCTCGGTCACTCTGTTCCCGCCCTCCTCTGCGGCCGCAGGTTCTGAACAAAAGCTGATC
TCAGAAGAAGATCTATCCCA TCATCACCATCATCAT
**SEQ ID NO: 109** - representative linker sequence
   GGGS
**SEQ ID NO: 110** - representative linker sequence
   ETSGGGGSRL
**SEQ ID NO: 111** - representative linker sequence
   SGGGGSGGGGSGGGGS
**SEQ ID NO: 112** - representative linker sequence
   GGGGS
**SEQ ID NO: 113** - representative linker sequence
   GGGGGS
**SEQ ID NO: 114 -** representative linker sequence
   GGGGGGS
**SEQ ID NO: 115** - representative linker sequence
   GGGGSGGGGSGGGGS
**SEQ ID NO: 116** - representative linker sequence
   GGGGG
**SEQ ID NO: 117** - representative linker sequence
   GGGGSGGGGS
   **SEQ ID NO: 118** - representative linker sequence
   GGGGSGGGGSGGGGSGGGGS
**SEQ ID NO: 119** - representative linker sequence
   GGGGGG
**SEQ ID NO: 120** - representative linker sequence
   G₆
**SEQ ID NO: 121** - representative linker sequence
   G₈
**SEQ ID NO: 122** - representative linker sequence
   KESGSVSSEQLAQFRSLD
**SEQ ID NO: 123** - representative linker sequence
   EGKSSGSGSESKST
**SEQ ID NO: 124** - representative linker sequence
   GSAGSAAGSGEF
**SEQ ID NO: 125** - representative linker sequence
   SGGGGSAGSAAGSGEF
   **SEQ ID NO: 126** - representative linker sequence
   SGGGLLLLLLLLGGGS
**SEQ ID NO: 127** - representative linker sequence
   SGGGAAAAAAAAGGGS
**SEQ ID NO: 128** - representative linker sequence
   SGGGAAAAAAAAAAAAAAAAGGGS
**SEQ ID NO: 129** - representative linker sequence
   SGALGGLALAGLLLAGLGLGAAGS
   **SEQ ID NO: 130** - representative linker sequence
   SLSLSPGGGGGPAR
**SEQ ID NO: 131** - representative linker sequence
   SLSLSPGGGGGPARSLSLSPGGGGG
**SEQ ID NO: 132** - representative linker sequence
   GSSGSS
**SEQ ID NO: 133** - representative linker sequence
   GSSSSSS
**SEQ ID NO: 134** - representative linker sequence
   GGSSSS
**SEQ ID NO: 135** - representative linker sequence
   GSSSSS
**SEQ ID NO: 136** - representative linker sequence
   SGGGGS
**SEQ ID NO: 137** - amino acid sequence of the human CD8alpha transmembrane domain which represents amino acids 183 to 203 of human CD8α
   IYIWAPLAGTCGVLLLSLVIT
**SEQ ID NO: 138** - amino acid sequence of a CH2CH3 hinge domain 
PCPAPPVAGPSVFLFPPKPKDTLMIARTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSRDELTKNQ
VSLTCLVKGFYPSDIAVEWESNGQPENNYKTTPPVLDSDGSFFLYSKLTVDKSRWQQGNVFSCSVM
HEALHNHYTQKSLSLSPGK
**SEQ ID NO: 139** - amino acid sequence of a modified combined CD8alpha hinge and transmembrane domain (the transmembrane domain is underlined) 
FVPVFLPAKPTTTPAPRPPTPAPTIASQPLSLRPEASRPAAGGAVHTRGLDFADIYIWAPLAGTCGVL
LLSLVITLYCNHR
**SEQ ID NO: 140** - amino acid sequence of a modified CD8alpha hinge domain
   FVPVFLPAKPTTTPAPRPPTPAPTIASQPLSLRPEASRPAAGGAVHTRGLDFAD
**SEQ ID NO: 141** - amino acid sequence of wildtype CD8alpha combined hinge and transmembrane domain (the transmembrane domain is underlined) 
FVPVFLPAKPTTTPAPRPPTPAPTIASQPLSLRPEACRPAAGGAVHTRGLDFACDIYIWAPLAGTCGV
LLLSLVITLYCNHR
**SEQ ID NO: 142** - amino acid sequence of a CD28 hinge and transmembrane domain (the transmembrane domain is underlined)
   IEVMYPPPYLDNEKSNGTIIHVKGKHLCPSPLFPGPSKP**FWVLVVVGGVLACYSLLVTVAFIIFWV**
**SEQ ID NO: 143** - amino acid sequence of a signal/leader sequence
   MALPVTALLLPLALLLHAAAP
**SEQ ID NO: 144 -** amino acid sequence of the wildtype CD8alpha leader sequence
   MALPVTALLLPLALLLHAARP
**SEQ ID NO: 145** - amino acid sequence of the CD3 zeta intracellular domain 
RVKFSRSADAPAYQQGQNQLYNELNLGRREEYDVLDKRRGRDPEMGGKPRRKNPQEGLYNELQK
DKMAEAYSEIGMKGERRRGKGHDGLYQGLSTATKDTYDALHMQALPPR
**SEQ ID NO: 146** - amino acid sequence of a CD28 combined transmembrane and intracellular signalling domain (the transmembrane domain is underlined) 
FWVLVVVGGVLACYSLLVTVAFIIFWVRSKRSRLLHSDYMNMTPRRPGPTRKHYQPYAPPRDFAAYR
S
**SEQ ID NO: 147** - amino acid sequence of a CD28 intracellular signalling domain
   WVRSKRSRLLHSDYMNMTPRRPGPTRKHYQPYAPPRDFAAYRS
**SEQ ID NO: 148** - amino acid sequence of OX40 signalling domain
   ALYLLRRDQRLPPDAHKPPGGGSFRTPIQEEQADAHSTLAKI
**SEQ ID NO: 149** - amino acid sequence of 4-1BB signalling domain
   KRGRKKLLYIFKQPFMRPVQTTQEEDGCSCRFPEEEEGGCEL
**SEQ ID NO: 150** - amino acid sequence of ICOS signalling domain
   CWLTKKKYSSSVHDPNGEYMFMRAVNTAKKSRLTDVTL
**SEQ ID NO: 151** - amino acid sequence of TNFRSF25 signalling domain 
TYTYRHCWPHKPLVTADEAGMEALTPPPATHLSPLDSAHTLLAPPDSSEKICTVQLVGNSWTPGYPE
TQEALCPQVTWSWDQLPSRALGPAAAPTLSPESPAGSPAMMLQPGPQLYDVMDAVPARRWKEFVR
TLGLREAEIEAVEVEIGRFRDQQYEMLKRWRQQQPAGLGAVYAALERMGLDGCVEDLRSRLQRGP
**SEQ ID NO: 152** is the amino acid sequence which represents amino acid numbers 266 to 551 of human IL-2 receptor beta chain 
NCRNTGPWLKKVLKCNTPDPSKFFSQLSSEHGGDVQKWLSSPFPSSSFSPGGLAPEISPLEVLERDK
VTQLLLQQDKVPEPASLSSNHSLTSCFTNQGYFFFHLPDALEIEACQVYFTYDPYSEEDPDEGVAGA
PTGSSPQPLQPLSGEDDAYCTFPSRDDLLLFSPSLLGGPSPPSTAPGGSGAGEERMPPSLQERVPR
DWDPQPLGPPTPGVPDLVDFQPPPELVLREAGEEVPDAGPREGVSFPWSRPPGQGEFRALNARLP
LNTDAYLSLQELQGQDPTHLV
**SEQ ID NO: 153** is the amino acid sequence which represents a truncated and sequence modified variant of SEQ ID NO: 152 (Y510) 
NCRNTGPWLKKVLKCNTPDPSKFFSQLSSEHGGDVQKWLSSPFPSSSFSPGGLAPEISPLEVLERDK
VTQLLPLNTDAYLSLQELQGQDPTHLV
**SEQ ID NO: 154** is the amino acid sequence which represents a truncated and sequence modified variant of SEQ ID NO: 152 (Y510 and Y392) 
NCRNTGPWLKKVLKCNTPDPSKFFSQLSSEHGGDVQKWLSSPFPSSSFSPGGLAPEISPLEVLERDK
VTQLLDAYCTFPSRDDLLLFSPSLLGGPSPPSTAPGGSGAGEERMPPSLQERVPRDWDPQPLGPPT
PGVPDLVDFQPPPELVLREAGEEVPDAGPREGVSFPWSRPPGQGEFRALNARLPLNTDAYLSLQEL
QGQDPTHLV
**SEQ ID NO: 155** - amino acid sequence of wildtype FOXP3 (UniProtKB accession Q9BZS1)
**SEQ ID NO: 156** - amino acid sequence of a N and C terminally truncated FOXP3 fragment described within WO2019/241549 
GGAHASSSSL NPMPPSQLQL PTLPLVMVAP SGARLGPLPH LQALLQDRPH FMHQLSTVDA
HARTPVLQVH PLESPAMISL TPPTTATGVF SLKARPGLPP GINVASLEWV SREPALLCTF
PNPSAPRKDS TLSAVPQSSY PLLANGVCKW PGCEKVFEEP EDFLKHCQAD HLLDEKGRAQ
CLLQREMVQS LEQQLVLEKE KLSAMQAHLA GKMALTKASS VASSDKGSCC IVAAGSQGPV
VPAWSGPREA PDSLFAVRRH LWGSHGNSTF PEFLHNMDYF KFHNMRPPFT YATLIRWAIL
EAPEKQRTLN EIYHWFTRMF AFFRNHPATW KNAIRHNLSL HKCFVRVESE KGAVWTVDEL EF
**SEQ** ID NO: 157 - amino acid sequence of a FOXP3 variant having a mutation at amino acid 418 
MPNPRPGKPSAPSLALGPSPGASPSWRAAPKASDLLGARGPGGTFQGRDLRGGAHASSSSLNPMP
PSQLQLPTLPLVMVAPSGARLGPLPHLQALLQDRPHFMHQLSTVDAHARTPVLQVHPLESPAMISLT
PPTTATGVFSLKARPGLPPGINVASLEWVSREPALLCTFPNPSAPRKDSTLSAVPQSSYPLLANGVCK
WPGCEKVFEEPEDFLKHCQADHLLDEKGRAQCLLQREMVQSLEQQLVLEKEKLSAMQAHLAGKMA
LTKASSVASSDKGSCCIVAAGSQGPVVPAWSGPREAPDSLFAVRRHLWGSHGNSTFPEFLHNMDYF
KFHNMRPPFTYATLIRWAILEAPEKQRTLNEIYHWFTRMFAFFRNHPATWKNAIRHNLSLHKCFVRVE
SEKGAVWTVDELEFRKKREQRPSRCSNPTPGP
**SEQ** ID NO: 158 - amino acid sequence of a FOXP3 variant having a mutation at amino acid 422 
MPNPRPGKPSAPSLALGPSPGASPSWRAAPKASDLLGARGPGGTFQGRDLRGGAHASSSSLNPMP
PSQLQLPTLPLVMVAPSGARLGPLPHLQALLQDRPHFMHQLSTVDAHARTPVLQVHPLESPAMISLT
 
PPTTATGVFSLKARPGLPPGINVASLEWVSREPALLCTFPNPSAPRKDSTLSAVPQSSYPLLANGVCK
WPGCEKVFEEPEDFLKHCQADHLLDEKGRAQCLLQREMVQSLEQQLVLEKEKLSAMQAHLAGKMA
LTKASSVASSDKGSCCIVAAGSQGPVVPAWSGPREAPDSLFAVRRHLWGSHGNSTFPEFLHNMDYF
KFHNMRPPFTYATLIRWAILEAPEKQRTLNEIYHWFTRMFAFFRNHPATWKNAIRHNLSLHKCFVRVE
SEKGAVWTVDELEFRKKRSQRPARCSNPTPGP
**SEQ** ID NO: 159 - amino acid sequence of a FOXP3 variant having mutations at amino acids 418 and 422 
MPNPRPGKPSAPSLALGPSPGASPSWRAAPKASDLLGARGPGGTFQGRDLRGGAHASSSSLNPMP
PSQLQLPTLPLVMVAPSGARLGPLPHLQALLQDRPHFMHQLSTVDAHARTPVLQVHPLESPAMISLT
PPTTATGVFSLKARPGLPPGINVASLEWVSREPALLCTFPNPSAPRKDSTLSAVPQSSYPLLANGVCK
WPGCEKVFEEPEDFLKHCQADHLLDEKGRAQCLLQREMVQSLEQQLVLEKEKLSAMQAHLAGKMA
LTKASSVASSDKGSCCIVAAGSQGPVVPAWSGPREAPDSLFAVRRHLWGSHGNSTFPEFLHNMDYF
KFHNMRPPFTYATLIRWAILEAPEKQRTLNEIYHWFTRMFAFFRNHPATWKNAIRHNLSLHKCFVRVE
SEKGAVWTVDELEFRKKREQRPARCSNPTPGP
**SEQ ID NO: 160** - amino acid sequence of an illustrative FOXP3 polypeptide 
MPNPRPGKPSAPSLALGPSPGASPSWRAAPKASDLLGARGPGGTFQGRDLRGGAHASSSSLNPMP
PSQLQLPTLPLVMVAPSGARLGPLPHLQALLQDRPHFMHQLSTVDAHARTPVLQVHPLESPAMISLT
PPTTATGVFSLKARPGLPPGINVASLEWVSREPALLCTFPNPSAPRKDSTLSAVPQSSYPLLANGVCK
WPGCEKVFEEPEDFLKHCQADHLLDEKGRAQCLLQREMVQSLEQVEELSAMQAHLAGKMALTKAS
SVASSDKGSCCIVAAGSQGPVVPAWSGPREAPDSLFAVRRHLWGSHGNSTFPEFLHNMDYFKFHN
MRPPFTYATLIRWAILEAPEKQRTLNEIYHWFTRMFAFFRNHPATWKNAIRHNLSLHKCFVRVESEKG
AVWTVDELEFRKKRSQRPSRCSNPTPGPEGRGSLLTCGDVEEN
**SEQ ID NO: 161** - nucleotide sequence encoding an illustrative FOXP3 polypeptide 
ATGCCCAACCCCAGGCCTGGCAAGCCCTCGGCCCCTTCCTTGGCCCTTGGCCCATCCCCAGGA
GCCTCGCCCAGCTGGAGGGCTGCACCCAAAGCCTCAGACCTGCTGGGGGCCCGGGGCCCAGG
GGGAACCTTCCAGGGCCGAGATCTTCGAGGCGGGGCCCATGCCTCCTCTTCTTCCTTGAACCCC
ATGCCACCATCGCAGCTGCAGCTGCCCACACTGCCCCTAGTCATGGTGGCACCCTCCGGGGCA
CGGCTGGGCCCCTTGCCCCACTTACAGGCACTCCTCCAGGACAGGCCACATTTCATGCACCAGC
TCTCAACGGTGGATGCCCACGCCCGGACCCCTGTGCTGCAGGTGCACCCCCTGGAGAGCCCAG
CCATGATCAGCCTCACACCACCCACCACCGCCACTGGGGTCTTCTCCCTCAAGGCCCGGCCTG
GCCTCCCACCTGGGATCAACGTGGCCAGCCTGGAATGGGTGTCCAGGGAGCCGGCACTGCTCT
GCACCTTCCCAAATCCCAGTGCACCCAGGAAGGACAGCACCCTTTCGGCTGTGCCCCAGAGCTC
CTACCCACTGCTGGCAAATGGTGTCTGCAAGTGGCCCGGATGTGAGAAGGTCTTCGAAGAGCCA
GAGGACTTCCTCAAGCACTGCCAGGCGGACCATCTTCTGGATGAGAAGGGCAGGGCACAATGT
CTCCTCCAGAGAGAGATGGTACAGTCTCTGGAGCAGCAGCTGGTGCTGGAGAAGGAGAAGCTG
AGTGCCATGCAGGCCCACCTGGCTGGGAAAATGGCACTGACCAAGGCTTCATCTGTGGCATCAT
CCGACAAGGGCTCCTGCTGCATCGTAGCTGCTGGCAGCCAAGGCCCTGTCGTCCCAGCCTGGT
 
CTGGCCCCCGGGAGGCCCCTGACAGCCTGTTTGCTGTCCGGAGGCACCTGTGGGGTAGCCATG
GAAACAGCACATTCCCAGAGTTCCTCCACAACATGGACTACTTCAAGTTCCACAACATGCGACCC
CCTTTCACCTACGCCACGCTCATCCGCTGGGCCATCCTGGAGGCTCCAGAGAAGCAGCGGACA
CTCAATGAGATCTACCACTGGTTCACACGCATGTTTGCCTTCTTCAGAAACCATCCTGCCACCTG
GAAGAACGCCATCCGCCACAACCTGAGTCTGCACAAGTGCTTTGTGCGGGTGGAGAGCGAGAA
GGGGGCTGTGTGGACCGTGGATGAGCTGGAGTTCCGCAAGAAACGGAGCCAGAGGCCCAGCA
GGTGTTCCAACCCTACACCTGGCCCCTGA
**SEQ ID NO: 162** - nucleotide sequence encoding an illustrative FOXP3 polypeptide 
GAATTCGTCGACATGCCCAACCCCAGACCCGGCAAGCCTTCTGCCCCTTCTCTGGCCCTGGGAC
CATCTCCTGGCGCCTCCCCATCTTGGAGAGCCGCCCCTAAAGCCAGCGATCTGCTGGGAGCTA
GAGGCCCTGGCGGCACATTCCAGGGCAGAGATCTGAGAGGCGGAGCCCACGCCTCTAGCAGC
AGCCTGAATCCCATGCCCCCTAGCCAGCTGCAGCTGCCTACACTGCCTCTCGTGATGGTGGCCC
CTAGCGGAGCTAGACTGGGCCCTCTGCCTCATCTGCAGGCTCTGCTGCAGGACCGGCCCCACT
TTATGCACCAGCTGAGCACCGTGGACGCCCACGCCAGAACACCTGTGCTGCAGGTGCACCCCC
TGGAAAGCCCTGCCATGATCAGCCTGACCCCTCCAACCACAGCCACCGGCGTGTTCAGCCTGAA
GGCCAGACCTGGACTGCCCCCTGGCATCAATGTGGCCAGCCTGGAATGGGTGTCCCGCGAACC
TGCCCTGCTGTGCACCTTCCCCAATCCTAGCGCCCCCAGAAAGGACAGCACACTGTCTGCCGTG
CCCCAGAGCAGCTATCCCCTGCTGGCTAACGGCGTGTGCAAGTGGCCTGGCTGCGAGAAGGTG
TTCGAGGAACCCGAGGACTTCCTGAAGCACTGCCAGGCCGACCATCTGCTGGACGAGAAAGGC
AGAGCCCAGTGCCTGCTGCAGCGCGAGATGGTGCAGTCCCTGGAACAGCAGCTGGTGCTGGAA
AAAGAAAAGCTGAGCGCCATGCAGGCCCACCTGGCCGGAAAGATGGCCCTGACAAAAGCCAGC
AGCGTGGCCAGCTCCGACAAGGGCAGCTGTTGTATCGTGGCCGCTGGCAGCCAGGGACCTGTG
GTGCCTGCTTGGAGCGGACCTAGAGAGGCCCCCGATAGCCTGTTTGCCGTGCGGAGACACCTG
TGGGGCAGCCACGGCAACTCTACCTTCCCCGAGTTCCTGCACAACATGGACTACTTCAAGTTCC
ACAACATGAGGCCCCCCTTCACCTACGCCACCCTGATCAGATGGGCCATTCTGGAAGCCCCCGA
GAAGCAGCGGACCCTGAACGAGATCTACCACTGGTTTACCCGGATGTTCGCCTTCTTCCGGAAC
CACCCCGCCACCTGGAAGAACGCCATCCGGCACAATCTGAGCCTGCACAAGTGCTTCGTGCGG
GTGGAAAGCGAGAAGGGCGCCGTGTGGACAGTGGACGAGCTGGAATTTCGGAAGAAGCGGTCC
CAGAGGCCCAGCCGGTGTAGCAATCCTACACCTGGCCCTGAGGGCAGAGGAAGTCTGCTAACA
TGCGGTGACGTCGAGGAGAATCC
**SEQ ID NO: 163** - amino acid sequence of the P2A domain
   ATNFSLLKQAGDVEENPGP
**SEQ ID NO: 164** - amino acid sequence of the T2A domain
   EGRGSLLTCGDVEENPGP
**SEQ ID NO: 165** - amino acid sequence of the E2A domain
   QCTNYALLKLAGDVESNPGP
**SEQ ID NO: 166** - amino acid sequence of the F2A domain
   VKQTLNFDLLKLAGDVESNPGP
**SEQ ID NO: 167** - amino acid sequence of a furin cleavage site
   RXXR
**SEQ ID NO: 168** - amino acid sequence of a furin cleavage site
   RRKR
**SEQ ID NO: 169** - nucleotide sequence of the SFFV promoter 
GTAACGCCATTTTGCAAGGCATGGAAAAATACCAAACCAAGAATAGAGAAGTTCAGATCAAGGGC
GGGTACATGAAAATAGCTAACGTTGGGCCAAACAGGATATCTGCGGTGAGCAGTTTCGGCCCCG
GCCCGGGGCCAAGAACAGATGGTCACCGCAGTTTCGGCCCCGGCCCGAGGCCAAGAACAGAT
GGTCCCCAGATATGGCCCAACCCTCAGCAGTTTCTTAAGACCCATCAGATGTTTCCAGGCTCCC
CCAAGGACCTGAAATGACCCTGCGCCTTATTTGAATTAACCAATCAGCCTGCTTCTCGCTTCTGT
TCGCGCGCTTCTGCTTCCCGAGCTCTATAAAAGAGCTCACAACCCCTCACTCGGCGCGCCAGTC
CTCCGACAGACTGAGTCGGCCGG
**SEQ ID NO: 170** - amino acid sequence of human DGCR2 (the transmembrane domain is underlined) 
TEPLRPELRCNPGQFACRSGTIQCIPLPWQCDGWATCEDESDEANCPEVTGEVRPHHGKEAVDPR
QGRARGGDPSHFHAVNVAQPVRFSSFLGKCPTGWHHYEGTASCYRVYLSGENYWDAAQTCQRLN
GSLATFSTDQELRFVLAQEWDQPERSFGWKDQRKLWVGYQYVITGRNRSLEGRWEVAFKGSSEVF
LPPDPIFASAMSENDNVFCAQLQCFHFPTLRHHDLHSWHAESCYEKSSFLCKRSQTCVDIKDNVVDE
GFYFTPKGDDPCLSCTCHGGEPEMCVAALCERPQGCQQYRKDPKECCKFMCLDPDGNSLFDSMAS
GMRLVVSCISSFLILSLLLFMVHRLRQRRRERIESLIGANLHHFNLGRRIPGFDYGPDGFGTGLTPLHL
SDDGEGGTFHFHDPPPPYTAYKYPDIGQPDDPPPPYEASIHPDSVFYDPADDDAFEPVEVSLPAPGD
GGSEGALLRRLEQPLPTAGASLADLEDSADSSSALLVPPDPAQSGSTPAAEALPGGGRHSRSSLNTV
V
**SEQ ID NO: 171** - amino acid sequence of mouse DGCR2 (the transmembrane domain is underlined) 
RPELRCNPGQFACHGGTIQCIPLPWQCDGWPTCEDKSDEADCPVTGEARPYGKETVDLRQGRARG
GDPTHFHTVNVAQPVRFSSFLGKCPSGWHHYEGTASCYRVYLSGENYWDAAQTCQRVNGSLATFS
TDQELRFVLAQEWDQPERSFGWKDQRKLWVGYQYVITGRNHSLEGRWEVAFKGSPEVFLPPDPIFA
SAMSENDNVFCAQLQCFHFPTLRHHDLHSWHAESCSEKSSFLCKRSQTCVDIKDNVVDEGFYFTPK
GDDPCLSCTCHRGEPEMCVAALCERPQGCQQYRKDPKECCKFMCLDPDGSSLFDSMASGMRLVV
SCISSFLILSLLLFMVHRLAQRRRERIESLIGANLHHFNLGRRIPGFDYGPDGFGTGLTPLHLSDDGEG
GTFHFHDPPPPYTAYKYPDMDQPDDPPPPYEASINPDSVFYDPADDDAFEPVEASLPAPRDGGIEGA
LPRHLDQPLPPAETSLADLEDSTDSSSALLVPPDPAQSGSTPATEAPPGGGRLPRASLNTVV
**SEQ ID NO: 172** - exemplary linker sequence
   KLEEGEFSEARV
**SEQ ID NO: 173** - exemplary linker sequence
   ILEEGEFSEAGC
**SEQ ID NO: 174** - exemplary linker sequence
   X₁LEEGEFSEAX₂X₃ wherein X₁ is K or I, X₂ is R or G and X₃ is V or C
**SEQ ID NO: 175** - amino acid sequence of a CH2CH3 hinge domain 
EPKSPDKTHTCPPCPAPPVAGPSVFLFPPKPKDTLMIARTPEVTCVVVDVSHEDPEVKFNWYVDGVE
VHNAKTKPREEQYNSTYRVVSVLTVLHQDWLNGKEYKCKVSNKALPAPIEKTISKAKGQPREPQVYT
LPPSRDELTKNQVSLTCLVKGFYPSDIAVEWESNGQPENNYKTTPPVLDSDGSFFLYSKLTVDKSRW
QQGNVFSCSVMHEALHNHYTQKSLSLSPGK
**SEQ ID NO: 176** - wildtype human CD8alpha hinge domain
   FVPVFLPAKPTTTPAPRPPTPAPTIASQPLSLRPEA**C**RPAAGGAVHTRGLDFA**C**D
**SEQ ID NO: 177** - amino acid sequence of a CD4 transmembrane domain
   MALIVLGGVAGLLLFIGLGIFF
**SEQ ID NO: 178** - amino acid sequence of a CAR having an antigen binding domain derived from G3 
QVQLVQSGGGVVQPGRSLRLSCAASGFTFSSYGMHWVRQAPGKGLEWVAVISYDGSNKYYADSVK
GRFTISRDNSKNTLYLQMNSLRAEDTAVYYCAKDRGAVAGTDWYFDLWGRGTLVTVSSGGGGSGG
GGSGGGGSSSELTQDPAVSVALGQTVRITCQGDSLRSYYASWYQQKPGQAPVLVIYGKNNRPSGIP
DRFSGSSSGNTASLTITGAQAEDEADYYCNSRDSSGNHVVFGGGTKLTVLTRFVPVFLPAKPTTTPA
PRPPTPAPTIASQPLSLRPEACRPAAGGAVHTRGLDFACDIYIWAPLAGTCGVLLLSLVITLYCNHRPP
ALEWVRSKRSRLLHSDYMNMTPRRPGPTRKHYQPYAPPRDFAAYRSASCTRVKFSRSADAPAYQQ
GQNQLYNELNLGRREEYDVLDKRRGRDPEMGGKPRRKNPQEGLYNELQKDKMAEAYSEIGMKGER
RRGKGHDGLYQGLSTATKDTYDALHMQALPPR
**SEQ ID NO: 179** - amino acid sequence of a CAR having an antigen binding domain derived from G9 
QVQLVQSGGGLVQPGGSLRLSCAASGFTFSSYSMSWVRQAPGKGLEWVLGITRGGNTFYADSVTG
RFTISRDISKNTVFLQMDSLRVEDTAIYYCAKDWTMSGINVFDTWGQGTLVTVSSGGGGSGGGGSG
GGGSQAVLTQPPSVSGSPGQSVTISCTGTSSDVGAYNRVSWYQQPPGTAPKLMIYEVNNRPSGVP
DRFSGSKSGNTASLTISGLQAEDEADYYCSSSTTSSTYVFGTGTKVTVLTRFVPVFLPAKPTTTPAPR
PPTPAPTIASQPLSLRPEACRPAAGGAVHTRGLDFACDIYIWAPLAGTCGVLLLSLVITLYCNHRPPAL
EWVRSKRSRLLHSDYMNMTPRRPGPTRKHYQPYAPPRDFAAYRSASCTRVKFSRSADAPAYQQGQ
NQLYNELNLGRREEYDVLDKRRGRDPEMGGKPRRKNPQEGLYNELQKDKMAEAYSEIGMKGERRR
GKGHDGLYQGLSTATKDTYDALHMQALPPR
**SEQ ID NO: 180** - amino acid sequence of a CAR having an antigen binding domain derived from G11 
EVQLQQSGGGLVQPGGSLRLSCAASEFPFSSYAMSWVRQAPGKGLAWVSAISSSGGDTYYADSVK
GRFTISRDNSKNTLYLQMNSLRAEDTAVYYCAKNWNLDYWGQGTLVTVSSGGGGSGGGGSGGGG
SSSELTQDPAVSVALGQTVMITCQGHSLRSYYASWYQQKPGQAPLLVIYGDNRRPSGIPDRFSGSNS
GNTASLTITGTQAEDEADYYCNSRDSSGHHNVLFGGGTKLTVLTRFVPVFLPAKPTTTPAPRPPTPAP
TIASQPLSLRPEACRPAAGGAVHTRGLDFACDIYIWAPLAGTCGVLLLSLVITLYCNHRPPALEWVRSK
RSRLLHSDYMNMTPRRPGPTRKHYQPYAPPRDFAAYRSASCTRVKFSRSADAPAYQQGQNQLYNE
LNLGRREEYDVLDKRRGRDPEMGGKPRRKNPQEGLYNELQKDKMAEAYSEIGMKGERRRGKGHD
GLYQGLSTATKDTYDALHMQALPPR
**SEQ ID NO: 181** - amino acid sequence of a CAR having an antigen binding domain derived from A5 
EVQLVESGGGLVQPGGSLRLSCAASGFTVSSNYMSWVRQAPGKGLEWVSVIYSGGSTYYADSVKG
RFTISRDNAKNSLYLQMNSLRAEDTAVYYCARGWTTFDYWGQGTLVTVSSGGGGSGGGGSGGGG
SQAVLTQPPSLSVSPRQTVTITCSGDQLGDRYVSWYQQKPGQSPMLVIYQDDRRPSGVPERFSGSN
SGNTATLTIGGTQAMDQADYYCQTWDNSPVVFGGGTKLTVLTRFVPVFLPAKPTTTPAPRPPTPAPTI
ASQPLSLRPEACRPAAGGAVHTRGLDFACDIYIWAPLAGTCGVLLLSLVITLYCNHRPPALEWVRSKR
SRLLHSDYMNMTPRRPGPTRKHYQPYAPPRDFAAYRSASCTRVKFSRSADAPAYQQGQNQLYNEL
NLGRREEYDVLDKRRGRDPEMGGKPRRKNPQEGLYNELQKDKMAEAYSEIGMKGERRRGKGHDG
LYQGLSTATKDTYDALHMQALPPR
**SEQ ID NO: 182** - amino acid sequence of a CAR having an antigen binding domain derived from F5 
EVQLVQSGGGVVQPGRSLRLSCAASGFTFSSYGMHWVRQAPGKGLEWVAVISYDGSNKYYADSVK
GRFTISRDNSKNTLYLQMNSLRAEDTAVYYCAKDLTRSGYPSYYFDYWGQGTLVTVSSGGGGSGG
GGSGGGGSSSELTQDPAVSVALGQTVRITCQGDSLRSYYASWYQQKPGQAPVLVIYGKNNRPSGIP
DRFSGSSSGNTASLTITGAQAEDEADYYCNSRGSSGNNWVFGGGTKLTVLTRFVPVFLPAKPTTTPA
PRPPTPAPTIASQPLSLRPEACRPAAGGAVHTRGLDFACDIYIWAPLAGTCGVLLLSLVITLYCNHRPP
ALEWVRSKRSRLLHSDYMNMTPRRPGPTRKHYQPYAPPRDFAAYRSASCTRVKFSRSADAPAYQQ
GQNQLYNELNLGRREEYDVLDKRRGRDPEMGGKPRRKNPQEGLYNELQKDKMAEAYSEIGMKGER
RRGKGHDGLYQGLSTATKDTYDALHMQALPPR
**SEQ ID NO: 183** - amino acid sequence of a CAR having an antigen binding domain derived from D3 
QVQLVESGGGLVQPGGSLRLSCAASGFTFSSNAMSWVRQAPGKGLEWVSAIGTGGTTYYADSVKG
RFTISRDNSKNTLYLQMNSLRAEDTAVYYCAKTGQLDYWGQGTLVTVSSGGGGSGGGGSGGGGSQ
SALTQPASVSGSPGQSITISCTGTSSDVGGYNYVSWFQQHPDKAPKLMIYDVSNRPSGVSNRFSGS
KSGTSASLAISGLRSEDEADYYCAAWDDSLSGWVFGGGTKLTVLTRFVPVFLPAKPTTTPAPRPPTP
APTIASQPLSLRPEACRPAAGGAVHTRGLDFACDIYIWAPLAGTCGVLLLSLVITLYCNHRPPALEWVR
SKRSRLLHSDYMNMTPRRPGPTRKHYQPYAPPRDFAAYRSASCTRVKFSRSADAPAYQQGQNQLY
NELNLGRREEYDVLDKRRGRDPEMGGKPRRKNPQEGLYNELQKDKMAEAYSEIGMKGERRRGKG
HDGLYQGLSTATKDTYDALHMQALPPR
**SEQ** ID NO: 184 - amino acid sequence of a CAR having an antigen binding domain derived from D4 
QVQLVQSGGGLVQPGGSLRLSCAASGFTFSNYAMSWVRQAPGKGLEWVSGISGGGDNTYYTDSVK
GRFTISRDNSKNTLYLQMNSLTAEDTALYYCAKKQSGGSGWSLWGFDYWGQGTLVTVSSGGGGSG
GGGSGGGGSQAVVTQEPSLTVSPGGTVTLTCASSTGAVTSGYYPSWFQQKPGQAPRPLIYNTNNK
HSWSPARFSGSLLGGKAALTLSGVQPEDEADYYCLLYYGGRGVFGGGTRLTVLTRFVPVFLPAKPTT
TPAPRPPTPAPTIASQPLSLRPEACRPAAGGAVHTRGLDFACDIYIWAPLAGTCGVLLLSLVITLYCNH
RPPALEWVRSKRSRLLHSDYMNMTPRRPGPTRKHYQPYAPPRDFAAYRSASCTRVKFSRSADAPA
YQQGQNQLYNELNLGRREEYDVLDKRRGRDPEMGGKPRRKNPQEGLYNELQKDKMAEAYSEIGMK
GERRRGKGHDGLYQGLSTATKDTYDALHMQALPPR
**SEQ ID NO: 185** - amino acid sequence of a CAR having an antigen binding domain derived from F7 
EVQLLESGGGLVQPGGSLRLSCAASGFTFSSYAMSWVRQAPGKGLEWVSAISGSGGSTYYADSVK
GRFTISRDNSKNTLYLQMNSLRAEDTAVYYCAKNPIQLWSKIKNWFDPWGQGTLVTVSSGGGGSGG
GGSGGGGSQAVVTQEPSLSVSPGGTVSLTCGSSAGDVTYGHFPYWYQQKPGQAPRMLIYDVNNRA
SWTPARFSGSLVGGKAALTLSGAQPGDEADYYCALSYFGSMFFGGGTKLTVLTRFVPVFLPAKPTTT
PAPRPPTPAPTIASQPLSLRPEACRPAAGGAVHTRGLDFACDIYIWAPLAGTCGVLLLSLVITLYCNHR
PPALEWVRSKRSRLLHSDYMNMTPRRPGPTRKHYQPYAPPRDFAAYRSASCTRVKFSRSADAPAY
QQGQNQLYNELNLGRREEYDVLDKRRGRDPEMGGKPRRKNPQEGLYNELQKDKMAEAYSEIGMK
GERRRGKGHDGLYQGLSTATKDTYDALHMQALPPR
**SEQ ID NO: 186** - amino acid sequence of a CAR having an antigen binding domain derived from D2 
EVQLVQSGGGVVQPGRSLRLSCAASGFTFSSYGMHWVRQAPGKGLEWVAVISYDGSNKYYADSVK
GRFTISRDNSKNTLYLQMNSLRAEDTAVYYCAKDLTRSGYPSYYFDYWGQGTLVTVSSGGGGSGG
GGSGGGGSSSELTQDPAVSVALGRTVRITCQGDSLRSYYASWYQQKPGQAPVLVIYGKNNRPSGIP
DRFSGSSSGNTASLTITGAQAEDEADYYCNSRDSSGNLVFGGGTKLTVLTRFVPVFLPAKPTTTPAP
RPPTPAPTIASQPLSLRPEACRPAAGGAVHTRGLDFACDIYIWAPLAGTCGVLLLSLVITLYCNHRPPA
LEWVRSKRSRLLHSDYMNMTPRRPGPTRKHYQPYAPPRDFAAYRSASCTRVKFSRSADAPAYQQG
QNQLYNELNLGRREEYDVLDKRRGRDPEMGGKPRRKNPQEGLYNELQKDKMAEAYSEIGMKGERR
RGKGHDGLYQGLSTATKDTYDALHMQALPPR
**SEQ ID NO: 187** - amino acid sequence of a CAR having an antigen binding domain derived from G3 
QVQLVQSGGGVVQPGRSLRLSCAASGFTFSSYGMHWVRQAPGKGLEWVAVISYDGSNKYYADSVK
GRFTISRDNSKNTLYLQMNSLRAEDTAVYYCAKDRGAVAGTDWYFDLWGRGTLVTVSSGGGGSGG
GGSGGGGSSSELTQDPAVSVALGQTVRITCQGDSLRSYYASWYQQKPGQAPVLVIYGKNNRPSGIP
DRFSGSSSGNTASLTITGAQAEDEADYYCNSRDSSGNHVVFGGGTKLTVLTREPKSPDKTHTCPPC
PAPPVAGPSVFLFPPKPKDTLMIARTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSRDELTKNQVSL
TCLVKGFYPSDIAVEWESNGQPENNYKTTPPVLDSDGSFFLYSKLTVDKSRWQQGNVFSCSVMHEA
LHNHYTQKSLSLSPGKKDPKFWVLVVVGGVLACYSLLVTVAFIIFWVRSKRSRLLHSDYMNMTPRRP
 
GPTRKHYQPYAPPRDFAAYRSASCTRVKFSRSADAPAYQQGQNQLYNELNLGRREEYDVLDKRRG
RDPEMGGKPRRKNPQEGLYNELQKDKMAEAYSEIGMKGERRRGKGHDGLYQGLSTATKDTYDALH
MQALPPR
**SEQ ID NO: 188** - amino acid sequence of a CAR having an antigen binding domain derived from G9 
QVQLVQSGGGLVQPGGSLRLSCAASGFTFSSYSMSWVRQAPGKGLEWVLGITRGGNTFYADSVTG
RFTISRDISKNTVFLQMDSLRVEDTAIYYCAKDWTMSGINVFDTWGQGTLVTVSSGGGGSGGGGSG
GGGSQAVLTQPPSVSGSPGQSVTISCTGTSSDVGAYNRVSWYQQPPGTAPKLMIYEVNNRPSGVP
DRFSGSKSGNTASLTISGLQAEDEADYYCSSSTTSSTYVFGTGTKVTVLTREPKSPDKTHTCPPCPA
PPVAGPSVFLFPPKPKDTLMIARTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPPSRDELTKNQVSLTC
LVKGFYPSDIAVEWESNGQPENNYKTTPPVLDSDGSFFLYSKLTVDKSRWQQGNVFSCSVMHEALH
NHYTQKSLSLSPGKKDPKFWVLVVVGGVLACYSLLVTVAFIIFWVRSKRSRLLHSDYMNMTPRRPGP
TRKHYQPYAPPRDFAAYRSASCTRVKFSRSADAPAYQQGQNQLYNELNLGRREEYDVLDKRRGRD
PEMGGKPRRKNPQEGLYNELQKDKMAEAYSEIGMKGERRRGKGHDGLYQGLSTATKDTYDALHMQ
ALPPR
**SEQ ID NO: 189** - amino acid sequence of a CAR having an antigen binding domain derived from G11 
EVQLQQSGGGLVQPGGSLRLSCAASEFPFSSYAMSWVRQAPGKGLAWVSAISSSGGDTYYADSVK
GRFTISRDNSKNTLYLQMNSLRAEDTAVYYCAKNWNLDYWGQGTLVTVSSGGGGSGGGGSGGGG
SSSELTQDPAVSVALGQTVMITCQGHSLRSYYASWYQQKPGQAPLLVIYGDNRRPSGIPDRFSGSNS
GNTASLTITGTQAEDEADYYCNSRDSSGHHNVLFGGGTKLTVLTREPKSPDKTHTCPPCPAPPVAGP
SVFLFPPKPKDTLMIARTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSVMHEALHNHYTQK
SLSLSPGKKDPKFWVLVVVGGVLACYSLLVTVAFIIFWVRSKRSRLLHSDYMNMTPRRPGPTRKHYQ
PYAPPRDFAAYRSASCTRVKFSRSADAPAYQQGQNQLYNELNLGRREEYDVLDKRRGRDPEMGGK
PRRKNPQEGLYNELQKDKMAEAYSEIGMKGERRRGKGHDGLYQGLSTATKDTYDALHMQALPPR
**SEQ ID NO: 190** - amino acid sequence of a CAR having an antigen binding domain derived from A5 
EVQLVESGGGLVQPGGSLRLSCAASGFTVSSNYMSWVRQAPGKGLEWVSVIYSGGSTYYADSVKG
RFTISRDNAKNSLYLQMNSLRAEDTAVYYCARGWTTFDYWGQGTLVTVSSGGGGSGGGGSGGGG
SQAVLTQPPSLSVSPRQTVTITCSGDQLGDRYVSWYQQKPGQSPMLVIYQDDRRPSGVPERFSGSN
SGNTATLTIGGTQAMDQADYYCQTWDNSPVVFGGGTKLTVLTREPKSPDKTHTCPPCPAPPVAGPS
VFLFPPKPKDTLMIARTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVMHEALHNHYTQKS
LSLSPGKKDPKFWVLVVVGGVLACYSLLVTVAFIIFWVRSKRSRLLHSDYMNMTPRRPGPTRKHYQP
YAPPRDFAAYRSASCTRVKFSRSADAPAYQQGQNQLYNELNLGRREEYDVLDKRRGRDPEMGGKP
RRKNPQEGLYNELQKDKMAEAYSEIGMKGERRRGKGHDGLYQGLSTATKDTYDALHMQALPPR
**SEQ ID NO: 191** - amino acid sequence of a CAR having an antigen binding domain derived from F5 
EVQLVQSGGGVVQPGRSLRLSCAASGFTFSSYGMHWVRQAPGKGLEWVAVISYDGSNKYYADSVK
GRFTISRDNSKNTLYLQMNSLRAEDTAVYYCAKDLTRSGYPSYYFDYWGQGTLVTVSSGGGGSGG
GGSGGGGSSSELTQDPAVSVALGQTVRITCQGDSLRSYYASWYQQKPGQAPVLVIYGKNNRPSGIP
DRFSGSSSGNTASLTITGAQAEDEADYYCNSRGSSGNNWVFGGGTKLTVLTREPKSPDKTHTCPPC
PAPPVAGPSVFLFPPKPKDTLMIARTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSRDELTKNQVSL
TCLVKGFYPSDIAVEWESNGQPENNYKTTPPVLDSDGSFFLYSKLTVDKSRWQQGNVFSCSVMHEA
LHNHYTQKSLSLSPGKKDPKFWVLVVVGGVLACYSLLVTVAFIIFWVRSKRSRLLHSDYMNMTPRRP
GPTRKHYQPYAPPRDFAAYRSASCTRVKFSRSADAPAYQQGQNQLYNELNLGRREEYDVLDKRRG
RDPEMGGKPRRKNPQEGLYNELQKDKMAEAYSEIGMKGERRRGKGHDGLYQGLSTATKDTYDALH
MQALPPR
**SEQ ID NO: 192** - amino acid sequence of a CAR having an antigen binding domain derived from D3 
QVQLVESGGGLVQPGGSLRLSCAASGFTFSSNAMSWVRQAPGKGLEWVSAIGTGGTTYYADSVKG
RFTISRDNSKNTLYLQMNSLRAEDTAVYYCAKTGQLDYWGQGTLVTVSSGGGGSGGGGSGGGGSQ
SALTQPASVSGSPGQSITISCTGTSSDVGGYNYVSWFQQHPDKAPKLMIYDVSNRPSGVSNRFSGS
KSGTSASLAISGLRSEDEADYYCAAWDDSLSGWVFGGGTKLTVLTREPKSPDKTHTCPPCPAPPVA
GPSVFLFPPKPKDTLMIARTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSRDELTKNQVSLTCLVKG
FYPSDIAVEWESNGQPENNYKTTPPVLDSDGSFFLYSKLTVDKSRWQQGNVFSCSVMHEALHNHYT
QKSLSLSPGKKDPKFWVLVVVGGVLACYSLLVTVAFIIFWVRSKRSRLLHSDYMNMTPRRPGPTRKH
YQPYAPPRDFAAYRSASCTRVKFSRSADAPAYQQGQNQLYNELNLGRREEYDVLDKRRGRDPEMG
GKPRRKNPQEGLYNELQKDKMAEAYSEIGMKGERRRGKGHDGLYQGLSTATKDTYDALHMQALPP
R
**SEQ** ID NO: 193 - amino acid sequence of a CAR having an antigen binding domain derived from D4 
QVQLVQSGGGLVQPGGSLRLSCAASGFTFSNYAMSWVRQAPGKGLEWVSGISGGGDNTYYTDSVK
GRFTISRDNSKNTLYLQMNSLTAEDTALYYCAKKQSGGSGWSLWGFDYWGQGTLVTVSSGGGGSG
GGGSGGGGSQAVVTQEPSLTVSPGGTVTLTCASSTGAVTSGYYPSWFQQKPGQAPRPLIYNTNNK
HSWSPARFSGSLLGGKAALTLSGVQPEDEADYYCLLYYGGRGVFGGGTRLTVLTREPKSPDKTHTC
PPCPAPPVAGPSVFLFPPKPKDTLMIARTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPPSRDELTKN
QVSLTCLVKGFYPSDIAVEWESNGQPENNYKTTPPVLDSDGSFFLYSKLTVDKSRWQQGNVFSCSV
MHEALHNHYTQKSLSLSPGKKDPKFWVLVVVGGVLACYSLLVTVAFIIFWVRSKRSRLLHSDYMNMT
PRRPGPTRKHYQPYAPPRDFAAYRSASCTRVKFSRSADAPAYQQGQNQLYNELNLGRREEYDVLD
KRRGRDPEMGGKPRRKNPQEGLYNELQKDKMAEAYSEIGMKGERRRGKGHDGLYQGLSTATKDTY
DALHMQALPPR
**SEQ ID NO: 194** - amino acid sequence of a CAR having an antigen binding domain derived from F7 
EVQLLESGGGLVQPGGSLRLSCAASGFTFSSYAMSWVRQAPGKGLEWVSAISGSGGSTYYADSVK
GRFTISRDNSKNTLYLQMNSLRAEDTAVYYCAKNPIQLWSKIKNWFDPWGQGTLVTVSSGGGGSGG
GGSGGGGSQAVVTQEPSLSVSPGGTVSLTCGSSAGDVTYGHFPYWYQQKPGQAPRMLIYDVNNRA
SWTPARFSGSLVGGKAALTLSGAQPGDEADYYCALSYFGSMFFGGGTKLTVLTREPKSPDKTHTCP
PCPAPPVAGPSVFLFPPKPKDTLMIARTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSRDELTKNQ
VSLTCLVKGFYPSDIAVEWESNGQPENNYKTTPPVLDSDGSFFLYSKLTVDKSRWQQGNVFSCSVM
HEALHNHYTQKSLSLSPGKKDPKFWVLVVVGGVLACYSLLVTVAFIIFWVRSKRSRLLHSDYMNMTP
RRPGPTRKHYQPYAPPRDFAAYRSASCTRVKFSRSADAPAYQQGQNQLYNELNLGRREEYDVLDK
RRGRDPEMGGKPRRKNPQEGLYNELQKDKMAEAYSEIGMKGERRRGKGHDGLYQGLSTATKDTY
DALHMQALPPR
**SEQ ID NO: 195** - amino acid sequence of a CAR having an antigen binding domain derived from D2 
EVQLVQSGGGVVQPGRSLRLSCAASGFTFSSYGMHWVRQAPGKGLEWVAVISYDGSNKYYADSVK
GRFTISRDNSKNTLYLQMNSLRAEDTAVYYCAKDLTRSGYPSYYFDYWGQGTLVTVSSGGGGSGG
GGSGGGGSSSELTQDPAVSVALGRTVRITCQGDSLRSYYASWYQQKPGQAPVLVIYGKNNRPSGIP
DRFSGSSSGNTASLTITGAQAEDEADYYCNSRDSSGNLVFGGGTKLTVLTREPKSPDKTHTCPPCPA
PPVAGPSVFLFPPKPKDTLMIARTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPPSRDELTKNQVSLTC
LVKGFYPSDIAVEWESNGQPENNYKTTPPVLDSDGSFFLYSKLTVDKSRWQQGNVFSCSVMHEALH
NHYTQKSLSLSPGKKDPKFWVLVVVGGVLACYSLLVTVAFIIFWVRSKRSRLLHSDYMNMTPRRPGP
TRKHYQPYAPPRDFAAYRSASCTRVKFSRSADAPAYQQGQNQLYNELNLGRREEYDVLDKRRGRD
PEMGGKPRRKNPQEGLYNELQKDKMAEAYSEIGMKGERRRGKGHDGLYQGLSTATKDTYDALHMQ
ALPPR
**SEQ ID NO: 196** is the amino acid sequence of the CD8α hinge used in the CARs used in the examples
   FSSVVPVLQKVNSTTTKPVLRTPSPVHPTGTSQPQRPEDCRPRGSVKGTGLDFACDIY
**SEQ ID NO: 197** is the nucleotide sequence encoding the CD8α hinge used in the CARs used in the examples 
TTCAGCTCTGTGGTGCCCGTGCTGCAGAAAGTGAACAGCACCACCACAAAGCCTGTGCTGAGAA
CCCCTTCTCCTGTGCACCCTACCGGCACAAGCCAGCCTCAAAGACCTGAGGACTGCAGACCTAG
AGGCTCCGTGAAAGGCACAGGCCTGGACTTCGCCTGCGACATCTAT
**SEQ ID NO: 198** is a consensus sequence for VH CDR1
   GFTFSSYX¹, wherein X¹ is G, S or A
**SEQ ID NO: 199** is a consensus sequence for VH CDR1
   GFTFSX¹YA, wherein X¹ is N or S
**SEQ ID NO: 200** is a consensus sequence for VH CDR1
   GFTFSSX¹A, wherein X¹ is N or Y
**SEQ ID NO: 201** is a consensus sequence for VL CDR1
   SSDVGX¹YNX², wherein X¹ is A or G and X² is R or Y
**SEQ ID NO: 202** is a consensus sequence for VL CDR2
   GX¹N, wherein X¹ is K or D
**SEQ ID NO: 203** is a consensus sequence for VL CDR2
   DVX¹, wherein X¹ is S or N
**SEQ ID NO: 204** is a consensus sequence for VL CDR2
   X¹VN, wherein X¹ is E or D
**SEQ ID NO: 205** is a consensus sequence for VL CDR3
   NSRDSSGNX¹V, wherein X¹ is H or L
**SEQ ID NO: 206** is a consensus sequence for VL CDR3
   NSRDSSGX¹HX²V, wherein X¹ is N or H and X² is N or V

### EXAMPLES

### Example 1 - Generation of DGCR2 scFv binders and DGCR2-specific CARs

### Materials & Methods

### Generation of scFv binders

scFv binders were generated by phage display using human naive phage display library HAL9/10. A cell based panning approach was performed by panning against murine or human DGCR2 recombinantly expressed on HEK293T cells. Therefore, these cells were transiently transfected with a vector expressing the recombinant protein and eGFP downstream separated by a P2A site. This allowed for recognition of the membrane protein in its natural conformation. Phage display library or binders of consecutive panning round were incubated first with untransfected HEK293T cells to allow for depletion of unspecific binders. Afterwards supernatant was incubated with antigen transfected cells, washed and sorted for GFP high cells. scFvs binding GFP high cells (and therefore cells expressing high amount of target antigen) were eluted form those cells by trypsin digestion. Eluted scFv binders were amplified and used for next panning round. Panning was conducted for 3 consecutive rounds.

After the last round individual scFv binders were initially screened. Classic protein panning was also performed.

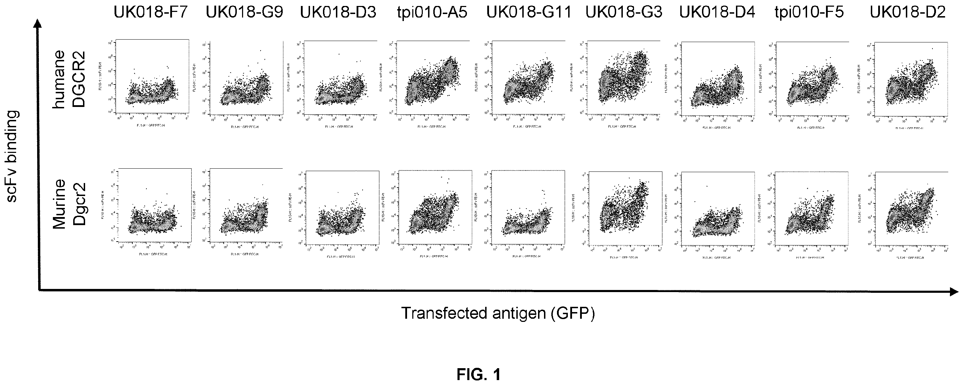

### Screening of scFV binders (Figure 1)

scFv binders initially obtained by phage display cell panning were screened first for recognition of DGCR2 expressed on cells. Therefore, soluble scFv were produced in *E.coli* and used to stain HEK293T cells transiently transfected with a vector expressing the recombinant protein and eGFP downstream separated by a P2A site. Secondary staining was performed by PE staining the His-Tag of soluble scFv. Cells were measured in flow cytometry for GFP (antigen expression) and PE (scFv binding).

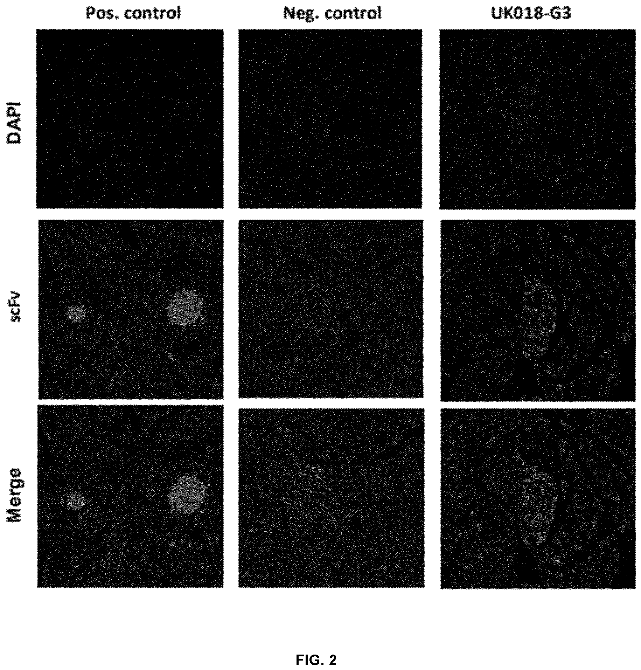

### Pancreas section staining (Figure 2)

Cryo IHC was performed to proof islet recognition of selected scFvs in pancreatic tissues. Therefore, fresh pancreas of C57BI/6 mice were embedded in Tissue-Tek^{®} Cryomold^{®} and quick frozen in liquid nitrogen. 5 µm sections were prepared in a cryostat-microtome. Autofluorescence of sections was quenched by incubation in Glycin buffer. Blocking was performed by incubation in 2% BSA. Soluble scFvs (see above) were used to stain islets. Consecutively, sections were stained with rabbit α-Myc-Tag antibody, recognising the Myc Tag of soluble scFvs. Tertiary staining was performed by incubation with FITC-labelled Goat α-rabbit antibody. Sections were embedded with Fluoromount-G^{™} Mounting Medium containing DAPI and analysed by fluorescence microscopy for islets staining.

### Cloning of CAR vectors

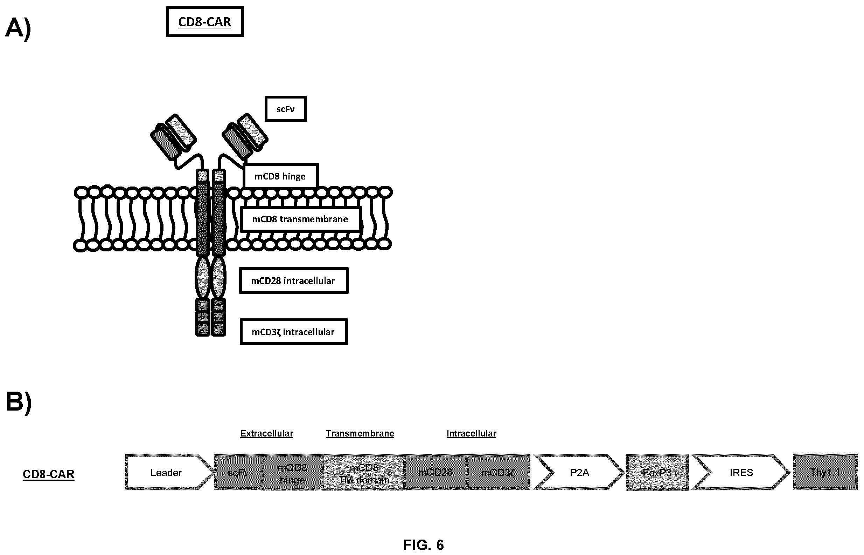

Cloning of scFvs into murine retroviral vectors, respectively, was performed to generate CARs with DGCR2 specificity. The configuration of the CAR constructs used is shown in Figure 6. The CARs had a murine CD8α hinge and transmembrane domain (short hinge). The sequence of the CD8α hinge is shown in SEQ ID NO: 196 and may be encoded by SEQ ID NO: 197. Retroviral vectors containing LTR flanked 2nd generation CAR scaffolds were used for cloning. Cloning was performed by digestion of vectors with Ncol/Notl restriction enzymes and subsequent scFv ligation into the CAR backbone. Murine CAR backbones contained an additional FoxP3 expression cassette allowing converted Tregs (cTreg) generation out of CD4+ T cells but lacked such a cassette for generation of CAR Teffs or natural Tregs (nTregs). Vectors also contained Thy1.1. (CD90.1) as a CAR expression marker.

### Production of recombinant CAR expressing retroviral virus and transduction of T cells and cell lines

Retroviral particles allowing for CAR transduction of T cells were produced in HEK293T cells. They were K73 ecotropic-pseudotyped for murine constructs and VSV-G pantropic-pseudotyped for human constructs, respectively. For CAR transduction CD4+ cells were enriched from murine spleenocytes by magnetic beads separation and activated with CD3/CD28 beads for 2 days, followed by spin-transduction with virus particles and protaminsulfat and underwent continued cultivation for 1 - 4 days. For CAR activation assay NFAT-GFP reporter murine T cell hybridoma cells were transduced in an analogous manner.

### Hybridoma NFAT activation assay

Murine T cell hybridoma cell line reporting NFAT activation by GFP expression were transduced with CAR vector as described above. In parallel HEK293T cells were transfected to express the target antigen DGCR2 either as its murine or human homolog. After 48 h both cell lines were cultivated for 24 h to allow for CAR activation. Direct coated (human) DGCR2 protein and untreated wells served as controls. Cells were stained for marker Erb2 to allow discrimination of HEK293T cells and anti-Fab antibody binding the CAR domains for CAR expression. Level of CAR activation reported by GFP and CAR expression was measured by flow cytometry.

### Results

A number of scFvs that bind to DGCR2 were identified.

Figure 1 shows FACS staining of illustrative scFv binders on antigen transfected HEK293T cells. Binding of the scFvs is shown by anti-His PE antibody staining. Antigen expression is reported by GFP.

Human antigen is shown on the first row. Murine antigen is shown on the second row.

Figure 1 shows that the scFvs labelled "UK018-D2", "UK018-G3", "UK018-G9", "UK018-G11", "tpi010-A5", "tpi010-F5", "UK018-D3", "UK018-D4" and "UK018-F7" (referred to herein as D2, G3, G9, G11, A5, F5, D3, D4 and F7 respectively) bind to HEK293T cells expressing both human and murine DGCR2.

Immunohistochemistry staining on cryo C57BL/6 mouse pancreas sections is shown in Figure 2. The top row shows DAPI staining, the second row shows staining using G3 scFv and the bottom row merges the DAPI and G3 staining. Figure 2 shows that the G3 scFv specifically binds to mouse pancreas sections.

The A5, F5, G3 and D4 scFvs were incorporated into CARs as described above and expressed in murine hybridoma cells. NFAT was activated as a result of CAR binding to DGCR2 and is reported by GFP. Anti-Fab antibody was used for CAR staining of the A5, F5, G3 and D4 scFv CARs.

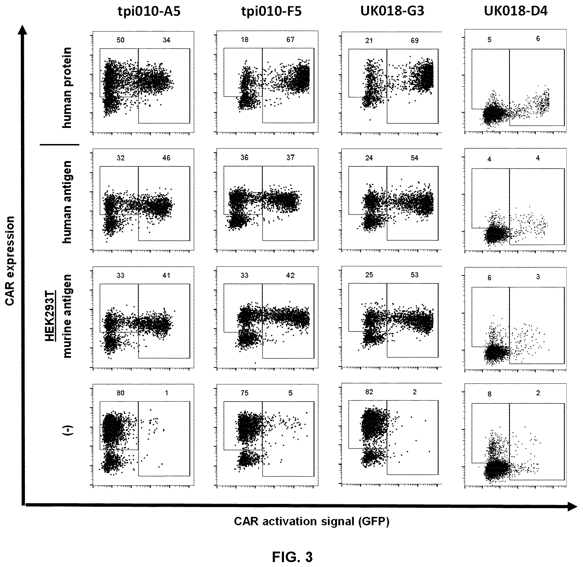

Activation by human DGCR2 protein (top row), HEK cells expressing human DGCR2 antigen (second row), HEK cells expressing murine DGCR2 antigen (third row) and untransfected HEK cells (control) is shown in Figure 3.

### Example 2 - Enrichment of T-effector cells expressing DGCR2-specific CAR in the pancreatic tissue of NOD mice.

### Materials & Methods

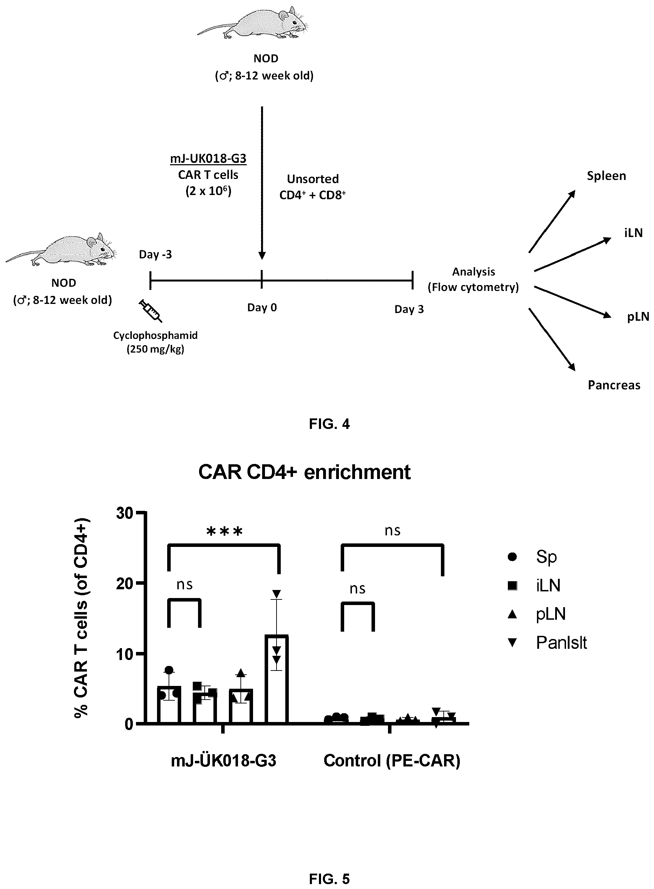

CD4+ cells of NOD mice were transduced with a CAR incorporating the G3 scFv binder (with the short hinge) or unrelated control CARs specific to phycoerythrin (PE). The transduced cells (2 × 10⁶ cells) were adoptively transferred to NOD mice treated with cyclophosphamide 72 h in advance and observed for enrichment within pancreatic tissue on day 3. An overview of the experimental protocol is shown in Figure 4. Statistical significance was determined by two-way ANOVA with follow-up for multiple comparison test (Benjamini, Krieger, Yekutieli). **p* < *0.033* ***p* < *0.002 ***p* < *0.001.*

### Results

CD4+ T cells expressing G3 CAR were enriched within pancreatic islets (Panlslt) in cyclophosphamide treated NOD mice after 3 days compared to lymphatic organs spleen (Sp), inguinal (iLN) and pancreatic lymph nodes (pLN). The results are shown in Figure 5. Significance is shown for comparison with the spleen. No enrichment was observed for cells expressing the control CAR. An n = 3 was used for each group.

## Claims

1. A chimeric antigen receptor (CAR) comprising an antigen recognition domain that specifically binds to DGCR2.

2. The CAR according to claim 1, comprising:
a. an exodomain comprising the antigen recognition domain;
b. a transmembrane domain; and
c. an endodomain comprising an intracellular signalling domain.

3. The CAR according to claim 2, further comprising a hinge domain and/or one or more co-stimulatory domains.

4. The CAR according to claim 3, wherein the hinge domain is selected from the hinge regions of CD28, CD8α, CD4, CD7, CH2CH3, an immunoglobulin, or a part or variant thereof, preferably wherein the CAR comprises a CD8α or CH2CH3 hinge region; and/or wherein the co-stimulatory domain is selected from the intracellular domains of CD28, ICOS, CD134 (OX40), CD137 (4-1BB), CD27, or TNFRSF25, or a part or variant thereof, preferably wherein the CAR comprises a CD28 co-stimulatory domain.

5. The CAR according to any of claims 2 to 4, wherein the CAR comprises one or more transmembrane domains selected from the transmembrane domains of CD28, ICOS, CD8α, CD4, CD134 (OX40), CD137 (4-1BB), CD3 zeta, CD45, CD9, CD16, CD22, CD33, CD64, CD80, CD86, CD154 or a part or variant thereof, preferably wherein the CAR comprises a CD4, CD28, or CD8α transmembrane domain and/or wherein the CAR comprises one or more intracellular signalling domains selected from the group consisting of the CD3 zeta signalling domain or any of its homologs, a CD3 polypeptide, a syk family tyrosine kinase, a src family tyrosine kinase, CD2, CD5, CD28, or a part or variant thereof, preferably wherein the CAR comprises the CD3 zeta signalling domain.

6. The CAR according to any of claims 2 to 5, wherein the CAR comprises: a CD8α or CH2CH3 hinge domain, a CD28 or CD8α transmembrane domain, a CD28 co-stimulatory domain, and the CD3zeta signalling domain, wherein when the hinge domain is CD8α, the transmembrane domain is CD8α, and when the hinge domain is CH2CH3, the transmembrane domain is CD28.

7. The CAR according to any preceding claim, wherein the antigen recognition domain is an antibody, an antibody fragment, or derived from an antibody, preferably wherein the antigen recognition domain is a single chain antibody (scFv).

8. The CAR according to any preceding claim, wherein the antigen recognition domain comprises:
(i) VH CDR1, 2 and 3 sequences set forth in SEQ ID NOs: 1, 2 and 3 respectively and VL CDR1, 2 and 3 sequences set forth in SEQ ID NOs: 4, 5 and 6 respectively;
(ii) VH CDR1, 2 and 3 sequences set forth in SEQ ID NOs: 7, 8 and 9 respectively and VL CDR1, 2 and 3 sequences set forth in SEQ ID NOs: 10, 11 and 12 respectively;
(iii) VH CDR1, 2 and 3 sequences set forth in SEQ ID NOs: 13, 14 and 15 respectively and VL CDR1, 2 and 3 sequences set forth in SEQ ID NOs: 16, 17 and 18 respectively;
(iv) VH CDR1, 2 and 3 sequences set forth in SEQ ID NOs: 19. 20 and 21 respectively and VL CDR1, 2 and 3 sequences set forth in SEQ ID NOs: 22, 23 and 24 respectively;
(v) VH CDR1, 2 and 3 sequences set forth in SEQ ID NOs: 25, 26 and 27 respectively and VL CDR1, 2 and 3 sequences set forth in SEQ ID NOs: 28, 29 and 30 respectively;
(vi) VH CDR1, 2 and 3 sequences set forth in SEQ ID NOs: 31, 32 and 33 respectively and VL CDR1, 2 and 3 sequences set forth in SEQ ID NOs: 34, 35 and 36 respectively;
(vii) VH CDR1, 2 and 3 sequences set forth in SEQ ID NOs: 37, 38 and 39 respectively and VL CDR1, 2 and 3 sequences set forth in SEQ ID NOs: 40, 41 and 42 respectively;
(viii) VH CDR1, 2 and 3 sequences set forth in SEQ ID NOs: 43, 44 and 45 respectively and VL CDR1, 2 and 3 sequences set forth in SEQ ID NOs: 46, 47 and 48 respectively; or
(ix) VH CDR1, 2 and 3 sequences set forth in SEQ ID NOs: 49, 50 and 51 respectively and VL CDR1, 2 and 3 sequences set forth in SEQ ID NOs: 52, 53 and 54 respectively;
wherein one or more of said CDR sequences of (i) to (ix) may optionally comprise 1 to 3 amino acid modifications relative to an aforementioned CDR sequence, particularly wherein one or more of said CDR sequences may optionally be modified by substitution, addition or deletion of 1 to 3 amino acids.

9. A nucleic acid molecule comprising a nucleotide sequence encoding the CAR of any preceding claim.

10. A vector comprising the nucleic acid molecule of claim 9, optionally further comprising a nucleic acid molecule encoding a FOXP3 polypeptide.

11. A cell comprising the CAR of any of claims 1 to 8, the nucleic acid molecule of claim 9, or the vector of claim 10.

12. The cell of claim 11, wherein the cell is a regulatory T cell (Treg), or a precursor thereof, or an iPSC cell, particularly wherein the cell further comprises an exogenous nucleic acid comprising a nucleotide sequence encoding a FOXP3 polypeptide.

13. A cell population comprising a cell of claim 11 or 12.

14. A pharmaceutical composition comprising a cell of claim 11 or claim 12, a cell population of claim 13, or a vector of claim 10.

15. The cell of claim 11 or claim 12, a cell population of claim 13, or the pharmaceutical composition of claim 14, for use in therapy.

16. The cell, cell population or pharmaceutical composition for use of claim 15, wherein the therapy is adoptive cell transfer therapy.

17. The cell of claim 11 or claim 12, a cell population of claim 13, or pharmaceutical composition of claim 14, for use in treating or preventing an autoimmune or inflammatory disease, or for use in inducing immunosuppression, or for use in promoting tissue repair and/or tissue regeneration, particularly wherein the cell is a Treg.

18. The cell, cell population or pharmaceutical composition for use of claim 17, wherein the autoimmune or inflammatory disease is type 1 diabetes, for example recent-onset type 1 diabetes.

19. A method of making a cell of claim 11 or claim 12, which comprises the step of introducing into the cell the nucleic acid molecule of claim 9 or the vector of claim 10.

20. A cell obtainable by the method of claim 19.
